# EUROPEAN PATENT APPLICATION

(11) **EP 4 242 227 A1**
(43) Date of publication of application: **13.09.2023**
(21) Application number: 22382225.5
(22) Date of filing: 09.03.2022
(51) Int. Cl.: C07K 16/10, A61K 39/395, A61P 31/14, C12N 15/13, G01N 33/569, G01N 33/577

(54) **NANOBODIES AGAINST SEVERE ACCUTE RESPIRATORY SYNDROME CORONAVIRUS 2**

(71) Applicant: Consejo Superior De Investigaciones Científicas, 28006 Madrid (ES); Universidad de Las Palmas de Gran Canaria, 35001 Las Palmas de Gran Canaria (ES)
(72) Inventor: FERNÁNDEZ HERRERO, Luis Ángel, 28049 Madrid (ES); CASASNOVAS SUELVES, José María, 28049 Madrid (ES); MARGOLLES AZPIAZU, Yago, 28049 Madrid (ES); NORIEGA FEBRERO, María de los Ángeles, 28049 Madrid (ES); GASTAMINZA LANDART, Pablo, 28049 Madrid (ES); GARAIGORTA DE DIOS, Urtzi, 28049 Madrid (ES); MARTÍN ACEBES, Miguel Ángel, 28040 Madrid (ES); SAIZ CALAHORRA, Juan Carlos, 28040 Madrid (ES); CORBERA SÁNCHEZ, Juan Alberto, 35016 Las Palmas de Gran Canaria (ES)
(74) Representative: ABG Intellectual Property Law, S.L.

(57) **Abstract**

The invention relates to a nanobody directed against the receptor binding domain (RBD) of spike protein from severe acute respiratory syndrome coronavirus 2 (SARS-CoV-2), to a fusion protein comprising said nanobody, and to a heavy chain antibody comprising two heavy chains, wherein each heavy chain comprises said fusion protein. The invention also relates to a bivalent construction comprising two nanobodies, wherein said two nanobodies are linked by a peptide bond. The invention also relates to a nucleic acid encoding the said nanobody, fusion protein or bivalent construction, to a vector comprising said nucleic acid, to a host cell comprising said nucleic acid or said vector, and to a pharmaceutical composition comprising said nanobody, fusion protein, heavy chain antibody or bivalent construction. The invention also relates to the medical uses of said nanobody, fusion protein, heavy chain antibody, bivalent construction or pharmaceutical composition for use in the treatment and/or prevention of an infection caused by SARS-CoV-2, and to a method for diagnosing an infection SARS-CoV-2 in a subject comprising contacting a sample from the subject with said nanobody, fusion protein, heavy chain antibody or bivalent construction.

## Description

### FIELD OF THE INVENTION

The invention relates to the field of therapies against severe acute respiratory syndrome coronavirus 2 (SARS-CoV-2), in particular, therapies based on antibodies directed against the receptor binding domain of spike protein.

### BACKGROUND

The COVID-19 pandemic is caused by severe acute respiratory syndrome coronavirus 2 (SARS-CoV-2), and is a major threat to global public health that has caused over 5 million deaths because of the absence of specific therapeutics. During this year, several COVID-19 vaccines based on different technologies were authorized in different countries, as well as some SARS-CoV-2 neutralizing antibodies generated from COVID-convalescent individuals (Corti et al., 2021, Cell 184: 3086-3108; Kumar et al., 2021, PLOS Pathogens 17: e1009885). Nonetheless, these therapies are been challenged by the emergence of virus variants with enhanced transmission that scape from immune neutralization, which likely requires the update of vaccines and therapeutic Abs.

Most of the antibodies that neutralize viruses, such as the SARS-CoV-2, bind to exposed proteins on the virus particle, and prevent virus cell entry, the initial step in the viral life cycle. In CoV, the virus envelope spike (S) glycoprotein is used for virus attachment to host cells and membrane fusion, and it is the main target of neutralizing antibodies. The SARS-CoV-2 S forms a trimer on the virion surface, and it is cleaved during virus assembly into the N-terminal S1 and the membrane-bound S2 regions, which confers greater infectivity to this CoV. Additional S cleavage at the target cell surface primes SARS-CoV-2 cell entry. Several structures have defined the S domain architecture and identified two conformations (closed and open) for its receptor-binding domain (RBD), which locates at the C-terminal portion of the S1 region. The RBD in the open conformation engages the SARS-CoV-2 entry receptor on the host cell surface, angiotensin-converting enzyme 2 (ACE2), which triggers S protein rearrangements that lead to membrane fusion and virus penetration into the cell. The SARS-CoV-2 RBD spans S residues 330 to 527 and is composed of two subdomains, the core, with a central β-sheet, and the external subdomain or receptor binding motif (RBM, residues 438 to 506), which is connected to two adjacent β-strand of the core; the RBM becomes the most membrane distal site of the S in its receptor-binding open conformation. The RDB is the main antigenic site in CoVs and the target of potent neutralizing antibodies. In SARS-CoV-2, antibodies bind to different RBD regions or subsites, either at the core or RBM. Based on their RBD epitopes, Abs have been classified in at least 4 different classes. Some are RDB conformation-specific, while others can recognize its open and closed forms.

Since the beginning of the pandemic, SARS-CoV-2 circulating strains acquired mutations that facilitate its transmission, like the S2 D614G substitution that replaced the original Wuhan (WA1) strain to become dominant globally. Current SARS-CoV-2 variants contain additional mutations in the RBD that enhance virus transmission by increasing affinity for human ACE2 (e.g. N501Y, K417N/T, L452R) and facilitate escape from neutralizing antibodies (Abs) (e.g. E484K, L452R). Among current variants, the B.1.1.7 (alpha), first detected in the United Kingdom, and the B.1.1.28 or P.2 (zeta) initially identified in Brazil, carry the single RBD mutations N501Y and the E484K, respectively. Nonetheless, other variants of concern contain multiple RBD mutations, as the B.1.351 (beta), reported initially at South Africa with the substitutions K417N, E484K and N501Y, or the related B.1.1.28.1 or P.1 (gamma), which contains changes at the same residues, but with K417T. Highly contagious virus variants have globally disseminated and rapidly become prevalent. The B.1.617.2 (delta) variant, and its related B.1.617.1 (kappa), were identified initially in India and carry RBD L452R-T478K or the L452R-E484Q substitutions, respectively, which are likely responsible of their enhanced transmission. More recently, the B.1.1.529 (omicron) variant, identified in South Africa, contains an extreme evolution of the RBD with fifteen substitutions (i.e. G339D, S371L, S373P, S375F, K417N, N440K, G446S, S477N, T478K, E484A, Q493K, G496S, Q498R, N501Y, Y505H). The RBD mutations found in these variants pose a threat to the efficacy of current vaccines and human therapeutic antibodies, which may also generate an additional pressure for viral evolution and selection of new variants that escape human immune neutralization.

Heavy chain antibodies (hcAb) derived from single variable V_{HH} domains or Nanobodies (Nbs) naturally found in camelids (e.g., dromedaries, llamas, alpacas), hold a great potential given their unique structural, biophysical, and epitope-binding properties. Despite their small size (-14 kDa), Nbs show identical affinity and antigen specificity as conventional antibody molecules with heavy and light chains (i.e., human/mouse IgGs); they have superior properties such as enhanced stability to thermal and chemical denaturation, higher solubility, resistance to proteolysis, and the ability to bind to small protein cavities and conserved inner regions on pathogen surfaces, which are hidden to conventional antibodies (e.g. IgGs) to avoid immune neutralization. Nanobodies can be expressed as monomeric, monovalent molecules, or as fusions with other nanobodies and/or protein domains (e.g. Fc of human IgGs) producing bivalent and multivalent molecules with mono-, bi-, or multi-specific binding capabilities. These properties provide important advantages in the use of Nbs for diagnostic and therapeutic applications. In addition, nanobodies share high sequence identity with human VHs and can be readily humanized or directly applied in therapy. Nanobodies (or fusions containing them) can be delivered intravenously as conventional Abs and by direct inhalation in the lung.

Nanobodies have been developed against different viral infections. Since the beginning of the COVID-19 pandemic, different V_{HH} libraries have been screened to isolate nanobodies that bind the RBD of SARS-CoV-2. Various nanobodies neutralizing viral SARS-CoV-2 WA1 strain *in vitro* were reported. More recent studies also reported nanobodies binding to the RBD of alpha, beta, and gamma variants and that neutralized SARS-CoV-2 *in vitro.* However, few studies have demonstrated *in vivo* protection of SARS-CoV-2 infection with nanobodies, and characterized their binding to circulating variants (Pymm et al., 2021, Proc Natl Acad Sci U S A 118: e2101918118; Wu et al., 2021, Cell reports 37: 109869).

In this context, it becomes highly important the identification of neutralizing antibodies with therapeutic potential and capable of binding and neutralizing different SARS-CoV-2 variants.

### SUMMARY OF THE INVENTION

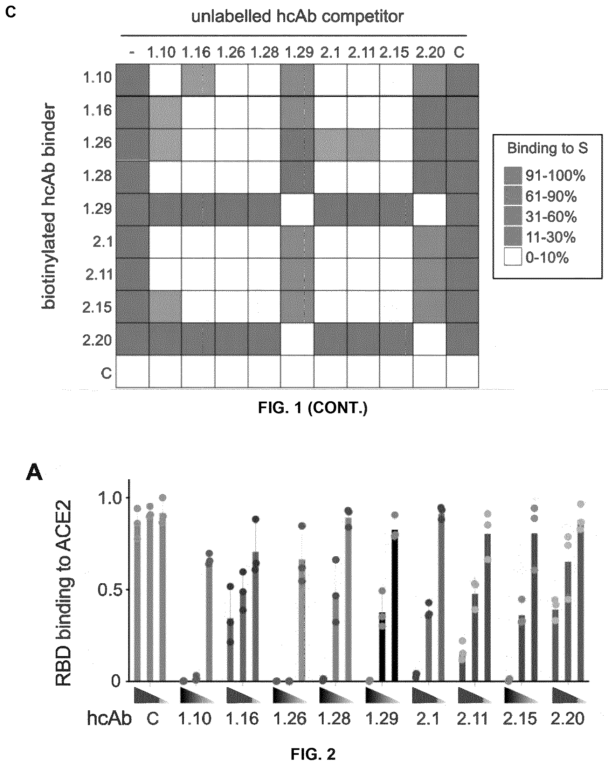

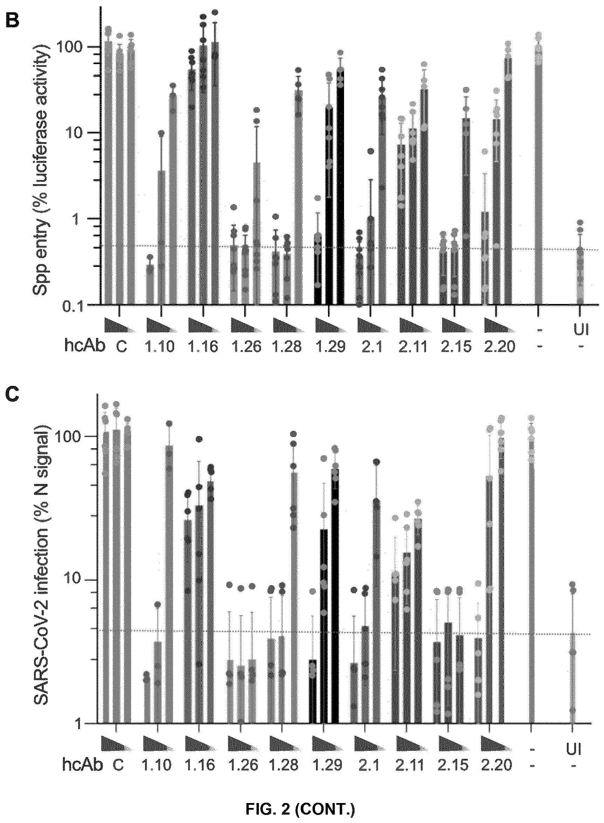

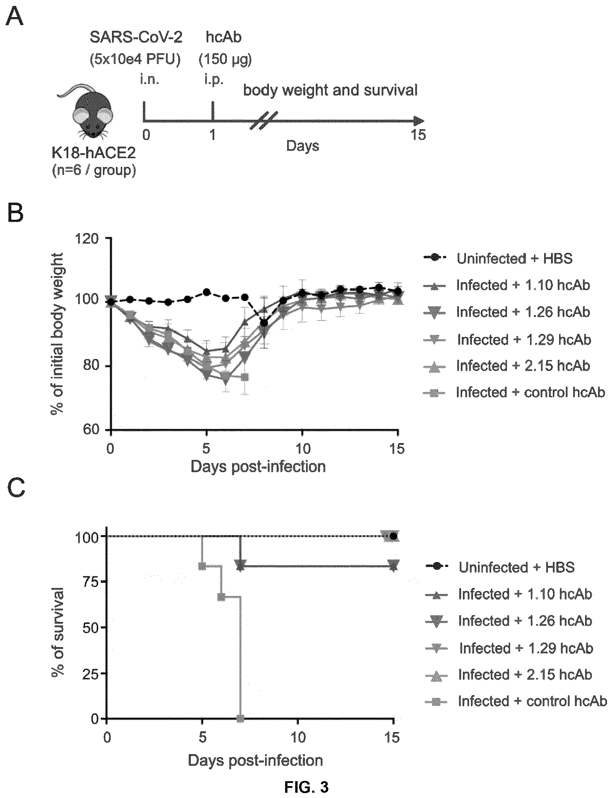

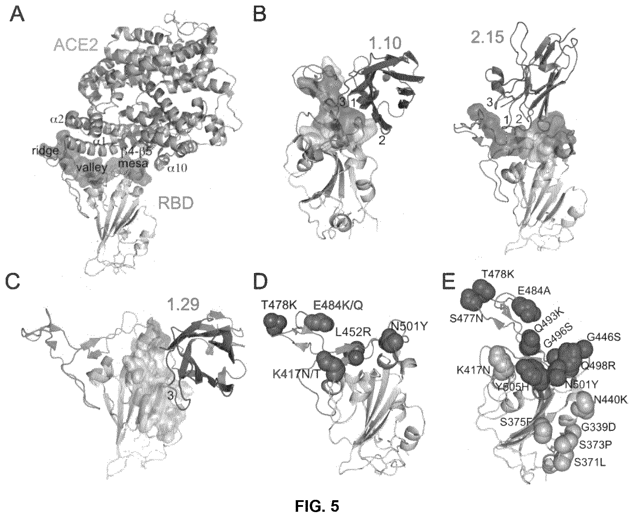

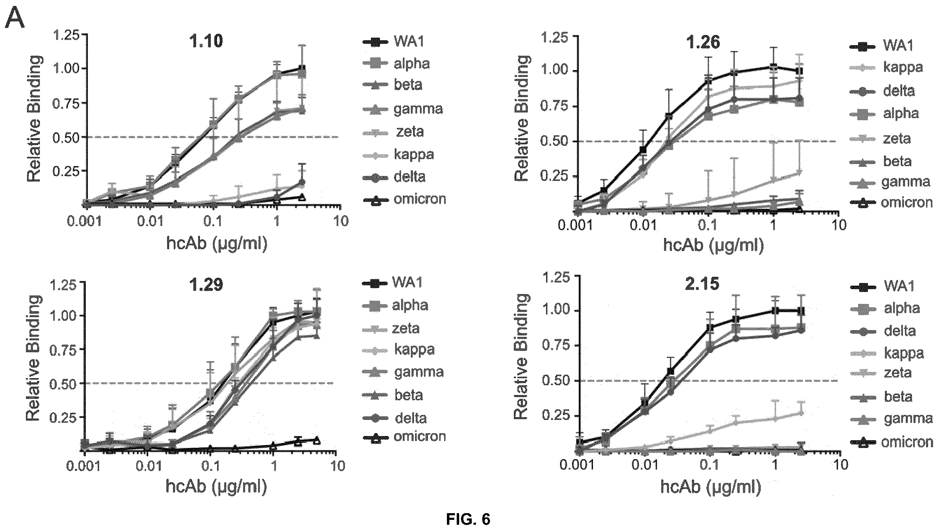

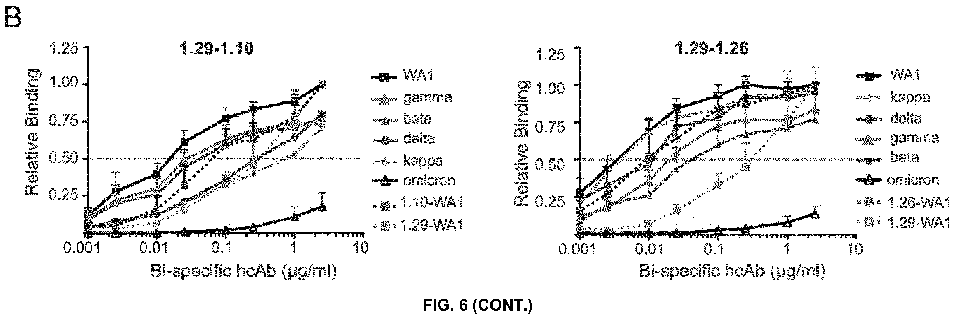

The inventors have developed a panel of high affinity nanobodies (Nb) binding to diverse SARS-CoV-2 RBD epitopes, and a set of nanobody-derived neutralizing hcAbs with therapeutic potential *in vivo,* as they can protect hACE2-transgenic mice after infection with a lethal dose of SARS-CoV-2 (Fig. 3). A broad Nb/hcAb characterization using biophysical, cell and structural biology methods showed how they recognized distinct RBD sites with very high affinities (Table 2); most of them blocked RBD binding to its ACE2 receptor and inhibited SARS-CoV-2 cell entry (Fig. 2). In addition, the inventors have determined the specificity of the neutralizing hcAbs for major RBD variants (alpha, beta, gamma, kappa, zeta, delta and omicron) (Fig. 5), and identified mono- and bi-specific hcAbs that recognized with high affinity all variant RBDs except omicron (Fig. 6). The data showed in the Examples showed the therapeutic potential of these Nbs and hcAbs against most SARS-CoV-2 variants.

Accordingly, in a first aspect, the invention relates to a nanobody directed against the receptor binding domain (RBD) of spike protein from severe acute respiratory syndrome coronavirus 2 (SARS-CoV-2), wherein the nanobody comprises a variable domain comprising a CDR1 comprising the amino acid sequence shown in SEQ ID NO: 7, a CDR2 comprising the amino acid sequence shown in SEQ ID NO: 8 and a CDR3 comprising the amino acid sequence shown in SEQ ID NO: 9, or functionally equivalent variants of said CDRs.

In a second aspect, the invention relates to a fusion protein comprising the nanobody of the first aspect and the Fc region of a heavy chain.

In a third aspect, the invention relates to a heavy chain antibody comprising two heavy chains, wherein each heavy chain comprises the fusion protein according to the second aspect, wherein both heavy chains are identical.

In a fourth aspect, the invention relates to a fusion protein comprising two nanobodies, wherein
(i) the first nanobody is the nanobody according to the first aspect and
(ii) the second nanobody comprises a variable domain selected from the group consisting of:
   (a) a variable domain comprising a CDR1 comprising the amino acid sequence shown in SEQ ID NO: 1, a CDR2 comprising the amino acid sequence shown in SEQ ID NO: 2 and a CDR3 comprising the amino acid sequence shown in SEQ ID NO: 3, or functionally equivalent variants of said CDRs, or
   (b) a CDR1 comprising the amino acid sequence shown in SEQ ID NO: 4, a CDR2 comprising the amino acid sequence shown in SEQ ID NO: 5 and a CDR3 comprising the amino acid sequence shown in SEQ ID NO: 6, or functionally equivalent variants of said CDRs, or
   (c) a CDR1 comprising the amino acid sequence shown in SEQ ID NO: 10, a CDR2 comprising the amino acid sequence shown in SEQ ID NO: 11 and a CDR3 comprising the amino acid sequence shown in SEQ ID NO: 12, or functionally equivalent variants of said CDRs, or
   (d) a CDR1 comprising the amino acid sequence shown in SEQ ID NO: 13, a CDR2 comprising the amino acid sequence shown in SEQ ID NO: 14 and a CDR3 comprising the amino acid sequence shown in SEQ ID NO: 15, or functionally equivalent variants of said CDRs, or
   (e) a CDR1 comprising the amino acid sequence shown in SEQ ID NO: 16, a CDR2 comprising the amino acid sequence shown in SEQ ID NO: 17 and a CDR3 comprising the amino acid sequence shown in SEQ ID NO: 18, or functionally equivalent variants of said CDRs, or
   (f) a CDR1 comprising the amino acid sequence shown in SEQ ID NO: 19, a CDR2 comprising the amino acid sequence shown in SEQ ID NO: 20 and a CDR3 comprising the amino acid sequence shown in SEQ ID NO: 21, or functionally equivalent variants of said CDRs.

In another aspect, the invention relates to a nucleic acid encoding the nanobody according to the first aspect or the fusion protein according to the second aspect or according to the fourth aspect.

In another aspect, the invention relates to a vector comprising the nucleic acid according to the previous aspect or a host cell comprising said nucleic acid or said vector.

In another aspect, the invention relates to a pharmaceutical composition comprising the nanobody according to the first aspect, or the fusion protein according to the second aspect, or the heavy chain antibody according to the third aspect, or the fusion protein according to the fourth aspect, and a pharmaceutically acceptable excipient or carrier.

In another aspect, the invention relates to the nanobody according to the first aspect, or the fusion protein according to the second aspect, or the heavy chain antibody according to the third aspect, or the fusion protein according to the fourth aspect, for use as a medicament.

In another aspect, the invention relates to the nanobody according to the first aspect, or the fusion protein according to the second aspect, or the heavy chain antibody according to the third aspect, or the fusion protein according to the fourth aspect, for use in the treatment and/or prevention of an infection caused by severe acute respiratory syndrome coronavirus 2 (SARS-CoV-2).

In another aspect, the invention relates to an *in vitro* method for diagnosing an infection by severe acute respiratory syndrome coronavirus 2 (SARS-CoV-2) in a subject comprising contactingnanobody according to the first aspect, or the fusion protein according to the second aspect, or the heavy chain antibody according to the third aspect, or the fusion protein according to the fourth aspect, with a sample from the subject and detecting the specific binding of said nanobody, fusion protein or heavy chain antibody to said sample, wherein if said nanobody, fusion protein or heavy chain antibody specifically bind to said sample, the subject is diagnosed as suffering from an infection by SARS-Cov-2.

In another aspect, the invention relates to the use of the nanobody according to the first aspect, or the fusion protein according to the second aspect, or the heavy chain antibody according to the third aspect, or the fusion protein according to the fourth aspect for diagnosing an infection by severe acute respiratory syndrome coronavirus 2 (SARS-CoV-2) in a subject.

### DESCRIPTION OF THE DRAWINGS

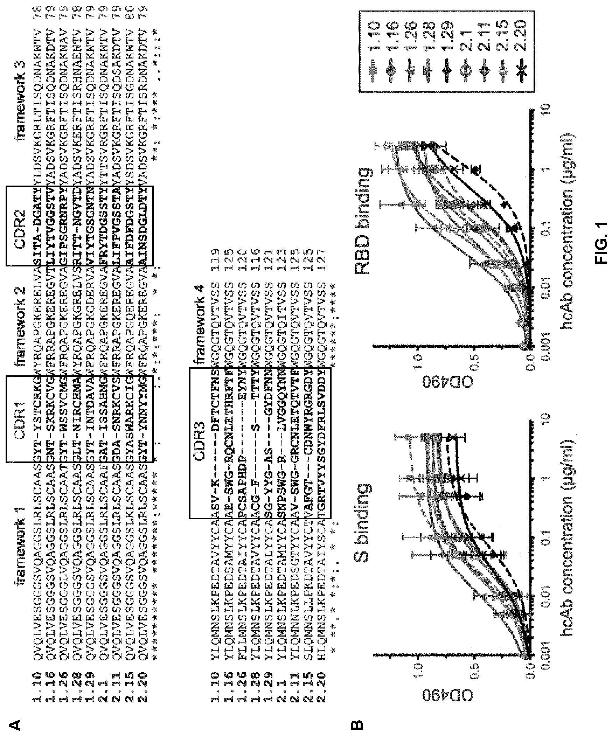

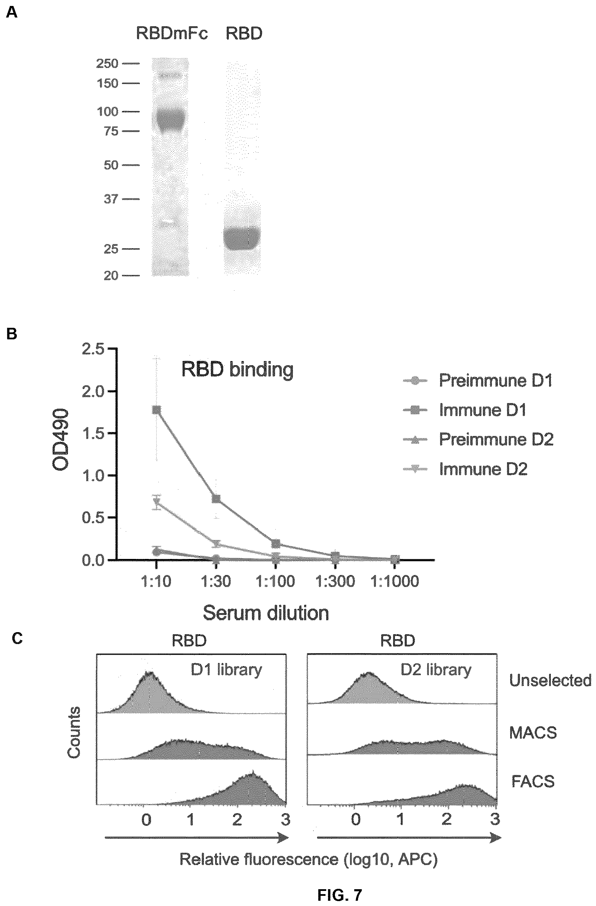

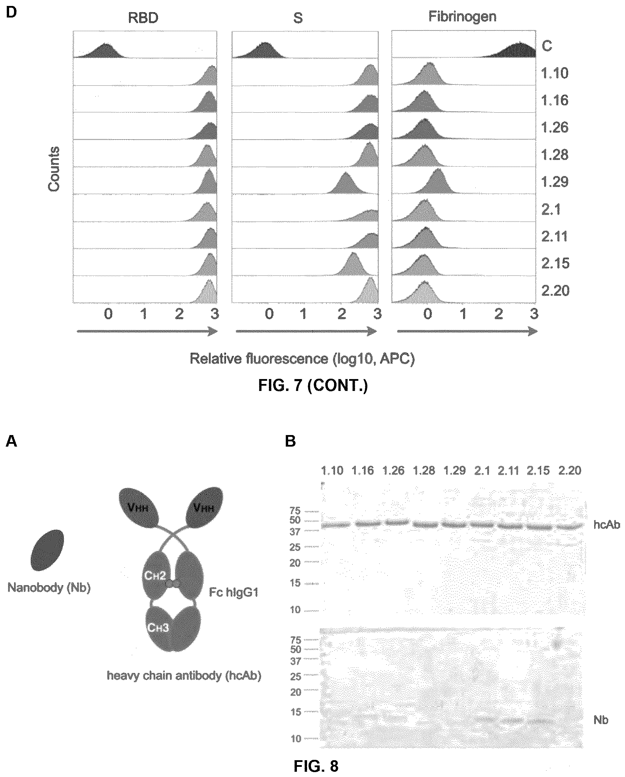

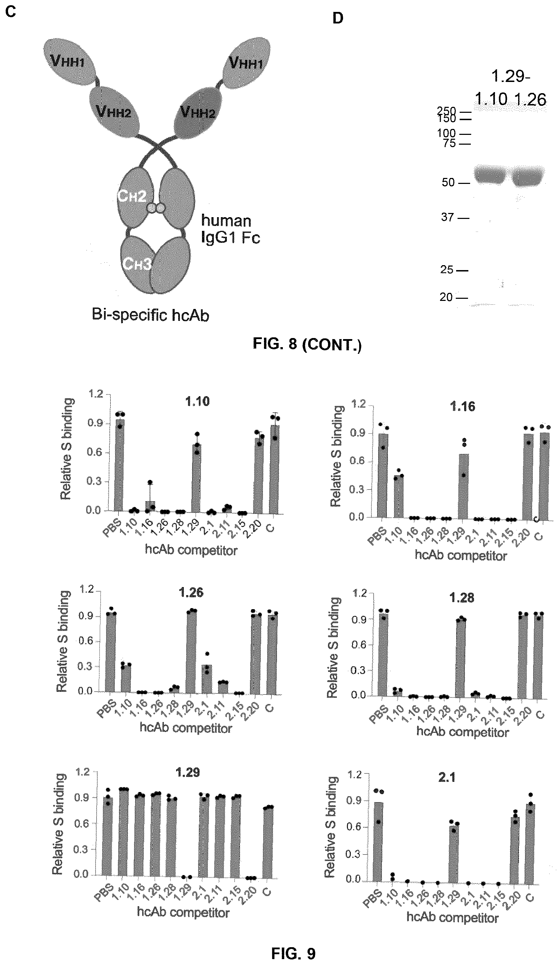

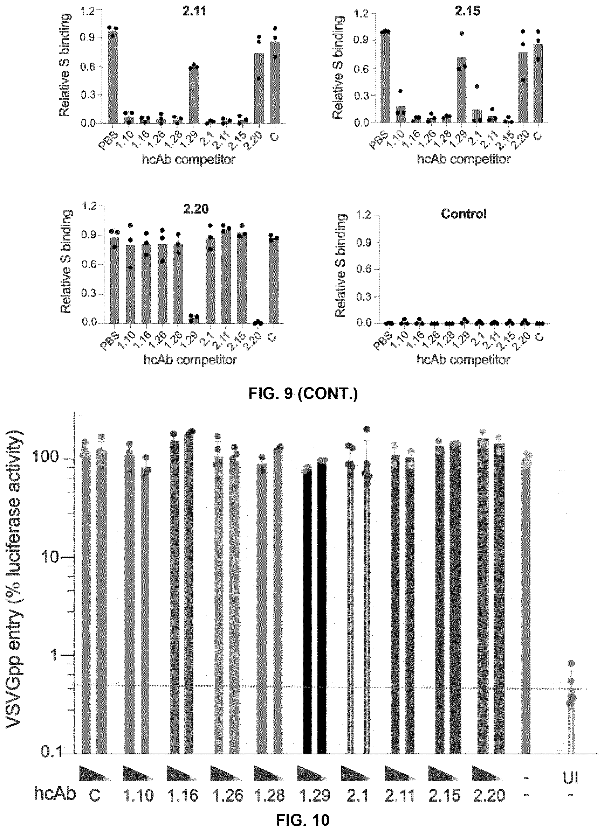

**Figure 1****.** SARS-CoV-2 RBD-specific Nanobodies (Nbs), binding to S and RBD proteins, and cross-competition analysis. **A.** Sequence alignment of the 1.10 (SEQ ID NO: 22), 1.16 (SEQ ID NO: 44), 1.26 (SEQ ID NO. 23), 1.28 (SEQ ID NO: 24), 1.29 (SEQ ID NO: 25), 2.1 (SEQ ID NO: 26), 2.11 (SEQ ID NO: 45), 2.15 (SEQ ID NO: 27) and 2.20 Nbs (SEQ ID NO: 28) selected from bacterial libraries that displayed VHH domains isolated from two immunized dromedaries with the SARS-CoV-2 RBD (Fig. 7). Their VHH frameworks and complementarity determining regions (CDRs) 1 to 3 are indicated. Alignment generated with Clustal Omega. **B.** Binding of Nb-derived hcAbs to the S (left) or RBD (right). The hcAbs contained the RBD-specific Nb domains fused to the human IgG1 hinge and Fc portion (Fig. 8A). Binding of serial hcAb dilutions to plastic-bound proteins measured as Optical Density at 490 nm (OD₄₉₀). A concentration of 1 µg/ml of hcAb corresponds to 25 nM. **Average and standard deviations (n≥3). C.** S binding competition among hcAbs. Heatmap representation of the binding data shown in Figure 9, and determined with biotin labeled hcAbs (left) without (-) or with the unlabeled hcAb competitor shown on the top. A control hcAb (C) was also included. Mean of three independent experiments (n=3).

**Figure 2****. hcAb inhibition of RBD binding to ACE2 and SARS-CoV-2 cell infection *in vitro.* A.** RBD binding to hACE2 in the presence of the indicated hcAbs (1.10, 1.16, 1.26, 1.28, 2.1, 2.11, 2.15, 2.20) or a control hcAb (C). Immunoassays with biotinylated RBD-Fc (20 nM) and the indicated hcAb at increasing concentrations (50, 100 and 200 nM, from right to left). Mean and SD from three independent assays (n=3). **B.** Inhibition of Vero-E6 cell entry of pseudotyped viral particles with SARS-CoV-2 S protein (Spp) by the indicated hcAbs at increasing concentrations (0.5, 5 and 50 nM; equivalent to 0.02, 0.2 and 2 µg/ml, respectively). The luciferase activity of cell cultures was determined 48 h.p.i. Background luciferase activity of uninfected cell cultures is shown with a dashed line. **C.** Inhibition of SARS-CoV-2 virus cell infection by the indicated hcAbs as in B. Infection efficiency was determined by immunofluorescence 24 h.p.i. by staining with anti-N monoclonal Ab (Materials and methods). Background fluorescence detected in uninfected cell cultures is shown with a dashed line. (B and C) Infected cell cultures without hcAb (-) used as positive controls for Spp and SARS-CoV-2 infections.

**Figure 3****.** hcAbs protection of hACE2-trangenic mice after a lethal SARS-CoV-2 infection. **A.** *In vivo* experiment design. On day 0, groups of K18-hACE2 mice (n=6/group) were either infected intranasally (i.n) with a lethal dose of 5×10⁴ plaque forming units (PFU) of SARS-CoV-2 (infected groups) or mock infected with PBS (uninfected group). On day 1 post-infection, 150 µg of 1.10, 1.26, 1.29, 2.15 or control hcAbs, were administered intraperitoneally (i.p.) to animals in the infected groups. The uninfected group was treated i.p. with PBS. **B.** Percentage of daily body weight of animals in each experimental group (as indicated) relative to their body weight on day 0, before infection. Mean and SD of 6 animals. **C.** Percentage of daily mice survival in each experimental group up to 15 d.p.i.

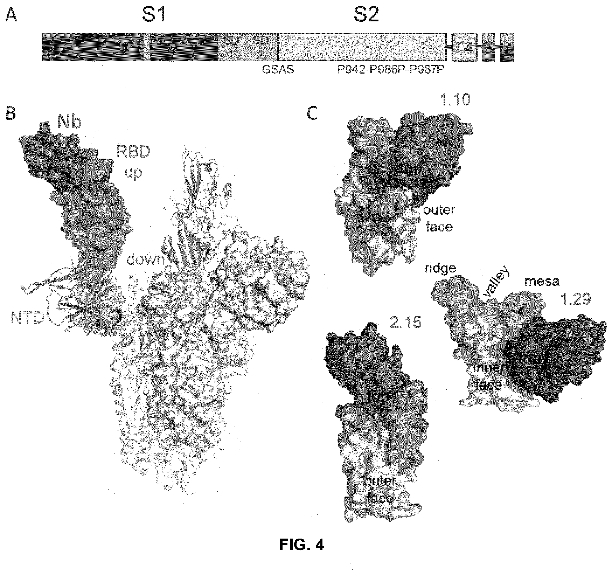

**Figure 4****.** Cryo-EM structure of the SARS-CoV-2 S in complex with RBD-specific Nbs. **A.** Scheme of the S construct used in to generate the S-Nb complexes. The extracelular S1 wih the N-terminal domain (NTD), receptor-binding domain (RBD), subdomains 1 and 2, and the S2 region are shown. The soluble S contained a T4 trimerization domain, a FLAG peptide and a His-tag at its C-terminal end. The furin site was substituted by the GSAS sequence and three prolines were introduced at the S2 region to enhance protein stability and expression. **B.** Cryo-EM structure of the trimeric S with a Nb (2.15) bound to its RBD. The S monomers with the RBD up are represented as surfaces, whereas the monomer with the RBD down is shown as ribbons. The modelled Nbs bound to the RBDs are shown in dark and labeled. **C.** Structures of the SARS-CoV-2 neutralizing Nbs bound to the RBD. Surface representations of RBD-Nb complexes determined by cryo-EM; the RBDs are shown with the RBM and the RBD core in different light gray, and the Nbs in dark gray and labelled. The RBD sided facing toward or opposite to the trimer center, and the RBM regions are indicated.

**Figure 5****.** Nb binding epitopes at the SARS-CoV-2 RBD, overlapping with the ACE2 receptor binding region and with mutations in the SARS-CoV-2 RBD variants. A. Scheme of the S construct used in to generate the S-Nb complexes. The extracellular S1 wih the N-terminal domain (NTD), receptor-binding domain (RBD), subdomains 1 and 2, and the S2 region are shown. The soluble S contained a T4 trimerization domain, a FLAG peptide and a His-tag at its C-terminal end. The furin site was substituted by the GSAS sequence and three prolines were introduced at the S2 region to enhance protein stability and expression. **B.** Cryo-EM structure of the trimeric S with a Nb (2.15) bound to its RBD. The S monomers with the RBD up are represented as surfaces, whereas the monomer with the RBD down is shown as ribbons. The modelled Nbs bound to the RBDs are shown in dark and labeled. **C.** Structures of the SARS-CoV-2 neutralizing Nbs bound to the RBD. Surface representations of RBD-Nb complexes determined by cryo-EM; the RBDs are shown with the RBM and the RBD core in different light gray, and the Nbs in dark gray and labelled. The RBD sided facing toward or opposite to the trimer center, and the RBM regions are indicated.

**Figure 6****.** Monospecific and bispecific hcAb recognition of RBD variants. **A.** Binding of hcAbs 1.10, 1.26, 1.29 and 2.15 (indicated on top of each graph) to RBD-Fc protein variants, which are shown and color coded on the right of each graph; listed from higher (top) to lower (bottom) hcAb binding activity (EC₅₀). The OD₄₉₀ determined with serial hcAb dilutions as in Figure 1 was normalized to the maximum binding signal with the WA1 protein. The dotted line represents half of the maximum hcAb binding to WA1 RBD-Fc, used to determined EC₅₀ values. Mean and standard deviation (SD) of at least three independent assays (n≥3). **B.** Binding of bispecific hcAbs 1.29-1.10 and 1.29-1.26 carried out as in A. Binding of the monospecific hcAbs 1.29, 1.10 or 1.26 to the WA1 RBD are also shown for direct comparison with binding of bispecific hcAbs. Data are mean and standard deviation (SD) of three independent assays (n=3).

**Figure 7****.** Selection of nanobodies binding to SARS-CoV-2 RBD by E. coli surface display. **A.** Proteins used in immunization and nanobody selection. SDS-PAGE (10%) under reducing conditions of purified dimeric RBDmFc and monomeric RBD used for immunization of two dromedaries and for nanobody selection, respectively. Size (kDa) and migration of molecular weight markers are indicated on the left. **B.** Anti-RBD antibody titers in the serum of immunized animals determined by ELISA. Optical density at 490 nm (OD490) determined at different dilution of the sera collected before and 4 days after dromedary D1 and D2 immunization (see Materials and methods). Mean and standard deviation (SD) of three independent assays (n=3). **C.** Enrichment of bacteria displaying Nb libraries generated from dromedaries (D1 and D2) immunized with the SARS-CoV-2 RBD. Flow cytometry of bacterial pools before (unselected) and after selection with monomeric RBD by magnetic cell sorting (MACS) and fluorescence activated cell sorting (FACS). Bacteria were incubated biotin-labeled RBD (5 nM) and stained with Streptavidin-APC. **D.** Flow cytometry of bacterial clones selected from the D1 (1.10, 1.16, 1.26 and 1.28) and the D2 (2.1, 2.11, 2.15 and 2.20) libraries. Bacteria were incubated with the indicated biotin-labeled protein, RBD (5 nM), S (5 nM), or human fibrinogen (100 nM), and stained with Streptavidin-APC. Bacteria displaying a Nb binding to human fibrinogen were used as control (C).

**Figure 8****.** Nb and hcAb proteins prepared in mammalian cells. **A.** Graphical representation of single VHH domain or Nanobody (Nb), and of the heavy-chain antibody (hcAb) generated by the fusion of a VHH with the Fc region of human IgG1 (Fc hIgG1). **B.** SDS-PAGE (12%) of purified 1.10, 1.16, 1.26, 1.28, 2.1, 2.11, 2.15 and 2.20 hcAbs (top) and the Nbs (bottom) generated from the the hcAbs as described in Materials and methods. **C.** Representation of a bispecific hcAb with two distinct VHH domains in tandem and fused to the Fc hIgG1. **D.** SDS-PAGE (10%) of affinity purified the 1.29-1.10 and 1.29-1.26 bispecific hcAbs from transfected cell culture supernatants. For B and D size (in kDa) and migration of molecular weight markers are indicated on the left.

**Figure 9****.** Binding competition between hcAb clones. Graphs showing relative binding of the S protein by the indicated biotin-labelled hcAb clone (on top of each graph) in the presence of unlabelled hcAb competitors (indicated in the x-axis). Biotin-labelled hcAb mixed with 200-fold molar excess of the indicated unlabelled hcAb and added to immunoplates coated with S protein. Bound biotinylated hcAbs are developed with streptavidin-POD conjugate and read at OD490 (see Materials and methods). Mean values in the absence of competitor (PBS) for each hcAb clone are considered 1. A control hcAb (C) binding an unrelated bacterial antigen is used as specificity control. Data of three independent experiments (n=3).

**Figure 10****.** RBD-specific hcAb cell entry inhibition of retrovirus pseudotyped with the Vesicular Stomatitis virus G protein (VSVGpp). Cell entry of luciferase-encoding retroviral particles in Vero-E6 cells was determined from the cell-associated luciferase signal 16 h.p.i. (see Materials and methods). Relative entry of virus particles preincubated with the indicated hcAbs (1.10, 1.16, 1.26, 1.28, 2.1, 2.11, 2.15, 2.20) or a control (C) at two protein concentrations (50 and 5 nM), with respect to virus samples without hcAbs (-). Background luciferase activity of uninfected cell cultures is shown with a dashed line. Plotted mean and SD.

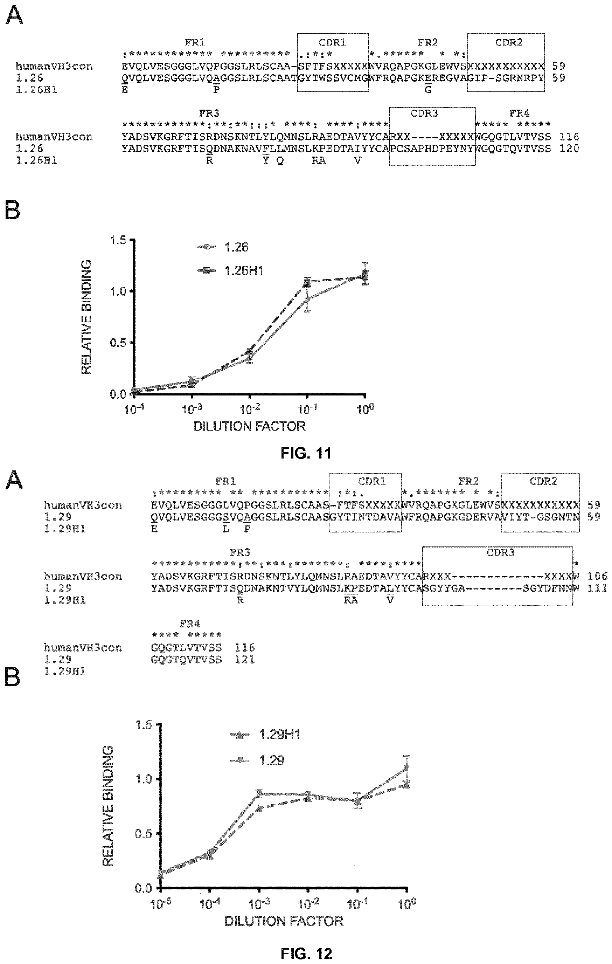

**Figure 11****.** Humanization of nanobody 1.26 to produce 1.26H1. **A.** The amino acid sequence of camelid VHH 1.26 is shown aligned with the consensus sequence of human VH3 using Clustal Omega. The framework regions 1 to 4 (FR1 to FR4) and CDRs 1 to 3 are labelled.The residues mutated in the humanized 1.26H1 sequence are shown below the original residue of 1.26. B. ELISA of serial dilutions of 293F-cell culture supernantants containing secreted hcAbs 1.26 or 1.26H1, as indicated. Binding of the hcAb to immunoplates coated with S protein of SARS-CoV-2 was developed with secondary anti-human IgG1-HRP conjugate.

**Figure 12****.** Humanization of nanobody 1.29 to produce 1.29H1. **A.** The amino acid sequence of camelid VHH 1.29 is shown aligned with the consensus sequence of human VH3 using Clustal Omega. The framework regions 1 to 4 (FR1 to FR4) and CDRs 1 to 3 are labelled. The residues mutated in the humanized 1.29H1 sequence are shown below the original residue of 1.29. **B.** ELISA of serial dilutions of 293F-cell culture supernantants containing secreted hcAbs 1.29 or 1.29H1, as indicated. Binding of the hcAb to immunoplates coated with S protein of SARS-CoV-2 was developed with secondary anti-human IgG1-HRP conjugate.

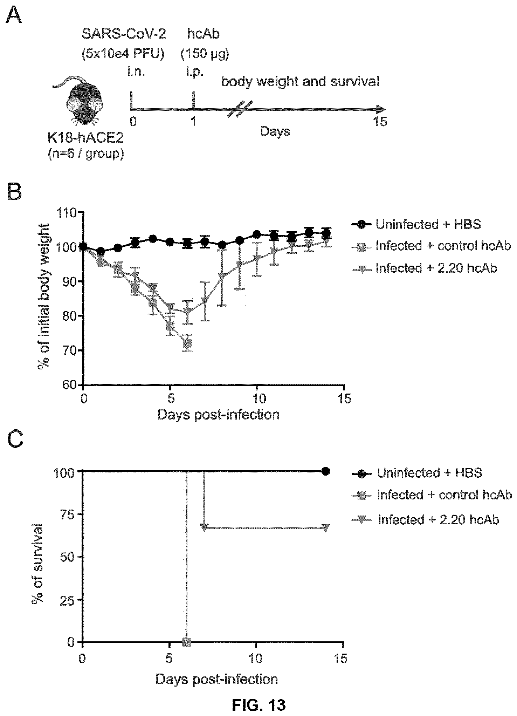

**Figure 13****.** Protection of hACE2-trangenic mice by hcAb 2.20 after a lethal SARS-CoV-2 infection. **A.** *In vivo* experiment design. On day 0, groups of K18-hACE2 mice (n=6/group) were either infected intranasally (i.n) with a lethal dose of 5×10⁴ plaque forming units (PFU) of SARS-CoV-2 (infected groups) or mock infected with PBS (uninfected group). On day 1 post infection, 150 µg of 2.20 or control hcAbs, were administered intraperitoneally (i.p.) to animals in the infected groups. The uninfected group was treated i.p. with HBS. **B.** Percentage of daily body weight of animals in each experimental group (as indicated) relative to their body weight on day 0, before infection. Mean and SD of 6 animals. **C.** Percentage of daily mice survival in each experimental group up to 15 d.p.i.

### DETAILED DESCRIPTION OF THE INVENTION

### Nanobodies of the invention

In a first aspect, the invention relates to a nanobody selected from the group consisting of:
(A) A nanobody directed against the receptor binding domain (RBD) of spike protein from severe acute respiratory syndrome coronavirus 2 (SARS-CoV-2), wherein the nanobody comprises a variable domain comprising a CDR1 comprising the amino acid sequence shown in SEQ ID NO: 10, a CDR2 comprising the amino acid sequence shown in SEQ ID NO: 11 and a CDR3 comprising the amino acid sequence shown in SEQ ID NO: 12, or functionally equivalent variants of said CDRs.
(B) A nanobody directed against the receptor binding domain (RBD) of spike protein from severe acute respiratory syndrome coronavirus 2 (SARS-CoV-2), wherein the nanobody comprises a variable domain comprising a CDR1 comprising the amino acid sequence shown in SEQ ID NO: 1, a CDR2 comprising the amino acid sequence shown in SEQ ID NO: 2 and a CDR3 comprising the amino acid sequence shown in SEQ ID NO: 3, or functionally equivalent variants of said CDRs.
(C)A nanobody directed against the receptor binding domain (RBD) of spike protein from severe acute respiratory syndrome coronavirus 2 (SARS-CoV-2), wherein the nanobody comprises a variable domain comprising a CDR1 comprising the amino acid sequence shown in SEQ ID NO: 4, a CDR2 comprising the amino acid sequence shown in SEQ ID NO: 5 and a CDR3 comprising the amino acid sequence shown in SEQ ID NO: 6, or functionally equivalent variants of said CDRs.
(D)A nanobody directed against the receptor binding domain (RBD) of spike protein from severe acute respiratory syndrome coronavirus 2 (SARS-CoV-2), wherein the nanobody comprises a variable domain comprising a CDR1 comprising the amino acid sequence shown in SEQ ID NO: 7, a CDR2 comprising the amino acid sequence shown in SEQ ID NO: 8 and a CDR3 comprising the amino acid sequence shown in SEQ ID NO: 9, or functionally equivalent variants of said CDRs.
(E) A nanobody directed against the receptor binding domain (RBD) of spike protein from severe acute respiratory syndrome coronavirus 2 (SARS-CoV-2), wherein the nanobody comprises a variable domain comprising a CDR1 comprising the amino acid sequence shown in SEQ ID NO: 13, a CDR2 comprising the amino acid sequence shown in SEQ ID NO: 14 and a CDR3 comprising the amino acid sequence shown in SEQ ID NO: 15, or functionally equivalent variants of said CDRs.
(F) A nanobody directed against the receptor binding domain (RBD) of spike protein from severe acute respiratory syndrome coronavirus 2 (SARS-CoV-2), wherein the nanobody comprises a variable domain comprising a CDR1 comprising the amino acid sequence shown in SEQ ID NO: 16, a CDR2 comprising the amino acid sequence shown in SEQ ID NO: 17 and a CDR3 comprising the amino acid sequence shown in SEQ ID NO: 18, or functionally equivalent variants of said CDRs.
(G)A nanobody directed against the receptor binding domain (RBD) of spike protein from severe acute respiratory syndrome coronavirus 2 (SARS-CoV-2), wherein the nanobody comprises a variable domain comprising a CDR1 comprising the amino acid sequence shown in SEQ ID NO: 19, a CDR2 comprising the amino acid sequence shown in SEQ ID NO: 20 and a CDR3 comprising the amino acid sequence shown in SEQ ID NO: 21, or functionally equivalent variants of said CDRs.

In a preferred embodiment, the nanobody comprises a variable domain comprising a CDR1 comprising the amino acid sequence shown in SEQ ID NO: 7, a CDR2 comprising the amino acid sequence shown in SEQ ID NO: 8 and a CDR3 comprising the amino acid sequence shown in SEQ ID NO: 9, or functionally equivalent variants of said CDRs.

The term "nanobody", as used herein, refers to the variable domain of a single heavy chain antibody. Single heavy chain antibodies are typically found in camelids and comprise two heavy chains but are devoid of light chains. The heavy chain of camelid antibodies is formed by a variable domain (VHH) and two constant domains (CH1 and CH2), but lack the constant domain CH1 usually present in conventional antibodies.

The nanobodies of the invention are directed against the receptor binding region of spike protein from severe acute respiratory syndrome coronavirus 2 (SARS-CoV-2).

The term **"severe acute respiratory syndrome coronavirus** 2" or **"SARS-CoV-2"** refers to the strain of coronavirus that causes coronavirus disease 2019 (COVID-19), a respiratory syndrome that manifests a clinical pathology resembling mild upper respiratory tract disease (common cold-like symptoms) and occasionally severe lower respiratory tract illness and extra-pulmonary manifestations leading to multi-organ failure and death. In particular, the term refers to the virus identified by Zhou et al. (2020). Nature 579: 270-273. SARS-CoV-2 is a positive sense single-stranded RNA virus. The term **"SARS-Cov-2"** includes any the variants of this virus. Illustrative non-limitative embodiments of SARS-Cov-2 variants are the originally discovered Wuhan (or WT) virus (NCBI Reference Sequence: NC_045512.2 (18 July 2020), B.1.1.7 (alpha), B.1.351 (beta) , B.1.1.28.1 or P.1 (gamma), B.1.1.28 or P.2 (zeta), , B.1.1.207, B.1.1.248, B.1.1.317, B.1.1.318, B.1.429, B.1.525, B.1.526, B.1.617.2 (delta), B.1.617.1 (kappa), B.1.618, B.1.620, and P.3 (theta), B.1.621 (Mu), C.37 (Lambda), Ay.4.2, C.1.2, B.1.640, B.1.427/B.1.429 (epsilon), B.1.616, B.1.617.3, B.1.214.2, A.23.1. A.27, A.28, C.16, and B.1.1.529 (omicron).

The term "spike protein" or "S protein", as used herein, refers to one of the four structural proteins of SARS-CoV-2, namely spike (S) protein, envelope (E) protein, membrane (M) protein and nucleocapsid (N) protein. S protein is a glycoprotein responsible for allowing the virus to attach to and fuse with the membrane of the host cell. S protein is a trimeric protein, each monomer having about 180 kDa and containing two subunitis: S1 and S2. S protein binds to the receptor angiotensine converting enzyme 2 (ACE2) in human cells, mediateing the entrance of the virus in the cell. S1 subunit binds ACE2 and S2 subunit mediates membrane fusion. S1 is composed of 672 amino acids (residues 14-685) and organized into four domains: an N-terminal domain (NTD), a C-terminal domain (CTD, also known as the receptor-binding domain, RBD), and two subdomains (SD1 and SD2). The transmembrane S2 subunit is composed of 588 amino acids (residues 686-1273) and contains an N-terminal hydrophobic fusion peptide (FP), two heptad repeats (HR1 and HR2), a transmembrane domain (TM), and a cytoplasmic tail (CT), arranged as FP-HR1-HR2-TM-CT. The term "S protein" as used herein, refers to the S protein from any of the SARS-CoV-2 variants. In particular, the term S protein refers to the protein having the sequence defined by the accesion number P0DTC2 in the UniProtKB database (Entry version 9, 29 September 2021; Sequence version 1, 22 April 2020) or any of the variants of this sequence defined in the mentioned database.

The term "receptor binding region" or "RBD" refers to a region of the S1 subunit of the SARS-CoV-2 S protein. The RBD domain spans from residues 330 to 527 and is composed of two subdomains, a core, with a central β-sheet, and an external subdomain or receptor binding motif (RBD), spanning from residues 438 to 506.

The nanobodies of the invention comprise a variable domain comprising three CDRs. The term "complementary determining region" or "CDR", as used herein, refers to the antigen binding sites found within the variable region of both heavy and light chain of antibodies. CDRs may be defined using various terms: (i) Complementarity Determining Regions (CDRs), three in the VH (HCDR1, HCDR2, HCDR3) and three in the VL (LCDR1, LCDR2, LCDR3) are based on sequence variability (Wu et al. (1970) J Exp Med 132: 211-50) (Kabat et al., Sequences of Proteins of Immunological Interest, 5th Ed. Public Health Service, National Institutes of Health, Bethesda, Md., 1991). (ii) "Hypervariable regions", "HVR", or "HV", three in the VH (H1, H2, H3) and three in the VL (L1, L2, L3) refer to the regions of an antibody variable domains which are hypervariable in structure as defined by Chothia and Lesk (Chothia et al. (1987) J Mol Biol 196: 901-17). The International ImMunoGeneTics (IMGT) database (http://www_imgt_org) provides a standardized numbering and definition of antigen-binding sites. The correspondence between CDRs, HVs and IMGT delineations is described in (Lefranc et al. (2003) Dev Comp Immunol 27: 55-77). The term "CDR", "HCDR1", "HCDR2", "HCDR3", "LCDR1", "LCDR2" and "LCDR3" as used herein includes CDRs defined by any of the methods described supra, Kabat, Chothia or IMGT, unless otherwise explicitly stated in the specification.

In a particular embodiment, the nanobodies of the invention comprise a variable domain comprising one of the following combinations of CDRs:
(A) a CDR1 comprising or consisting of the amino acid sequence shown in SEQ ID NO: 10, a CDR2 comprising or consisting of the amino acid sequence shown in SEQ ID NO: 11 and a CDR comprising or consisting of the amino acid sequence shown in SEQ ID NO: 12,
(B) a CDR1 comprising or consisting of the amino acid sequence shown in SEQ ID NO: 1, a CDR2 comprising or consisting of the amino acid sequence shown in SEQ ID NO: 2 and a CDR comprising or consisting of the amino acid sequence shown in SEQ ID NO: 3,
(C) a CDR1 comprising or consisting of the amino acid sequence shown in SEQ ID NO: 4, a CDR2 comprising or consisting of the amino acid sequence shown in SEQ ID NO: 5 and a CDR comprising or consisting of the amino acid sequence shown in SEQ ID NO: 6,
(D) a CDR1 comprising or consisting of the amino acid sequence shown in SEQ ID NO: 7, a CDR2 comprising or consisting of the amino acid sequence shown in SEQ ID NO: 8 and a CDR comprising or consisting of the amino acid sequence shown in SEQ ID NO: 9,
(E) a CDR1 comprising or consisting of the amino acid sequence shown in SEQ ID NO: 13, a CDR2 comprising or consisting of the amino acid sequence shown in SEQ ID NO: 14 and a CDR comprising or consisting of the amino acid sequence shown in SEQ ID NO: 15,
(F) a CDR1 comprising or consisting of the amino acid sequence shown in SEQ ID NO: 16, a CDR2 comprising or consisting of the amino acid sequence shown in SEQ ID NO: 17 and a CDR comprising or consisting of the amino acid sequence shown in SEQ ID NO: 18,
(G) a CDR1 comprising or consisting of the amino acid sequence shown in SEQ ID NO: 19, a CDR2 comprising or consisting of the amino acid sequence shown in SEQ ID NO: 20 and a CDR comprising or consisting of the amino acid sequence shown in SEQ ID NO: 21.

In a particular embodiment, the nanobodies of the invention comprise a variable domain comprising three CDRs, wherein said CDRs comprise or consists of a functionally equivalent variant of the above-mentioned sequences. In particular:
(A) a CDR1 comprising or consisting a functionally equivalent variant of the of the amino acid sequence of SEQ ID NO: 10, a CDR2 comprising or consisting of a functionally equivalent variant of the amino acid sequence shown in SEQ ID NO: 11 and a CDR3 comprising or consisting a functionally equivalent variant of the amino acid sequence of SEQ ID NO: 12,
(B) a CDR1 comprising or consisting a functionally equivalent variant of the of the amino acid sequence of SEQ ID NO: 1, a CDR2 comprising or consisting of a functionally equivalent variant of the amino acid sequence shown in SEQ ID NO: 2 and a CDR3 comprising or consisting a functionally equivalent variant of the amino acid sequence of SEQ ID NO: 3,
(C) a CDR1 comprising or consisting a functionally equivalent variant of the of the amino acid sequence of SEQ ID NO: 4, a CDR2 comprising or consisting of a functionally equivalent variant of the amino acid sequence shown in SEQ ID NO: 5 and a CDR3 comprising or consisting a functionally equivalent variant of the amino acid sequence of SEQ ID NO: 6,
(D) a CDR1 comprising or consisting a functionally equivalent variant of the of the amino acid sequence of SEQ ID NO: 7, a CDR2 comprising or consisting of a functionally equivalent variant of the amino acid sequence shown in SEQ ID NO: 8 and a CDR3 comprising or consisting a functionally equivalent variant of the amino acid sequence of SEQ ID NO: 9,
(E) a CDR1 comprising or consisting a functionally equivalent variant of the of the amino acid sequence of SEQ ID NO: 13, a CDR2 comprising or consisting of a functionally equivalent variant of the amino acid sequence shown in SEQ ID NO: 14 and a CDR3 comprising or consisting a functionally equivalent variant of the amino acid sequence of SEQ ID NO: 15,
(F) a CDR1 comprising or consisting a functionally equivalent variant of the of the amino acid sequence of SEQ ID NO: 16, a CDR2 comprising or consisting of a functionally equivalent variant of the amino acid sequence shown in SEQ ID NO: 17 and a CDR3 comprising or consisting a functionally equivalent variant of the amino acid sequence of SEQ ID NO: 18,
(G) a CDR1 comprising or consisting a functionally equivalent variant of the of the amino acid sequence of SEQ ID NO: 19, a CDR2 comprising or consisting of a functionally equivalent variant of the amino acid sequence shown in SEQ ID NO: 20 and a CDR3 comprising or consisting a functionally equivalent variant of the amino acid sequence of SEQ ID NO: 21.

The term "functionally equivalent variant of a CDR sequence", as used herein, refers to a sequence variant of a particular CDR sequence having substantially sequence identity with it and substantially maintaining its capacity to bind to its cognate antigen when being part of a nanobody, fusion protein, heavy chain antibody or bivalent construction as the ones described herein. For example, a functionally equivalent variant of a CDR sequence may be a polypeptide sequence derivative of said sequence comprising the addition, deletion or substitution of one or more amino acids.

The terms "substantially identical" or "having substantially sequence identity" in the context of two or more CDR sequences, peptides or polypeptides, refer to two or more sequences or subsequences that are the same or have a specified percentage of amino acid residues that are the same, when compared and aligned (introducing gaps, if necessary) for maximum correspondence, not considering any conservative amino acid substitutions as part of the sequence identity. The percent identity can be measured using sequence comparison software or algorithms or by visual inspection. Various algorithms and software are known in the art that can be used to obtain alignments of amino acid or nucleotide sequences. One such non-limiting example of a sequence alignment algorithm is the algorithm incorporated into the NBLAST and XBLAST programs (Altschul et al., 1991, Nucleic Acids Res., 25:3389-3402). In certain embodiments, Gapped BLAST can be used. BLAST-2, WU-BLAST-2, ALIGN, ALIGN-2 (Genentech, South San Francisco, California) or Megalign (DNASTAR) are additional publicly available software programs that can be used to align sequences. In certain embodiments, the percent identity between two nucleotide sequences is determined using the GAP program in GCG software (e.g., using a NWSgapdna.CMP matrix and a gap weight of 40, 50, 60, 70, or 90 and a length weight of 1, 2, 3, 4, 5, or 6). In certain alternative embodiments, the GAP program in the GCG software package can be used to determine the percent identity between two amino acid sequences (e.g., using either a Blossum 62 matrix or a PAM250 matrix, and a gap weight of 16, 14, 12, 10, 8, 6, or 4 and a length weight of 1, 2, 3, 4, 5). Alternatively, in certain embodiments, the percent identity between nucleotide or amino acid sequences is determined using the algorithm of Myers and Miller (CABIOS, 4:11-17 (1989)). For example, the percent identity can be determined using the ALIGN program (version 2.0) and using a PAM120 with residue table, a gap length penalty of 12 and a gap penalty of 4. Appropriate parameters for maximal alignment by particular alignment software can be determined by one skilled in the art. In certain embodiments, the default parameters of the alignment software are used. In certain embodiments, the percentage identity "X" of a first amino acid sequence to a second sequence amino acid is calculated as 100 × (Y/Z), where Y is the number of amino acid residues scored as identical matches in the alignment of the first and second sequences (as aligned by visual inspection or a particular sequence alignment program) and Z is the total number of residues in the second sequence. If the second sequence is longer than the first sequence, then the percent identity may be determined only in the region of overlap between said first and second sequences. In this case, the same formula as above can be used but using as Z value the length of the region wherein the first and second sequence overlaps, said region having a length which is substantially the same as the length of the first sequence. Unless indicated otherwise, the sequence identity is calculated over the whole length of the reference sequence.

As a non-limiting example, whether any particular polynucleotide has a certain percentage sequence identity (e.g., is at least 70% identical, at least 75% identical, at least 80% identical, at least 85% identical, at least 90% identical, and in some embodiments, at least 95%, 96%, 97%, 98%, or 99% identical) to a reference sequence can, in certain embodiments, be determined using the Bestfit program (Wisconsin Sequence Analysis Package, Version 8 for Unix, Genetics Computer Group, University Research Park, 575 Science Drive, Madison, WI 53711). Bestfit uses the local homology algorithm of Smith and Waterman, Advances in Applied Mathematics 2: 482 489 (1981), to find the best segment of homology between two sequences. When using Bestfit or any other sequence alignment program to determine whether a particular sequence is, for instance, 95% identical to a reference sequence according to the present invention, the parameters are set such that the percentage of identity is calculated over the full length of the reference amino acid sequence and that gaps in homology of up to 5% of the total number of amino acids in the reference sequence are allowed.

In a particular embodiment, functionally equivalent variants of a CDR sequence according to the invention include CDR sequences having at least approximately 70%, at least 75%, at least 80%, at least 85%, at least 90%, at least 91%, at least 92%, at least 93%, at least 94%, at least 95%, at least 96%, at least 97%, at least 98% or at least 99% sequence identity with the corresponding amino acid sequences shown in one of SEQ ID NOs: 1 to 21. It is also contemplated that functionally equivalent variants of a CDR sequence comprise additions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence shown in one of SEQ ID NOs: 1 to 21. Likewise, it is also contemplated that variants comprise deletions consisting of at least 1 amino acid, or at least 2 amino acids, or at least 3 amino acids, or at least 4 amino acids, or at least 5 amino acids, or at least 6 amino acids, or at least 7 amino acids, or at least 8 amino acids, or at least 9 amino acids, or at least 10 amino acids or more amino acids at the N-terminus, or at the C-terminus, or both at the N- and C-terminus of the corresponding amino acid sequence shown in one of SEQ ID NOs: 1 to 21.
In a particular embodiment, functionally equivalent variants of the CDR sequences of the invention comprise conservative amino acid substitutions, meaning that one amino acid is substituted with another amino acid having similar properties. As used herein, the term "conservative substitution" refers to the replacement of an amino acid by another amino acid having similar chemical properties. Conservative substitution tables providing functionally similar amino acids are well known in the art. The following six groups each contain amino acids that are conservative substitutions for one another:
- Alanine (A), Serine (S), Threonine (T);
- Aspartic acid (D), Glutamic acid (E);
- Asparagine (N), Glutamine (Q);
- Arginine (R), Lysine (K);
- Isoleucine (I), Leucine (L), Methionine (M), Valine (V); and
- Phenylalanine (F), Tyrosine (Y), Tryptophan (W).

Functionally equivalent variants a CDR sequence according to the invention will preferably maintain at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, at least 100%, at least 105%, at least 110%, at least 115%, at least 120%, at least 125%, at least 130%, at least 135%, at least 140%, at least 145%, at least 150%, at least 200% or more of the capacity of the corresponding amino acid sequence shown in one of SEQ ID NOs: 1 to 21 to bind to its cognate antigen when being part of a nanobody, fusion protein, heavy chain antibody or bivalent construction as the ones of the invention. This capacity to bind to its cognate antigen may be determined as a value of affinity, avidity, specificity and/or selectivity of the antibody or antibody fragment to its cognate antigen.

The capacity of the binding agents according to the invention, and in particular of the antibody or antibody fragment as described herein, to bind to the RBD domain of S protein from SARS-Cov-2 can be determined by a number of assays that are well known in the art. Preferably, the binding capacity of the binding agents is determined by immunoprecipitation or by an *in vitro* binding assay, such as radioimmunoassay (RIA), enzyme-linked immunoabsorbent assay (ELISA), surface plasmon resonance or by immunofluorescent techniques such as immunohistochemistry (IHC), fluorescence microscopy or flow cytometry. The affinities of the nanobodies and heavy chains antibodies of the invention for the RBD domain of S protein and for the S protein are shown in Table 2. The affinity of a nanobody can be determined by methods know by the skilled person, such as those used in the examples herein included. In a particular embodiment, the affinity of the nanobody is determined by Surface Plasmon Resonance (SPR).

The variable domain of the nanobodies of the invention comprises framework regions. The term "framework region", as used herein, refers to the regions of the relatively preserved variable region of an antibody other than the regions of the CDR. In a particular embodiment, the variable domain comprises four framework regions: FR1 is located at the N-terminus of CDR1; FR2 is located between CDR2 and CDR3; FR3 is located between CDR3 and CDR4; FR4 is located at the C-terminus of CDR4. Said framework regions are know from the prior art. In a particular embodiment, the nanobodies of the invention comprise a variable domain comprising or consisting of the following sequences, which result from the combination of the above-mentioned sequences of the CDRs with particular framework regions as shown in Figure 1A:
(A) SEQ ID NO: 25 or a functionally equivalent variant thereof. As it can be appreciated, SEQ ID NO: 25 comprises a CDR1 of SEQ ID NO: 10, a CDR2 of SEQ ID NO: 11 and a CDR3 of SEQ ID NO: 12.
(B) SEQ ID NO: 22 or a functionally equivalent variant thereof. As it can be appreciated, SEQ ID NO: 22 comprises a CDR1 of SEQ ID NO: 1, a CDR2 of SEQ ID NO: 2 and a CDR3 of SEQ ID NO: 3.
(C) SEQ ID NO: 23 or a functionally equivalent variant thereof. As it can be appreciated, SEQ ID NO: 23 comprises a CDR1 of SEQ ID NO: 4, a CDR2 of SEQ ID NO: 5 and a CDR3 of SEQ ID NO: 6.
(D) SEQ ID NO: 24 or a functionally equivalent variant thereof. As it can be appreciated, SEQ ID NO: 24 comprises a CDR1 of SEQ ID NO: 7, a CDR2 of SEQ ID NO: 8 and a CDR3 of SEQ ID NO: 9.
(E) SEQ ID NO: 26 or a functionally equivalent variant thereof. As it can be appreciated, SEQ ID NO: 26 comprises a CDR1 of SEQ ID NO: 13, a CDR2 of SEQ ID NO: 14 and a CDR3 of SEQ ID NO: 15.
(F) SEQ ID NO: 27 or a functionally equivalent variant thereof. As it can be appreciated, SEQ ID NO: 27 comprises a CDR1 of SEQ ID NO: 16, a CDR2 of SEQ ID NO: 17 and a CDR3 of SEQ ID NO: 18.
(G)SEQ ID NO: 28 or a functionally equivalent variant thereof. As it can be appreciated, SEQ ID NO: 28 comprises a CDR1 of SEQ ID NO: 19, a CDR2 of SEQ ID NO: 20 and a CDR3 of SEQ ID NO: 21.

In a particular embodiment, the nanobodies of the invention comprise a variable domain comprising or consisting of a sequence selected from SEQ ID NO: 22-28. In a more particular embodiment, the nanobodies of the invention comprise a variable domain consisting of the amino acid sequence of:
- SEQ ID NO: 22. This nanobody is herein called Nb 1.10.
- SEQ ID NO: 23. This nanobody is herein called Nb 1.26.
- SEQ ID NO: 24. This nanobody is herein called Nb 1.28.
- SEQ ID NO: 25. This nanobody is herein called Nb 1.29.
- SEQ ID NO: 26. This nanobody is herein called Nb 2.1.
- SEQ ID NO: 27. This nanobody is herein called Nb 2.15.
- SEQ ID NO: 28. This nanobody is herein called Nb 2.20.

The equivalence between the name of the nanobodies and heavy chain antibodies used in the examples and the sequences of the variable domains and CDRs of said antibodies is shown in Table 1.

**Table 1: nanobodies and heavy chain antibodies of the invention. N.A. means not applicable**

| **Nanobody/hc antibody** | **CDRs SEQ ID NO** | **Variable regions SEQ ID NO** | **Humanized SEQ ID NO** |
|---|---|---|---|
| 1.29 | 10,11,12 | 25 | 31 |
| 1.10 | 1,2,3 | 22 | 29 |
| 1.26 | 4, 5, 6 | 23 | 30 |
| 1.28 | 7, 8, 9 | 24 | N.A. |
| 2.1 | 13, 14, 15 | 26 | N.A. |
| 2.15 | 16, 17, 18 | 27 | 32 |
| 2,20 | 19, 20, 21 | 28 | N.A. |

In another particular embodiment, the nanobodies of the invention comprise a variable region comprising or consisting of a functionally equivalent variant of a sequence selected from the group consisting of SEQ ID NO: 22-28. Functionally equivalent variants have already been defined for the variants of the CDR sequences. This definition applies mutatis mutandis to the variants of the variable domains of sequence SEQ ID NO: 22-28. In a particular embodiment, the nanobodies of the invention comprise a variable domain comprising or consisting of a functionally equivalent variant of any one of the sequences of SEQ ID NO: 22-28 having at least having at least approximately 70%, at least 75%, at least 80%, at least 85%, at least 90%, at least 91%, at least 92%, at least 93%, at least 94%, at least 95%, at least 96%, at least 97%, at least 98% or at least 99% sequence identity with the corresponding amino acid sequences shown in one of SEQ ID NOs: 22-28, preferably at least 80% sequence identity. In a more particular embodiment, the functionally equivalent variants of the variable region sequences according to the invention substantially maintain the capacity of the corresponding variable region sequence to bind to its cognate antigen when being part of a nanobody, fusion protein, heavy chain antibody or bivalent construction as the ones of the invention, preferably maintain at least 50%, at least 60%, at least 70%, at least 75%, at least 80%, at least 85%, at least 90%, at least 91%, at least 92%, at least 93%, at least 94%, at least 95%, at least 96%, at least 97%, at least 98%, at least 99%, at least 100%, at least 105%, at least 110%, at least 115%, at least 120%, at least 125%, at least 130%, at least 135%, at least 140%, at least 145%, at least 150%, at least 200% or more of the capacity of the corresponding amino acid sequence shown in one of SEQ ID NOs: 22 to 28 to bind to its cognate antigen when being part of a nanobody, fusion protein, heavy chain antibody or bivalent construction as the ones of the invention.

In a particular embodiment, functionally equivalent variants of the variable region sequences of the invention comprise conservative amino acid substitutions.

In a particular embodiment, the functionally equivalent variants of the variable region sequences of SEQ ID NO: 22-28 maintain the amino acid sequence of the CDRs, that is, they comprise the sequence of the CDRs included in the variable regions of SEQ ID NO: 22-28 and the amino acid substitutions, deletions or additions affect only the framework regions. Therefore, in a particular embodiment, the nanobodies of the invention comprise a variable region comprising:
(A) A functionally equivalent variant of the amino acid sequence of SEQ ID NO: 25, more particularly, a functionally equivalent variant having at least 80%, at least 85%, at least 90%, at least 95%, at least 96%, at least 97%, at least 98% or at least 99% sequence identity with SEQ ID NO: 25, wherein said variant comprises a CDR1 comprising or consisting of the amino acid sequence of SEQ ID NO: 10, a CDR2 comprising or consisting of the amino acid sequence of SEQ ID NO: 11 and a CDR3 comprising or consisting of the amino acid sequence of SEQ ID NO: 12.
(B) A functionally equivalent variant of the amino acid sequence of SEQ ID NO: 22, more particularly, a functionally equivalent variant having at least 80%, at least 85%, at least 90%, at least 95%, at least 96%, at least 97%, at least 98% or at least 99% sequence identity with SEQ ID NO: 22, wherein said variant comprises a CDR1 comprising or consisting of the amino acid sequence of SEQ ID NO: 1, a CDR2 comprising or consisting of the amino acid sequence of SEQ ID NO: 2 and a CDR3 comprising or consisting of the amino acid sequence of SEQ ID NO: 3.
(C) A functionally equivalent variant of the amino acid sequence of SEQ ID NO: 23, more particularly, a functionally equivalent variant having at least 80%, at least 85%, at least 90%, at least 95%, at least 96%, at least 97%, at least 98% or at least 99% sequence identity with SEQ ID NO: 23, wherein said variant comprises a CDR1 comprising or consisting of the amino acid sequence of SEQ ID NO: 4, a CDR2 comprising or consisting of the amino acid sequence of SEQ ID NO: 5 and a CDR3 comprising or consisting of the amino acid sequence of SEQ ID NO: 6.
(D) A functionally equivalent variant of the amino acid sequence of SEQ ID NO: 24, more particularly, a functionally equivalent variant having at least 80%, at least 85%, at least 90%, at least 95%, at least 96%, at least 97%, at least 98% or at least 99% sequence identity with SEQ ID NO: 24, wherein said variant comprises a CDR1 comprising or consisting of the amino acid sequence of SEQ ID NO: 7, a CDR2 comprising or consisting of the amino acid sequence of SEQ ID NO: 8 and a CDR3 comprising or consisting of the amino acid sequence of SEQ ID NO: 9.
(E) A functionally equivalent variant of the amino acid sequence of SEQ ID NO: 26, more particularly, a functionally equivalent variant having at least 80%, at least 85%, at least 90%, at least 95%, at least 96%, at least 97%, at least 98% or at least 99% sequence identity with SEQ ID NO: 26, wherein said variant comprises a CDR1 comprising or consisting of the amino acid sequence of SEQ ID NO: 13, a CDR2 comprising or consisting of the amino acid sequence of SEQ ID NO: 14 and a CDR3 comprising or consisting of the amino acid sequence of SEQ ID NO: 15.
(F) A functionally equivalent variant of the amino acid sequence of SEQ ID NO: 27, more particularly, a functionally equivalent variant having at least 80%, at least 85%, at least 90%, at least 95%, at least 96%, at least 97%, at least 98% or at least 99% sequence identity with SEQ ID NO: 27, wherein said variant comprises a CDR1 comprising or consisting of the amino acid sequence of SEQ ID NO: 16, a CDR2 comprising or consisting of the amino acid sequence of SEQ ID NO: 17 and a CDR3 comprising or consisting of the amino acid sequence of SEQ ID NO: 18.
(G) A functionally equivalent variant of the amino acid sequence of SEQ ID NO: 28, more particularly, a functionally equivalent variant having at least 80%, at least 85%, at least 90%, at least 95%, at least 96%, at least 97%, at least 98% or at least 99% sequence identity with SEQ ID NO: 28, wherein said variant comprises a CDR1 comprising or consisting of the amino acid sequence of SEQ ID NO: 19, a CDR2 comprising or consisting of the amino acid sequence of SEQ ID NO: 20 and a CDR3 comprising or consisting of the amino acid sequence of SEQ ID NO: 21.

In a particular embodiment, the nanobodies of the invention are humanized. The term "humanized", as used herein, refers to an antibody (or nanobody) derived from a non-human antibody, that retains the antigen-binding properties of the parent antibody, but which is less immunogenic in humans. This may be achieved by various methods, including (a) grafting the entire non-human variable domains onto human constant regions to generate chimeric antibodies; (b) grafting only the non-human complementarity determining regions (CDRs) into human framework and constant regions with or without retention of critical framework residues; and (c) transplanting the entire non-human variable domains, but "cloaking" them with a human-like section by replacement of surface residues. Methods for humanizing non-human antibodies have been described in the art. Preferably, a humanized antibody has one or more amino acid residues introduced into it from a source which is non-human. These non-human amino acid residues are often referred to as "import" residues, which are typically taken from an "import" variable domain. It is further important that antibodies are humanized with retention of high affinity for the antigen and other favorable biological properties. To achieve this goal, humanized antibodies are prepared by a process of analysis of the parental sequences and various conceptual humanized products using three-dimensional models of the parental and humanized sequences. A further step in this approach, to make an antibody more similar to humans, is to prepare the so called primatised antibodies, i.e. a recombinant antibody which has been engineered to contain the variable heavy and light domains of a monkey (or other primate) antibody, in particular, a cynomolgus monkey antibody, and which contains human constant domain sequences, preferably the human immunoglobulin gamma 1 or gamma 4 constant domain (or PE variant). In the context of the invention, humanized nanobodies can be obtained by replacing specific residue of the framework region of the camelid VHH domains by the most common amino acid found in the corresponding positions of human VH3 sequences. An example of such methodology can be found in the examples herein included.

In a particular embodiment, the humanized nanobodies of the invention comprise a variable region that is a functionally equivalent variant of any one of the variable domains comprising or consisting of the amino acid sequence of SEQ ID NO: 22-28. In a more particular embodiment, said humanized functionally equivalent variants comprise or consist of the sequences of SEQ ID NO: 29-32. In particular:
- SEQ ID NO: 31 corresponds to a humanized variant of SEQ ID NO: 25. As it can be appreciated, SEQ ID NO: 31 comprises the CDRs having the amino acid sequences of SEQ ID NO: 10, 11 and 12. Import residues have only been included in the framework regions and corresponds to amino acid positions 1, 11, 14, 44, 71, 74, 78, 86 and 87.
- SEQ ID NO: 29 corresponds to a humanized variant of SEQ ID NO: 22. As it can be appreciated, SEQ ID NO: 29 comprises the CDR having the amino acid sequences of SEQ ID NO: 1, 2 and 3. Import residues have only been included in the framework regions and corresponds to amino acid positions 1, 11, 14, 72, 87, 88 and 93.
- SEQ ID NO: 30 corresponds to a humanized variant of SEQ ID NO: 23. As it can be appreciated, SEQ ID NO: 30 comprises the CDR having the amino acid sequences of SEQ ID NO: 4, 5 and 6. Import residues have only been included in the framework regions and correspond to amino acid positions 1, 14, 44, 72, 80, 82, 87, 88 and 93.
- SEQ ID NO: 32 corresponds to a humanized variant of SEQ ID NO: 27. As it can be appreciated, SEQ ID NO: 32 comprises the CDR having the amino acid sequences of SEQ ID NO: 16, 17 and 18. Import residues have only been included in the framework regions and correspond to amino acid positions 1, 11, 14, 45, 62, 73, 80, 81, 88, 89 and 90.

Apart from the amino acid residues modified in the humanized variants of SEQ ID NO: 29-32, other amino acid residues can be replaced in the humanized variants.

The nanobodies of the invention can be obtained by any suitable technique known by the skilled person for production of recombinant proteins in general, and antibodies in particular. This techniques comprises introducing the nucleic acid or vector of the invention into a host cell, culturing said host cell under conditions suitable for the expression of the nanobody of the invention and recovered and purifying the nanobody from the host cell. In a particular embodiment, the nanobody of the invention can be obtained from a fusion protein comprising one or more nanobodies and an Fc region of a heavy chain (first fusion protein of the invention) or from a heavy chain antibody comprising two heavy chains, each heavy chain consisting of the first fusion protein of the invention (heavy chain antibody of the invention). In this particular embodiment, the fusion protein comprises a protease recognition site, for example a thrombin recognition site (LVPRGS) (SEQ ID NO: 34) between the nanobody and the Fc domain. In this embodiment, after purification of the fusion protein or heavy chain antibody, the nanobody is obtained by digestion with the protease that recognizes the protease recognition site, such as thrombin.

### Fusion proteins of the invention

In another aspect, the invention relates to a fusion protein, hereinafter "first fusion protein of the invention", comprising the nanobody of the invention and the Fc region of a heavy chain.

In another aspect, the invention refers to a fusion protein, hereinafter "second fusion protein of the invention", comprising construction comprising two nanobodies, wherein the first and the second nanobody are different, wherein
(i) the first nanobody is the nanobody of the invention and
(ii) the second nanobody comprises a variable domain selected from the group consisting of:
   (A) a variable domain comprising a CDR1 comprising the amino acid sequence shown in SEQ ID NO: 10, a CDR2 comprising the amino acid sequence shown in SEQ ID NO: 11 and a CDR3 comprising the amino acid sequence shown in SEQ ID NO: 12, or functionally equivalent variants of said CDRs, or
   (B) a variable domain comprising a CDR1 comprising the amino acid sequence shown in SEQ ID NO: 1, a CDR2 comprising the amino acid sequence shown in SEQ ID NO: 2 and a CDR3 comprising the amino acid sequence shown in SEQ ID NO: 3, or functionally equivalent variants of said CDRs, or
   (C) a CDR1 comprising the amino acid sequence shown in SEQ ID NO: 4, a CDR2 comprising the amino acid sequence shown in SEQ ID NO: 5 and a CDR3 comprising the amino acid sequence shown in SEQ ID NO: 6, or functionally equivalent variants of said CDRs, or
   (D) a CDR1 comprising the amino acid sequence shown in SEQ ID NO: 7, a CDR2 comprising the amino acid sequence shown in SEQ ID NO: 8 and a CDR3 comprising the amino acid sequence shown in SEQ ID NO: 9, or functionally equivalent variants of said CDRs, or
   (E) a CDR1 comprising the amino acid sequence shown in SEQ ID NO: 13, a CDR2 comprising the amino acid sequence shown in SEQ ID NO: 14 and a CDR3 comprising the amino acid sequence shown in SEQ ID NO: 15, or functionally equivalent variants of said CDRs, or
   (F) a CDR1 comprising the amino acid sequence shown in SEQ ID NO: 16, a CDR2 comprising the amino acid sequence shown in SEQ ID NO: 17 and a CDR3 comprising the amino acid sequence shown in SEQ ID NO: 18, or functionally equivalent variants of said CDRs, or
   (G) a CDR1 comprising the amino acid sequence shown in SEQ ID NO: 19, a CDR2 comprising the amino acid sequence shown in SEQ ID NO: 20 and a CDR3 comprising the amino acid sequence shown in SEQ ID NO: 21, or functionally equivalent variants of said CDRs.

The term "fusion protein", as used herein, refers to a single polypeptide chain artificially designed which comprises two or more sequences from different origins, natural and/or artificial. The fusion protein, per definition, is never found in nature as such. From the definition of the fusion protein itself, it derives that the different components of the fusion proteins of the invention are linked by peptide bonds and forming a single polypeptide chain.

As it is used herein, the term "heavy chain" or "HC" encompasses both a full length heavy chain and fragments thereof. A full length heavy chain includes a variable region domain, VH, and three constant region domains, CH1, CH2 and CH3. The VH domain is at the amino terminal end of the polypeptide, and the CH3 domain is at the carboxyl terminal end.

The term "fragment crystallisable region" or "Fc region refers to the tail region of an antibody that interacts with cell surface receptors called Fc receptors and some proteins of the complement system. In IgG, IgA and IgD antibody isotypes, the Fc region is composed of two identical protein fragments, derived from the second and third constant domains (CH2 and CH3) of the antibody's two heavy chains; IgM and IgE Fc regions contain three heavy chain constant domains (C_{H} domains 2-4) in each polypeptide chain.

The term "Fc region of a heavy chain", as used herein, refers to the C-terminal region of an immunoglobulin heavy chain, including native sequence Fc regions and variant Fc regions. The fusion proteins of the invention can comprise any Fc region, for example, the Fc region from antibody isotypes IgG, IgA, IgD, IgM or IgE.

In a particular embodiment, the Fc region of a heavy chain is the Fc region of an IgG, more particularly the Fc region of IgG1.

In a particular embodiment, the Fc region of a heavy chain is the Fc region of a heavy chain of a human antibody.

In a particular embodiment, the Fc region of a heavy chain is the Fc region of a human IgG, more particularly the Fc region of human IgG1.

In a particular embodiment, the first fusion protein of the invention comprises a protease recognition site, for example a thrombin recognition site (LVPRGS) (SEQ ID NO: 34) between the nanobody and the Fc domain.

In a particular embodiment, the first fusion protein of the invention comprises a second nanobody directed against RBD of spike protein from SARS-CoV-2, wherein said second nanobody is different to the first nanobody and wherein the second nanobody comprises a variable domain selected from the group consisting of:
(A) a variable domain comprising a CDR1 comprising or consisting of the amino acid sequence shown in SEQ ID NO: 10, a CDR2 comprising or consisting of the amino acid sequence shown in SEQ ID NO: 11 and a CDR3 comprising or consisting of the amino acid sequence shown in SEQ ID NO: 12, or functionally equivalent variants of said CDRs, or
(B) a variable domain comprising a CDR1 comprising or consisting of the amino acid sequence shown in SEQ ID NO: 1, a CDR2 comprising or consisting of the amino acid sequence shown in SEQ ID NO: 2 and a CDR3 comprising or consisting of the amino acid sequence shown in SEQ ID NO: 3, or functionally equivalent variants of said CDRs, or
(C) a CDR1 comprising or consisting of the amino acid sequence shown in SEQ ID NO: 4, a CDR2 comprising or consisting of the amino acid sequence shown in SEQ ID NO: 5 and a CDR3 comprising or consisting of the amino acid sequence shown in SEQ ID NO: 6, or functionally equivalent variants of said CDRs, or
(D) a CDR1 comprising or consisting of the amino acid sequence shown in SEQ ID NO: 7, a CDR2 comprising or consisting of the amino acid sequence shown in SEQ ID NO: 8 and a CDR3 comprising or consisting of the amino acid sequence shown in SEQ ID NO: 9, or functionally equivalent variants of said CDRs, or
(E) a CDR1 comprising or consisting of the amino acid sequence shown in SEQ ID NO: 13, a CDR2 comprising or consisting of the amino acid sequence shown in SEQ ID NO: 14 and a CDR3 comprising or consisting of the amino acid sequence shown in SEQ ID NO: 15, or functionally equivalent variants of said CDRs, or
(F) a CDR1 comprising or consisting of the amino acid sequence shown in SEQ ID NO: 16, a CDR2 comprising or consisting of the amino acid sequence shown in SEQ ID NO: 17 and a CDR3 comprising or consisting of the amino acid sequence shown in SEQ ID NO: 18, or functionally equivalent variants of said CDRs, or
(G) a CDR1 comprising or consisting of the amino acid sequence shown in SEQ ID NO: 19, a CDR2 comprising or consisting of the amino acid sequence shown in SEQ ID NO: 20 and a CDR3 comprising or consisting of the amino acid sequence shown in SEQ ID NO: 21, or functionally equivalent variants of said CDRs.

As the skilled person will understand, the first fusion protein of the invention comprising a second nanobody and the second fusion protein of the invention are bivalent since they comprise two nanobodies each of one has the ability to specifically bind to a different epitope of the RBD of S protein from SARS-CoV-2. The epitopes to which each of the nanobodies bind can partially overlap or not.

In a particular embodiment, the first and second fusion protein of the invention comprises a first nanobody comprising the CDRs comprising or consisting of the amino acid sequence of SEQ ID NO: 10, 11 and 12 or functionally equivalent variants of said CDRs and a second nanobody selected from the group consisting of:
(i) a nanobody comprising the CDRs comprising or consisting of the amino acid sequence of SEQ ID NO: 1, 2 and 3, or functionally equivalent variants of said CDRs;
(ii) a nanobody comprising the CDRs comprising or consisting of the amino acid sequence of SEQ ID NO: 4, 5 and 6, or functionally equivalent variants of said CDRs;
(iii) a nanobody comprising the CDRs comprising or consisting of the amino acid sequence of SEQ ID NO: 7, 8 and 9, or functionally equivalent variants of said CDRs;
(iv) a nanobody comprising the CDRs comprising or consisting of the amino acid sequence of SEQ ID NO: 13, 14 and 15, or functionally equivalent variants of said CDRs;
(v)a nanobody comprising the CDRs comprising or consisting of the amino acid sequence of SEQ ID NO: 16, 17 and 18, or functionally equivalent variants of said CDRs;
(vi) a nanobody comprising the CDRs comprising or consisting of the amino acid sequence of SEQ ID NO: 19, 20 and 21, or functionally equivalent variants of said CDRs.

In a particular embodiment, the first and fusion protein of the invention comprises a first nanobody comprising the CDRs comprising or consisting of the amino acid sequence of SEQ ID NO: 1, 2 and 3 or functionally equivalent variants of said CDRs and a second nanobody selected from the group consisting of:
(i) a nanobody comprising the CDRs comprising or consisting of the amino acid sequence of SEQ ID NO: 4, 5 and 6, or functionally equivalent variants of said CDRs;
(ii) a nanobody comprising the CDRs comprising or consisting of the amino acid sequence of SEQ ID NO: 7, 8 and 9, or functionally equivalent variants of said CDRs;
(iii) a nanobody comprising the CDRs comprising or consisting of the amino acid sequence of SEQ ID NO: 13, 14 and 15, or functionally equivalent variants of said CDRs;
(iv) a nanobody comprising the CDRs comprising or consisting of the amino acid sequence of SEQ ID NO: 16, 17 and 18, or functionally equivalent variants of said CDRs;
(v)a nanobody comprising the CDRs comprising or consisting of the amino acid sequence of SEQ ID NO: 19, 20 and 21, or functionally equivalent variants of said CDRs.

In a particular embodiment, the first and second fusion protein of the invention comprises a first nanobody comprising the CDRs comprising or consisting of the amino acid sequence of SEQ ID NO: 4, 5 and 6 or functionally equivalent variants of said CDRs and a second nanobody selected from the group consisting of:
(i) a nanobody comprising the CDRs comprising or consisting of the amino acid sequence of SEQ ID NO: 7, 8 and 9, or functionally equivalent variants of said CDRs;
(ii) a nanobody comprising the CDRs comprising or consisting of the amino acid sequence of SEQ ID NO: 13, 14 and 15, or functionally equivalent variants of said CDRs;
(iii) a nanobody comprising the CDRs comprising or consisting of the amino acid sequence of SEQ ID NO: 16, 17 and 18, or functionally equivalent variants of said CDRs;
(iv) a nanobody comprising the CDRs comprising or consisting of the amino acid sequence of SEQ ID NO: 19, 20 and 21, or functionally equivalent variants of said CDRs.

In a particular embodiment, the first and second fusion protein of the invention comprises a first nanobody comprising the CDRs comprising or consisting of the amino acid sequence of SEQ ID NO: 7, 8 and 9 or functionally equivalent variants of said CDRs and a second nanobody selected from the group consisting of:
(i) a nanobody comprising the CDRs comprising or consisting of the amino acid sequence of SEQ ID NO: 13, 14 and 15, or functionally equivalent variants of said CDRs;
(ii) a nanobody comprising the CDRs comprising or consisting of the amino acid sequence of SEQ ID NO: 16, 17 and 18, or functionally equivalent variants of said CDRs;
(iii) a nanobody comprising the CDRs comprising or consisting of the amino acid sequence of SEQ ID NO: 19, 20 and 21, or functionally equivalent variants of said CDRs.

In a particular embodiment, the first and second fusion protein of the invention comprises a first nanobody comprising the CDRs comprising or consisting of the amino acid sequence of SEQ ID NO: 13, 14 and 15 or functionally equivalent variants of said CDRs and a second nanobody selected from the group consisting of:
(i) a nanobody comprising the CDRs comprising or consisting of the amino acid sequence of SEQ ID NO: 16, 17 and 18, or functionally equivalent variants of said CDRs;
(ii) a nanobody comprising the CDRs comprising or consisting of the amino acid sequence of SEQ ID NO: 19, 20 and 21, or functionally equivalent variants of said CDRs.

In a particular embodiment, the first and second fusion protein of the invention comprises a first nanobody comprising the CDRs comprising or consisting of the amino acid sequence of SEQ ID NO: 16, 17 and 18 or functionally equivalent variants of said CDRs and a second nanobody comprising the CDRs comprising or consisting of the amino acid sequence of SEQ ID NO: 19, 20 and 21, or functionally equivalent variants of said CDRs.

The functionally equivalent variants of the CDRs have been previously defined in connection with the nanobodies of the invention. The particular and preferred embodiments regarding these variants fully apply to the fusion proteins of the invention.

In a particular embodiment, the first and/or second nanobody comprises a variable region comprising or consisting of the amino acid sequence of SEQ ID NO: 22-28 or a functionally equivalent variant thereof. The functionally equivalent variants of the variable regions have been previously defined in connection with the nanobodies of the invention. The particular and preferred embodiments regarding these variants fully apply to the fusion proteins of the invention.

In a particular embodiment, the first and second fusion protein of the invention comprises a first nanobody comprising a variable domain comprising or consisting of the amino acid sequence of SEQ ID NO: 25 or a functionally equivalent variant thereof, and a second nanobody selected from the group consisting of:
(i) a nanobody comprising a variable domain comprising or consisting of the amino acid sequence of SEQ ID NO: 22, or a functionally equivalent variant thereof;
(ii) a nanobody comprising a variable domain comprising or consisting of the amino acid sequence of SEQ ID NO: 23, or a functionally equivalent variant thereof;
(iii) a nanobody comprising a variable domain comprising or consisting of the amino acid sequence of SEQ ID NO: 24, or a functionally equivalent variant thereof;
(iv) a nanobody comprising a variable domain comprising or consisting of the amino acid sequence of SEQ ID NO: 26, or a functionally equivalent variant thereof;
(v) a nanobody comprising a variable domain comprising or consisting of the amino acid sequence of SEQ ID NO: 27, or a functionally equivalent variant thereof;
(vi) a nanobody comprising a variable domain comprising or consisting of the amino acid sequence of SEQ ID NO: 28, or a functionally equivalent variant thereof.

In a particular embodiment, the first and second fusion protein of the invention comprises a first nanobody comprising a variable domain comprising or consisting of the amino acid sequence of SEQ ID NO: 22 or a functionally equivalent variant thereof, and a second nanobody selected from the group consisting of:
(i) a nanobody comprising a variable domain comprising or consisting of the amino acid sequence of SEQ ID NO: 23, or a functionally equivalent variant thereof;
(ii) a nanobody comprising a variable domain comprising or consisting of the amino acid sequence of SEQ ID NO: 24, or a functionally equivalent variant thereof;
(iii) a nanobody comprising a variable domain comprising or consisting of the amino acid sequence of SEQ ID NO: 26, or a functionally equivalent variant thereof;
(iv) a nanobody comprising a variable domain comprising or consisting of the amino acid sequence of SEQ ID NO: 27, or a functionally equivalent variant thereof;
(v) a nanobody comprising a variable domain comprising or consisting of the amino acid sequence of SEQ ID NO: 28, or a functionally equivalent variant thereof.

In a particular embodiment, the first and second fusion protein of the invention comprises a first nanobody comprising a variable domain comprising or consisting of the amino acid sequence of SEQ ID NO: 23 or a functionally equivalent variant thereof, and a second nanobody selected from the group consisting of:
(i) a nanobody comprising a variable domain comprising or consisting of the amino acid sequence of SEQ ID NO: 24, or a functionally equivalent variant thereof;
(ii) a nanobody comprising a variable domain comprising or consisting of the amino acid sequence of SEQ ID NO: 26, or a functionally equivalent variant thereof;
(iii) a nanobody comprising a variable domain comprising or consisting of the amino acid sequence of SEQ ID NO: 27, or a functionally equivalent variant thereof;
(iv) a nanobody comprising a variable domain comprising or consisting of the amino acid sequence of SEQ ID NO: 28, or a functionally equivalent variant thereof.

In a particular embodiment, the first and second fusion protein of the invention comprises a first nanobody comprising a variable domain comprising or consisting of the amino acid sequence of SEQ ID NO: 24 or a functionally equivalent variant thereof, and a second nanobody selected from the group consisting of:
(i) a nanobody comprising a variable domain comprising or consisting of the amino acid sequence of SEQ ID NO: 26, or a functionally equivalent variant thereof;
(ii) a nanobody comprising a variable domain comprising or consisting of the amino acid sequence of SEQ ID NO: 27, or a functionally equivalent variant thereof;
(iii) a nanobody comprising a variable domain comprising or consisting of the amino acid sequence of SEQ ID NO: 28, or a functionally equivalent variant thereof.

In a particular embodiment, the first and second fusion protein of the invention comprises a first nanobody comprising a variable domain comprising or consisting of the amino acid sequence of SEQ ID NO: 26 or a functionally equivalent variant thereof, and a second nanobody selected from the group consisting of:
(i) a nanobody comprising a variable domain comprising or consisting of the amino acid sequence of SEQ ID NO: 27, or a functionally equivalent variant thereof;
(ii) a nanobody comprising a variable domain comprising or consisting of the amino acid sequence of SEQ ID NO: 28, or a functionally equivalent variant thereof.

In a particular embodiment, the first and second fusion protein of the invention comprises a first nanobody comprising a variable domain comprising or consisting of the amino acid sequence of SEQ ID NO: 27 or a functionally equivalent variant thereof, and a second nanobody comprising a variable domain comprising or consisting of the amino acid sequence of SEQ ID NO: 28, or a functionally equivalent variant thereof.

In a particular embodiment, the functionally equivalent variant of the variable region of the first and or second nanobody have at least 80%, at least 85%, at least 90%, at least 95%, at least 96%, at least 97%, at least 98% or at least 99% or more sequence identity with any one of the variable region sequences of SEQ ID NO: 22-28.

In a particular embodiment, the functionally equivalent variants of the variable region sequences of SEQ ID NO: 22-28 of the first and or second nanobody comprise the sequence of the CDRs included in the variable regions of SEQ ID NO: 22-28 and the amino acid substitutions, deletions or additions affect only the framework regions, as previously detailed for the nanobodies of the invention.

In a particular embodiment, both the first and second nanobodies are humanized. In a particular embodiment, the humanized nanobodies comprised in the fusion proteins of the invention comprise a variable region that is a functionally equivalent variant of any one of the variable domains comprising or consisting of the amino acid sequence of SEQ ID NO: 22-28. In a more particular embodiment, said humanized functionally equivalent variants comprise or consist of the sequences of SEQ ID NO: 29-32.

In a particular embodiment, the first and second fusion protein of the invention comprises a first nanobody comprising a variable domain comprising or consisting of the amino acid sequence of SEQ ID NO: 31 or a functionally equivalent variant thereof, and a second nanobody selected from the group consisting of:
(i) a nanobody comprising a variable domain comprising or consisting of the amino acid sequence of SEQ ID NO: 29, or a functionally equivalent variant thereof;
(ii) a nanobody comprising a variable domain comprising or consisting of the amino acid sequence of SEQ ID NO: 30, or a functionally equivalent variant thereof;
(iii) a nanobody comprising a variable domain comprising or consisting of the amino acid sequence of SEQ ID NO: 32, or a functionally equivalent variant thereof.

In a particular embodiment, the first and second fusion protein of the invention comprises a first nanobody comprising a variable domain comprising or consisting of the amino acid sequence of SEQ ID NO: 29 or a functionally equivalent variant thereof, and a second nanobody selected from the group consisting of:
(i) a nanobody comprising a variable domain comprising or consisting of the amino acid sequence of SEQ ID NO: 30, or a functionally equivalent variant thereof;
(ii) a nanobody comprising a variable domain comprising or consisting of the amino acid sequence of SEQ ID NO: 32, or a functionally equivalent variant thereof.

In a particular embodiment, the first and second fusion protein of the invention comprises a first nanobody comprising a variable domain comprising or consisting of the amino acid sequence of SEQ ID NO: 30 or a functionally equivalent variant thereof, and a second nanobody comprising a variable domain comprising or consisting of the amino acid sequence of SEQ ID NO: 32, or a functionally equivalent variant thereof.

Without wanting to be found to any particular theory, it is thought that the nanobodies of the invention bind to two different non-overlapping epitopes which allows the classification of these nanobodies into two classes:
- Class A: antibodies 1.10, 1.26, 1.28, 2.1, 2.15 and
- Class B: 1.29 and 2.20 nanobodies.

Therefore, particularly advantageous bivalent fusion proteins of the invention are those comprising two nanobodies binding to different non-overlapping epitopes, in particular:
- a first nanobody selected from the go the group consisting of:
   (a) a nanobody comprising a variable domain comprising a CDR1 comprising or consisting of the amino acid sequence shown in SEQ ID NO: 10, a CDR2 comprising or consisting of the amino acid sequence shown in SEQ ID NO: 11 and a CDR3 comprising or consisting of the amino acid sequence shown in SEQ ID NO: 12, or functionally equivalent variants of said CDRs ; and
   (b) a nanobody comprising a variable domain comprising a CDR1 comprising or consisting of the amino acid sequence shown in SEQ ID NO: 19, a CDR2 comprising or consisting of the amino acid sequence shown in SEQ ID NO: 20 and a CDR3 comprising or consisting of the amino acid sequence shown in SEQ ID NO: 21, or functionally equivalent variants of said CDRs; and
- a second nanobody selected from the group consisting of:
   (a) a nanobody comprising a variable domain comprising a CDR1 comprising or consisting of the amino acid sequence shown in SEQ ID NO: 1, a CDR2 comprising or consisting of the amino acid sequence shown in SEQ ID NO: 2 and a CDR3 comprising or consisting of the amino acid sequence shown in SEQ ID NO: 3, or functionally equivalent variants of said CDRs;
   (b) a nanobody comprising a variable domain comprising a CDR1 comprising or consisting of the amino acid sequence shown in SEQ ID NO: 4, a CDR2 comprising or consisting of the amino acid sequence shown in SEQ ID NO: 5 and a CDR3 comprising or consisting of the amino acid sequence shown in SEQ ID NO: 6, or functionally equivalent variants of said CDRs;
   (c) a nanobody comprising a variable domain comprising a CDR1 comprising or consisting of the amino acid sequence shown in SEQ ID NO: 7, a CDR2 comprising or consisting of the amino acid sequence shown in SEQ ID NO: 8 and a CDR3 comprising or consisting of the amino acid sequence shown in SEQ ID NO: 9, or functionally equivalent variants of said CDRs;
   (d) a nanobody comprising a variable domain comprising a CDR1 comprising or consisting of the amino acid sequence shown in SEQ ID NO: 13, a CDR2 comprising or consisting of the amino acid sequence shown in SEQ ID NO: 14 and a CDR3 comprising or consisting of the amino acid sequence shown in SEQ ID NO: 15, or functionally equivalent variants of said CDRs and
   (e) a nanobody comprising a variable domain comprising a CDR1 comprising or consisting of the amino acid sequence shown in SEQ ID NO: 16, a CDR2 comprising or consisting of the amino acid sequence shown in SEQ ID NO: 17 and a CDR3 comprising or consisting of the amino acid sequence shown in SEQ ID NO: 18, or functionally equivalent variants of said CDRs.

Even more particularly advantageous bivalent fusion proteins of the invention are those comprising:
- a first nanobody comprising a variable domain comprising a CDR1 comprising or consisting of the amino acid sequence shown in SEQ ID NO: 10, a CDR2 comprising or consisting of the amino acid sequence shown in SEQ ID NO: 11 and a CDR3 comprising or consisting of the amino acid sequence shown in SEQ ID NO: 12, or functionally equivalent variants of said CDRs and a second nanobody comprising a variable domain comprising a CDR1 comprising or consisting of the amino acid sequence shown in SEQ ID NO: 1, a CDR2 comprising or consisting of the amino acid sequence shown in SEQ ID NO: 2 and a CDR3 comprising or consisting of the amino acid sequence shown in SEQ ID NO: 3, or functionally equivalent variants of said CDRs;
- a first nanobody comprising a variable domain comprising a CDR1 comprising or consisting of the amino acid sequence shown in SEQ ID NO: 10, a CDR2 comprising or consisting of the amino acid sequence shown in SEQ ID NO: 11 and a CDR3 comprising or consisting of the amino acid sequence shown in SEQ ID NO: 12, or functionally equivalent variants of said CDRs and a second nanobody comprising a variable domain comprising a CDR1 comprising or consisting of the amino acid sequence shown in SEQ ID NO: 4, a CDR2 comprising or consisting of the amino acid sequence shown in SEQ ID NO: 5 and a CDR3 comprising or consisting of the amino acid sequence shown in SEQ ID NO: 6, or functionally equivalent variants of said CDRs.
- a first nanobody comprising a variable domain comprising or consisting of the amino acid sequence of SEQ ID NO: 25 or a functionally equivalent variant thereof and a second nanobody comprising a variable domain comprising or consisting of the amino acid sequence of SEQ ID NO: 22 or a functionally equivalent variant thereof;
- a first nanobody comprising a variable domain comprising or consisting of the amino acid sequence of SEQ ID NO: 25 or a functionally equivalent variant thereof and a second nanobody comprising a variable domain comprising or consisting of the amino acid sequence of SEQ ID NO: 23 or a functionally equivalent variant thereof.

In a particular embodiment, when the fusion protein comprises two nanobodies, the first and the second nanobody can be linked by a peptide linker. The term "peptide linker", as used herein, refers to a peptide that connects two molecules and that frequently allows connected molecules to acquire a functional configuration. Without wanting to be bound to any particular theory, the peptide linker can increase the structural flexibility of the fusion protein comprising two nanobodies facilitating their binding to their epitopes. The linker peptide preferably comprises at least two amino acids, at least three amino acids, at least five amino acids, at least 6 amino acid, at least 10 amino acids, at least 15 amino acids, at least 20 amino acids or more. In a particular embodiment, the peptide linker comprises repetitions of Gly and Ser. In a more particular embodiment, the peptide linker comprises or consists of the sequence of SEQ ID NO: 33.

In a particular embodiment, when the fusion protein comprises just one nanobody, the fusion protein can comprise a detectable marker associated with said nanobody. In a particular embodiment, when the fusion protein comprises two nanobodies, the fusion protein can comprise a detectable marker associated with the first nanobody or with the second nanobody or with both nanobodies. The term "detectable marker", as used herein, refers to a functional group, moiety or residue that allows for detection of the nanobody and/or fusion protein. When the detectable marker is a peptide or protein it can form part of the fusion proteins of the invention. In a particular embodiment the detectable marker is biotin. The skilled person knows methods for the biotinylation of proteins in general and antibodies in particular. The biotinylated fusion protein can be detected via anti-biotin antibodies or avidin/streptavidin-tagged detection strategies such as enzyme reporters (e.g., horseradish peroxidase, alkaline phosphatase) or fluorescent probes.

In a particular embodiment, the first fusion proteins of the invention comprise the following elements, ordered from its N-terminus to its C-terminus:
(i) the nanobody of the invention and the Fc region of a heavy chain; or
(ii) the first nanobody, the second nanobody and the Fc region of a heavy chain; or
(iii) the second nanobody, the first nanobody and the Fc region of a heavy chain.

In a particular embodiment, the second fusion proteins of the invention comprise the following elements, ordered from its N-terminus to its C-terminus:
(i) the first nanobody of the invention and the second nanobody or
(ii) the second nanobody and the first nanobody.

### Heavy chain antibodies of the invention

In another aspect, the invention relates to a heavy chain antibody comprising two heavy chains, wherein each heavy chain comprises the fusion protein of the invention, and wherein both heavy chains are identical.

The term "heavy chain antibody" as used herein, refers to an antibody which consists only of two heavy chains and lacks the two light chains usually found in antibodies. Examples of heavy chain antibodies include the immunoglobulin new antigen receptor (IgNAR) of cartilaginous fishes, such as sharks, and the camelid antibody expressed in camelids, such as dromedaries, camels, llamas and alpacas. IgNARs have five constant domains (CH) per chain instead of the usual three, several disulfide bonds in unusual positions, and the CDR3 forms an extended loop covering the site which binds to a light chain in other antibodies. The heavy chains of the camelid antibodies have lost one of their constant domains (CH1) and underwent modifications in the variable domain (VH), both structural elements necessary for the binding of light chains.

The two heavy chains of the heavy chain antibodies of the invention are identical and correspond to the fusion proteins of the invention. Therefore, the heavy chain antibodies of the invention can be monovalent, if they just comprise one nanobody, or bivalent, if they comprise two nanobodies. All the particular and preferred embodiments previously explained for the fusion proteins of the invention, including nanobodies, sequences of the CDRs and variable regions, functionally equivalent variants, etc., fully apply to the heavy chain antibodies of the invention.

Particularly advantageous bispecific heavy chain antibodies of the invention are those comprising two nanobodies binding to different non-overlapping epitopes, in particular:
- a first nanobody selected from the go the group consisting of:
   (a) a nanobody comprising a variable domain comprising a CDR1 comprising or consisting of the amino acid sequence shown in SEQ ID NO: 10, a CDR2 comprising or consisting of the amino acid sequence shown in SEQ ID NO: 11 and a CDR3 comprising or consisting of the amino acid sequence shown in SEQ ID NO: 12, or functionally equivalent variants of said CDRs ; and
   (b) a nanobody comprising a variable domain comprising a CDR1 comprising or consisting of the amino acid sequence shown in SEQ ID NO: 19, a CDR2 comprising or consisting of the amino acid sequence shown in SEQ ID NO: 20 and a CDR3 comprising or consisting of the amino acid sequence shown in SEQ ID NO: 21, or functionally equivalent variants of said CDRs; and
- a second nanobody selected from the group consisting of:
   - a nanobody comprising a variable domain comprising a CDR1 comprising or consisting of the amino acid sequence shown in SEQ ID NO: 1, a CDR2 comprising or consisting of the amino acid sequence shown in SEQ ID NO: 2 and a CDR3 comprising or consisting of the amino acid sequence shown in SEQ ID NO: 3, or functionally equivalent variants of said CDRs;
   - a nanobody comprising a variable domain comprising a CDR1 comprising or consisting of the amino acid sequence shown in SEQ ID NO: 4, a CDR2 comprising or consisting of the amino acid sequence shown in SEQ ID NO: 5 and a CDR3 comprising or consisting of the amino acid sequence shown in SEQ ID NO: 6, or functionally equivalent variants of said CDRs;
   - a nanobody comprising a variable domain comprising a CDR1 comprising or consisting of the amino acid sequence shown in SEQ ID NO: 7, a CDR2 comprising or consisting of the amino acid sequence shown in SEQ ID NO: 8 and a CDR3 comprising or consisting of the amino acid sequence shown in SEQ ID NO: 9, or functionally equivalent variants of said CDRs;
   - a nanobody comprising a variable domain comprising a CDR1 comprising or consisting of the amino acid sequence shown in SEQ ID NO: 13, a CDR2 comprising or consisting of the amino acid sequence shown in SEQ ID NO: 14 and a CDR3 comprising or consisting of the amino acid sequence shown in SEQ ID NO: 15, or functionally equivalent variants of said CDRs and
   - a nanobody comprising a variable domain comprising a CDR1 comprising or consisting of the amino acid sequence shown in SEQ ID NO: 16, a CDR2 comprising or consisting of the amino acid sequence shown in SEQ ID NO: 17 and a CDR3 comprising or consisting of the amino acid sequence shown in SEQ ID NO: 18, or functionally equivalent variants of said CDRs.

Even more particularly advantageous bispecific heavy chain antibodies of the invention are those comprising:
- a first nanobody comprising a variable domain comprising a CDR1 comprising or consisting of the amino acid sequence shown in SEQ ID NO: 10, a CDR2 comprising or consisting of the amino acid sequence shown in SEQ ID NO: 11 and a CDR3 comprising or consisting of the amino acid sequence shown in SEQ ID NO: 12, or functionally equivalent variants of said CDRs and a second nanobody comprising a variable domain comprising a CDR1 comprising or consisting of the amino acid sequence shown in SEQ ID NO: 1, a CDR2 comprising or consisting of the amino acid sequence shown in SEQ ID NO: 2 and a CDR3 comprising or consisting of the amino acid sequence shown in SEQ ID NO: 3, or functionally equivalent variants of said CDRs;
- a first nanobody comprising a variable domain comprising a CDR1 comprising or consisting of the amino acid sequence shown in SEQ ID NO: 10, a CDR2 comprising or consisting of the amino acid sequence shown in SEQ ID NO: 11 and a CDR3 comprising or consisting of the amino acid sequence shown in SEQ ID NO: 12, or functionally equivalent variants of said CDRs and a second nanobody comprising a variable domain comprising a CDR1 comprising or consisting of the amino acid sequence shown in SEQ ID NO: 4, a CDR2 comprising or consisting of the amino acid sequence shown in SEQ ID NO: 5 and a CDR3 comprising or consisting of the amino acid sequence shown in SEQ ID NO: 6, or functionally equivalent variants of said CDRs.
- a first nanobody comprising a variable domain comprising or consisting of the amino acid sequence of SEQ ID NO: 25 or a functionally equivalent variant thereof and a second nanobody comprising a variable domain comprising or consisting of the amino acid sequence of SEQ ID NO: 22 or a functionally equivalent variant thereof;
- a first nanobody comprising a variable domain comprising or consisting of the amino acid sequence of SEQ ID NO: 25 or a functionally equivalent variant thereof and a second nanobody comprising a variable domain comprising or consisting of the amino acid sequence of SEQ ID NO: 23 or a functionally equivalent variant thereof.

### Nucleic acids, vectors and host cells of the invention

In another aspect, the invention relates to a nucleic acid encoding the nanobody of the invention or the first or second fusion protein of the invention.

The term "nucleic acid", as used herein, relates to a deoxyribonucleotide or ribonucleotide polymer in either single or double stranded form and, unless otherwise limited, encompasses natural nucleotides and analogues of natural nucleotides that hybridize to nucleic acids in a manner similar to naturally occurring nucleotides. The term "nucleotide" includes, but is not limited to, a monomer that includes a base (such as a pyrimidine, purine or synthetic analogs thereof) linked to a sugar (such as ribose, deoxyribose or synthetic analogs thereof), or a base linked to an amino acid, as in a peptide nucleic acid (PNA). A nucleotide is one monomer in an oligonucleotide or in a polynucleotide. A "nucleotide sequence" or "nucleic acid sequence" refers to the sequence of bases in an oligonucleotide or in a polynucleotide.

Different hosts often have preferences for a particular codon to be used for encoding a particular amino acid residue. Such codon preferences are well known and a DNA sequence encoding a desired fusion protein sequence can be altered, using *in vitro* mutagenesis for example, so that host-preferred codons are utilized for a particular host in which the fusion protein is to be expressed.

In a particular embodiment, the nucleic acid further comprises a sequence encoding a signal peptide fused in frame at the 5' terminus. The term "signal peptide", as used herein, also known as signal, localization signal, localization sequence, transit peptide, leader sequence or leader peptide refers to a short peptide present at the N-terminus of the majority of newly synthesized proteins that are destined towards the secretory pathway. "In frame" or "operatively linked", as used herein, means that the nucleic acid of the invention and the signal peptide are expressed in the correct reading frame under control of the expression control or regulating sequences.

In another aspect, the invention relates to a vector comprising the nucleic acid of the invention.

The term "vector", as used herein, refers to a nucleic acid sequence comprising the necessary sequences so that after transcribing and translating said sequences in a cell the first or second polypeptide of the invention is generated. Said sequence is operably linked to additional segments that provide for its autonomous replication in a host cell of interest. Preferably, the vector is an expression vector, which is defined as a vector, which in addition to the regions of the autonomous replication in a host cell, contains regions operably linked to the nucleic acid of the invention and which are capable of enhancing the expression of the products of the nucleic acid according to the invention. The vectors of the invention can be obtained by means of techniques widely known in the art.

Any vector containing a host-compatible promoter, origin of replication and termination sequences is suitable. By way of illustration, said suitable expression systems or vectors can be chosen according to the conditions and needs of each specific case, from said plasmids, bacmids, yeast artificial chromosomes (YACs), bacteria artificial chromosomes (BACs), bacteriophage P1-based artificial chromosomes (PACs), cosmids, or viruses, which can further have a heterologous replication origin, for example a bacterial or yeast origin, so that it can be amplified into bacteria or yeasts, as well as a marker that can be used to select the transfected cells that are different from the gene or genes of interest. These recombinant vectors can be obtained by persons skilled in the art by means of using conventional genetic engineering.

Vectors may further contain one or more selectable marker sequences suitable for use in the identification of cells which have or have not been transformed or transfected with the vector. Markers include, for example, genes encoding proteins which increase or decrease either resistance or sensitivity to antibiotics or other compounds (e.g. hyg encoding hygromycin resistance), genes which encode enzymes whose activities are detectable by standard assays known in the art (e.g. β-galactosidase or luciferase), and genes which visibly affect the phenotype of transformed or transfected cells, hosts, colonies or plaques such as various fluorescent proteins (e.g. green fluorescent protein, GFP). Alternatively, the vectors of the present invention may carry a non-antibiotic selection marker, including, for instance, genes encoding a catabolic enzyme which enables the growth in medium containing a substrate of said catabolic enzyme as a carbon source. An example of such a catabolic enzyme includes, but is not restricted to, lacYZ encoding lactose uptake and beta-galactosidase. Other selection markers that provide a metabolic advantage in defined media include, but are not restricted to, galTK for galactose utilization, sacPA for sucrose utilization, trePAR for trehalose utilization and xylAB for xylose utilization. Alternatively, the selection can involve the use of antisense mRNA to inhibit a toxic allele, for instance the sacB allele.

In a particular embodiment the vector is vector for expression in mammalian cells.

In another aspect, the invention relates to a host cell comprising the nucleic acid of the invention or the vector of the invention.

The term "host cell", as used herein, refers to a cell into which a nucleic acid of the invention, such as a polynucleotide or a vector according to the invention, has been introduced and is capable of expressing the polynucleotides of the invention. The terms "host cell" and "recombinant host cell" are used interchangeably herein. It should be understood that such terms refer not only to the particular subject cell but to the progeny or potential progeny of such a cell. Because certain modifications may occur in succeeding generations due to either mutation or environmental influences, such progeny may not, in fact, be identical to the parent cell, but are still included within the scope of the term as used herein.

Host cells suitable for the expression of the nucleic acid or vector of the invention include, without being limited thereto, cells from bacteria, fungi, plants, insects and mammals. Bacterial cells include, without being limited thereto, cells from Gram-positive bacteria, such as species from the genera Bacillus, Streptomyces and *Staphylococcus,* and cells from Gram-negative bacteria, such as cells from the genera *Escherichia* and *Pseudomonas.* Fungi cells preferably include cells from yeasts such as *Saccharomyces, Pichia pastoris* and *Hansenula polymorpha.* Insect cells include, without limitation, *Drosophila* cells and Sf9 cells. Plant cells include, amongst others, cells from cultivated plants, such as cereals, medicinal plants, ornamental plants or bulbs. Mammalian cells suitable for this invention include epithelial cell lines (porcine, etc.), osteosarcoma cell lines (human, etc.), neuroblastoma cell lines (human, etc.), epithelial carcinomas (human, etc.), glial cells (murine, etc.), hepatic cell lines (from monkeys, etc.), CHO (Chinese Hamster Ovary) cells, COS cells, BHK cells, HeLa, 911, AT1080, A549, 293 or PER.C6 cells, human NTERA-2 ECC cells, D3 cells from the mESC line, human embryonary stem cells, such as HS293 and BGV01, SHEF1, SHEF2 and HS181, NIH3T3, 293T, REH and MCF-7 cells, and hMSC cells.

In a preferred embodiment, the host cell is a cell capable of producing the extracellular production of the fusion protein of the invention.

In a preferred embodiment the host cell is a mammalian cell, such as human embryonic kidney (HEK-293F) mammalian cell.

### Pharmaceutical composition of the invention

In another aspect, the invention relates to a pharmaceutical composition comprising the nanobody of the invention, the first or second fusion protein of the invention, or the heavy chain antibody of the invention, and a pharmaceutically acceptable excipient or carrier.

The nanobody, fusion protein and heavy chain antibody of the invention have been previously defined. All the particular and preferred embodiments regarding said antibody, fusion protein or heavy chain antibody fully apply to the pharmaceutical.

The term "pharmaceutically acceptable carrier", as used herein, is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as TWEEN^{™}, PLURONlCS^{™} or polyethylene glycol (PEG).

In a particular embodiment, the route of administration of the pharmaceutical composition of the invention is intranasal or parenteral.

The term "parenteral" as used herein includes intravenous, intraperitoneal, intramuscular, subcutaneous, rectal or vaginal administration. The intravenous form of parenteral administration is generally preferred. In addition, the pharmaceutical composition of the invention may suitably be administered by pulse infusion, e.g., with declining doses. Preferably the dosing is given by injections, most preferably intravenous or subcutaneous injections, depending in part on whether the administration is brief or chronic.

In a preferred embodiment, the pharmaceutical compositions of the invention may be adapted for parenteral administration, such as sterile solutions, suspensions or lyophilized products in the appropriate unit dosage form.

It is especially advantageous to formulate the pharmaceutical compositions, in dosage unit form for ease administration and uniformity of dosage. Dosage unit form as used herein refers to physically discrete units suited as unitary dosages for the subject to be treated; each unit containing a predetermined quantity of active compound (e.g., the nanobody, fusion protein or heavy chain antibody) calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Generally an effective administered amount of a nanobody, fusion protein or heavy chain antibody of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.001 to 1,000 mg/kg body weight/day, preferably about 0.01 to about 100 mg/kg body weight/day, most preferably from about 0.05 to 10 mg/kg body weight/day. In a particular embodiment, the pharmaceutical composition is administered just once at a dose of 8 mg/kg body weight/day. These doses are those administered to mice. The present invention covers said doses as well as the equivalent doses and concentrations in humans. The skilled person knows how to convert mouse doses into human doses, for instance following the Human Equivalent Dose Calculations established in the FDA's "Guidance for Industry - Estimating the Maximum Safe Starting Dose in Initial Clinical Trials for Therapeutics in Adult Healthy Volunteers" of July 2005. The physician will determine the most suitable dosage regime of the compounds and this will vary with the dosage form and the particular compound that is chosen, and it will furthermore vary with the patient undergoing treatment, e.g. with the age or medical history of the patient, or with the type of disease or condition that it being treated.

The pharmaceutical compositions can be included in a container, pack, or dispenser together with instructions for administration.

### Medical uses of the invention

In another aspect, the invention relates to the nanobody, first or second fusion protein or heavy chain antibody of the invention for use as a medicament.

Alternatively, the invention relates to the use of the nanobody, first or second fusion protein or heavy chain antibody of the invention for the manufacture of a medicament.

In another aspect, the invention refers to the nanobody, first or second fusion protein or heavy chain antibody of the invention for use in the treatment and/or prevention of an infection caused by severe acute respiratory syndrome coronavirus 2 (SARS-CoV-2).

Alternatively, the invention relates to the use of the nanobody, first or second fusion protein or heavy chain antibody of the invention for the manufacture of a medicament for the treatment and/or prevention of an infection caused by severe acute respiratory syndrome coronavirus 2 (SARS-CoV-2).

Alternatively, the invention relates to a method for treating and/or preventing an infection caused by severe acute respiratory syndrome coronavirus 2 (SARS-CoV-2) in a subject comprising administering to said subject a therapeutically effective amount of the nanobody, first or second fusion protein or heavy chain antibody of the invention.

The nanobody, fusion protein and heavy chain antibody of the invention have been previously defined. All the particular and preferred embodiments regarding said antibody, fusion protein or heavy chain antibody fully apply to the pharmaceutical.

In a particular embodiment, the nanobody comprises a variable domain comprising or consisting of the amino acid sequence of SEQ ID NO: 22-28 or a functionally equivalent variant thereof. In a more particular embodiment, said functionally equivalent variant has at least 80%, at least 85%, at least 96%, at least 97%, at least 98% or at least 90%, at least 95%, at least 98%, at least 99% or more sequence identity with any one of SEQ ID NO: 22-28. In an even more particular embodiment, the functionally equivalent variants of the variable region sequences of SEQ ID NO: 22-28 comprise the sequence of the CDRs included in the variable regions of SEQ ID NO: 22-28 and the amino acid substitutions, deletions or additions affect only the framework regions, as previously detailed for the nanobodies of the invention.

In a particular embodiment, both nanobodies are humanized. In a particular embodiment, the humanized nanobodies comprise a variable region that is a functionally equivalent variant of any one of the variable domains comprising or consisting of the amino acid sequence of SEQ ID NO: 22-28. In a more particular embodiment, said humanized functionally equivalent variants comprise or consist of the sequences of SEQ ID NO: 29-32.

The term "subject", as used herein, refers to all animals classified as mammals and includes, without limitation, domestic and farm animals, primates and humans, e.g., human beings, non-human primates, cows, horses, pigs, sheep, goats, dogs, cats, or rodents. Preferably, the subject is a male or female human of any age or race.

The term "treatment", as used herein, refers to any type of therapy, which is aimed at terminating, preventing, ameliorating or reducing the susceptibility to a clinical condition or existing disease as described herein, including complete curing of a disease as well as amelioration or alleviation of said disease. In a preferred embodiment, the term treatment relates to prophylactic treatment (i.e. a therapy to reduce the susceptibility to a clinical condition), of a disorder or a condition as defined herein. Thus, "treatment," "treating," and their equivalent terms refer to obtaining a desired pharmacologic or physiologic effect, covering any treatment of a pathological condition or disorder in a mammal, including a human. The effect may be prophylactic in terms of completely or partially preventing a disorder or symptom thereof and/or may be therapeutic in terms of a partial or complete cure for a disorder and/or adverse effect attributable to the disorder. That is, "treatment" includes (1) preventing the disorder from occurring or recurring in a subject, (2) inhibiting the disorder, such as arresting its development, (3) stopping or terminating the disorder or, at least, clinical signs associated therewith, so that the host no longer suffers from the disorder or its clinical signs, such as causing regression of the disorder or its clinical signs, for example, by restoring or repairing a lost, missing or defective function, or stimulating an inefficient process, or (4) relieving, alleviating, or ameliorating the disorder, or clinical signs associated therewith, where ameliorating in the magnitude of a parameter, such as inflammation, pain, immune deficiency, reduction of the pathogen load, the pathogen shedding, or reduction in pathogen transmission.

The term "prevention" or "reduction" or "preventing" or "reducing", respectively, as used herein, means, but is not limited to a process of prophylaxis in which a subject is exposed to the nanobody, fusion protein or heavy chain antibody of the invention prior to the induction or onset of the disease process, and wherein said nanobody, fusion protein or heavy chain antibody, when administered to said subject, is able to prevent, cure, delay or reduce or ameliorate one or more symptoms of the disease.

The term "therapeutically effective amount", as used herein, relates to the sufficient amount of the nanobody, fusion protein or heavy chain of the invention to provide the desired effect, i.e. to achieve an appreciable prevention, cure, delay, reduction of severity or amelioration of one or more clinical signs derived is used in a broad sense to refer to at least a reduction from the disease, and will generally be determined by, among other causes, the characteristics of the agent itself and the therapeutic effect to be achieved. As used herein "therapeutically effective amount" also means an amount sufficient to reduce any clinical signs of the disease by at least 10%, preferably by at least 20%, preferably by at least 30%, preferably by at least 40%, preferably by at least 50%, preferably by at least 60%, preferably by at least 70%, preferably by at least 80%, preferably by at least 90%, preferably by at least 95%, preferably by at least 99%, and more preferably by at least 100% compared to a non-treated control group. It will also depend on the subject to be treated, the severity of the disease suffered by said subject, the chosen dosage form, etc. For this reason, the doses that may be mentioned in this invention must be considered only as guides for the person skilled in the art, who must adjust the doses depending on the aforementioned variables. Even though individual needs vary, determination of optimal ranges for immunologically or therapeutically effective amounts of the compounds according to the invention belongs to the common experience of those experts in the art. In general, the dosage needed to provide an effective treatment or prevention, which can be adjusted by one expert in the art, will vary depending on age, health, fitness, sex, diet, weight, degree of alteration of the receptor, frequency of treatment, nature and condition of the injury, nature and extent of impairment or illness, medical condition of the subject, route of administration, pharmacological considerations such as activity, efficacy, pharmacokinetic and toxicology profile of the particular compound used, if using a system drug delivery, and if the compound is administered as part of a combination of drugs.

In a particular embodiment of the medical uses of the invention, the nanobody, fusion protein or heavy chain antibody is administered in combination with at least an additional antibody, fusion protein or heavy chain antibody, wherein the additional nanobody, fusion protein or heavy chain antibody comprises a different variable region for that comprised in the first nanobody, fusion protein or heavy chain antibody, and wherein the at least one additional nanobody, fusion protein or heavy chain antibody comprises a variable domain selected from the group consisting of:
(A) a variable domain comprising a CDR1 comprising the amino acid sequence shown in SEQ ID NO: 10, a CDR2 comprising the amino acid sequence shown in SEQ ID NO: 11 and a CDR3 comprising the amino acid sequence shown in SEQ ID NO: 12, or functionally equivalent variants of said CDRs, or
(B) a variable domain comprising a CDR1 comprising the amino acid sequence shown in SEQ ID NO: 1, a CDR2 comprising the amino acid sequence shown in SEQ ID NO: 2 and a CDR3 comprising the amino acid sequence shown in SEQ ID NO: 3, or functionally equivalent variants of said CDRs, or
(C) a CDR1 comprising the amino acid sequence shown in SEQ ID NO: 4, a CDR2 comprising the amino acid sequence shown in SEQ ID NO: 5 and a CDR3 comprising the amino acid sequence shown in SEQ ID NO: 6, or functionally equivalent variants of said CDRs, or
(D) a CDR1 comprising the amino acid sequence shown in SEQ ID NO: 7, a CDR2 comprising the amino acid sequence shown in SEQ ID NO: 8 and a CDR3 comprising the amino acid sequence shown in SEQ ID NO: 9, or functionally equivalent variants of said CDRs, or
(E) a CDR1 comprising the amino acid sequence shown in SEQ ID NO: 13, a CDR2 comprising the amino acid sequence shown in SEQ ID NO: 14 and a CDR3 comprising the amino acid sequence shown in SEQ ID NO: 15, or functionally equivalent variants of said CDRs, or
(F) a CDR1 comprising the amino acid sequence shown in SEQ ID NO: 16, a CDR2 comprising the amino acid sequence shown in SEQ ID NO: 17 and a CDR3 comprising the amino acid sequence shown in SEQ ID NO: 18, or functionally equivalent variants of said CDRs, or
(G)a CDR1 comprising the amino acid sequence shown in SEQ ID NO: 19, a CDR2 comprising the amino acid sequence shown in SEQ ID NO: 20 and a CDR3 comprising the amino acid sequence shown in SEQ ID NO: 21, or functionally equivalent variants of said CDRs.

In case of administration of more than one fusion protein or heavy chain antibody, each fusion or heavy chain antibody can be monovalent (comprising just one nanobody of the invention) or bivalent (comprising two different nanobodies of the invention). In such a case, multiple different nanobodies can be administered, either forming part of the same fusion protein or heavy chain antibody or as a combination of several fusion proteins and/or heavy chain antibodies each of them comprising one nanobody. By this strategy, the subject can be treated with nanobodies specifically directed to different epitopes of the RBD domain of S protein from SARS-CoV-2.

When the medical uses of the invention comprise the administration of a combination of nanobodies, fusion proteins or heavy chain antibodies, the different nanobodies, fusion proteins or heavy chain antibodies can be administered simultaneously, as part of the same or different formulations, or sequentially.

Any combination of the nanobodies, fusion proteins or heavy chain antibodies of the invention previously defined can be used in the therapeutic methods of the invention. Particular advantageous combinations are those including nanobodies binding to different non-overlapping epitopes, in particular:
- a first nanobody selected from the go the group consisting of:
   (a) a nanobody comprising a variable domain comprising a CDR1 comprising or consisting of the amino acid sequence shown in SEQ ID NO: 10, a CDR2 comprising or consisting of the amino acid sequence shown in SEQ ID NO: 11 and a CDR3 comprising or consisting of the amino acid sequence shown in SEQ ID NO: 12, or functionally equivalent variants of said CDRs ; and
   (b) a nanobody comprising a variable domain comprising a CDR1 comprising or consisting of the amino acid sequence shown in SEQ ID NO: 19, a CDR2 comprising or consisting of the amino acid sequence shown in SEQ ID NO: 20 and a CDR3 comprising or consisting of the amino acid sequence shown in SEQ ID NO: 21, or functionally equivalent variants of said CDRs; and
- a second nanobody selected from the group consisting of:
   (a) a nanobody comprising a variable domain comprising a CDR1 comprising or consisting of the amino acid sequence shown in SEQ ID NO: 1, a CDR2 comprising or consisting of the amino acid sequence shown in SEQ ID NO: 2 and a CDR3 comprising or consisting of the amino acid sequence shown in SEQ ID NO: 3, or functionally equivalent variants of said CDRs;
   (b) a nanobody comprising a variable domain comprising a CDR1 comprising or consisting of the amino acid sequence shown in SEQ ID NO: 4, a CDR2 comprising or consisting of the amino acid sequence shown in SEQ ID NO: 5 and a CDR3 comprising or consisting of the amino acid sequence shown in SEQ ID NO: 6, or functionally equivalent variants of said CDRs;
   (c) a nanobody comprising a variable domain comprising a CDR1 comprising or consisting of the amino acid sequence shown in SEQ ID NO: 7, a CDR2 comprising or consisting of the amino acid sequence shown in SEQ ID NO: 8 and a CDR3 comprising or consisting of the amino acid sequence shown in SEQ ID NO: 9, or functionally equivalent variants of said CDRs;
   (d) a nanobody comprising a variable domain comprising a CDR1 comprising or consisting of the amino acid sequence shown in SEQ ID NO: 13, a CDR2 comprising or consisting of the amino acid sequence shown in SEQ ID NO: 14 and a CDR3 comprising or consisting of the amino acid sequence shown in SEQ ID NO: 15, or functionally equivalent variants of said CDRs and
   (e) a nanobody comprising a variable domain comprising a CDR1 comprising or consisting of the amino acid sequence shown in SEQ ID NO: 16, a CDR2 comprising or consisting of the amino acid sequence shown in SEQ ID NO: 17 and a CDR3 comprising or consisting of the amino acid sequence shown in SEQ ID NO: 18, or functionally equivalent variants of said CDRs.

Even more particularly advantageous combinations are those comprising:
- a first nanobody comprising a variable domain comprising a CDR1 comprising or consisting of the amino acid sequence shown in SEQ ID NO: 10, a CDR2 comprising or consisting of the amino acid sequence shown in SEQ ID NO: 11 and a CDR3 comprising or consisting of the amino acid sequence shown in SEQ ID NO: 12, or functionally equivalent variants of said CDRs and a second nanobody comprising a variable domain comprising a CDR1 comprising or consisting of the amino acid sequence shown in SEQ ID NO: 1, a CDR2 comprising or consisting of the amino acid sequence shown in SEQ ID NO: 2 and a CDR3 comprising or consisting of the amino acid sequence shown in SEQ ID NO: 3, or functionally equivalent variants of said CDRs;
- a first nanobody comprising a variable domain comprising a CDR1 comprising or consisting of the amino acid sequence shown in SEQ ID NO: 10, a CDR2 comprising or consisting of the amino acid sequence shown in SEQ ID NO: 11 and a CDR3 comprising or consisting of the amino acid sequence shown in SEQ ID NO: 12, or functionally equivalent variants of said CDRs and a second nanobody comprising a variable domain comprising a CDR1 comprising or consisting of the amino acid sequence shown in SEQ ID NO: 4, a CDR2 comprising or consisting of the amino acid sequence shown in SEQ ID NO: 5 and a CDR3 comprising or consisting of the amino acid sequence shown in SEQ ID NO: 6, or functionally equivalent variants of said CDRs.
- a first nanobody comprising a variable domain comprising or consisting of the amino acid sequence of SEQ ID NO: 25 or a functionally equivalent variant thereof and a second nanobody comprising a variable domain comprising or consisting of the amino acid sequence of SEQ ID NO: 22 or a functionally equivalent variant thereof;
- a first nanobody comprising a variable domain comprising or consisting of the amino acid sequence of SEQ ID NO: 25 or a functionally equivalent variant thereof and a second nanobody comprising a variable domain comprising or consisting of the amino acid sequence of SEQ ID NO: 23 or a functionally equivalent variant thereof.

In a particular embodiment of the medical uses of the invention, more than two, for example, three, four, five, six or the seven nanobodies of the invention can be combined.

In a particular embodiment, the nanobody, fusion protein or heavy chain antibody of the invention are used in combination with one or more compounds useful in the treatment of viral infections by RNA-viruses, preferably of the family *Coronaviridae,* especially by SARS-CoV-2. Some compounds that have been disclosed as potentially useful are selected from the group consisting of clofazimine, indomethacine, invermectin, disulfiram, boceprevir, paritaprevir, telaprevir, simeprevir, remdesivir, ribavirin, favipiravir, chloroquine, hydroxychloroquine, lopinavir, ritonavir, umifenovir and recombinant type I interferon, preferably clofazimine, indomethacine, invermectin, disulfiram, boceprevir, paritaprevir, telaprevir, simeprevir, remdesivir, lopinavir and ritonavir. Preferably, the combination is synergistic.

The term "combination" refers to a product comprising the defined nanobodies, fusion proteins or heavy chain antibodies and one or more of the compounds abovementioned potentially useful for treating respiratory syndrome-related coronaviruses, either in a single composition or in several compositions (or units), in which case the corresponding nanoparticles are distributed among the several compositions. Preferably, the combination refers to several compositions, in particular comprising one composition (or unit) per compound (compound as defined above) of the combination.

When the combination is in the form of a single composition, the nanobodies, fusion proteins or heavy chain antibodies and the compounds present in the combination are always administered simultaneously.

When the combination is in the form of several compositions (or units), each of them having at least one of the compounds of the combination, the compositions or (units) may be administered simultaneously, sequentially or separately.

Simultaneous administration means that the nanobodies, fusion proteins or heavy chain antibodies and the compounds or compositions (or units) are administered at the same time.

Sequential administration means that the nanobodies, fusion proteins or heavy chain antibodies and the compounds or compositions (or units) are administered at different time points in a chronologically staggered manner.

Separate administration means that the nanobodies, fusion proteins or heavy chain antibodies and the compounds or compositions (or units) are administered at different time points independently of each other.

### Diagnostic method and use of the invention

In another aspect, the invention relates to an *in vitro* method for diagnosing an infection by severe acute respiratory syndrome coronavirus 2 (SARS-CoV-2) in a subject comprising contacting the nanobody, fusion protein or heavy chain antibody of the invention with a sample from the subject and detecting the specific binding of said nanobody, fusion protein or heavy chain antibody to said sample, wherein if said nanobody, fusion protein or heavy chain antibody specifically bind to said sample, the subject is diagnosed as suffering from an infection by SARS-Cov-2.

In another aspect, the invention relates to the use of the nanobody, fusion protein or heavy chain antibody of the invention for diagnosing an infection by severe acute respiratory syndrome coronavirus 2 (SARS-CoV-2) in a subject.

The nanobody, fusion protein and heavy chain antibody of the invention have been previously defined. The terms "subject" and "SARS-CoV2" have been previously defined. All the particular and preferred embodiments regarding said terms fully apply to the diagnostic method and use of the invention.

In a particular embodiment, the nanobody comprises a variable domain comprising or consisting of the amino acid sequence of SEQ ID NO: 22-28 or a functionally equivalent variant thereof. In a more particular embodiment, said functionally equivalent variant has at least 80%, at least 85%, at least 90%, at least 95%, at least 98%, at least 99% or more sequence identity with any one of SEQ ID NO: 22-28. In an even more particular embodiment, the functionally equivalent variants of the variable region sequences of SEQ ID NO: 22-28 comprise the sequence of the CDRs included in the variable regions of SEQ ID NO: 22-28 and the amino acid substitutions, deletions or additions affect only the framework regions, as previously detailed for the nanobodies of the invention.

In a particular embodiment, both the nanobodies are humanized. In a particular embodiment, the humanized nanobodies comprise a variable region that is a functionally equivalent variant of any one of the variable domains comprising or consisting of the amino acid sequence of SEQ ID NO: 22-28. In a more particular embodiment, said humanized functionally equivalent variants comprise or consist of the sequences of SEQ ID NO: 29-32.

The term "diagnosis", as used herein, refers both to the process of attempting to determine and/or identify a possible disease in a subject, i.e. the diagnostic procedure, and to the opinion reached by this process, i.e. the diagnostic opinion. As such, it can also be regarded as an attempt at classification of an individual's condition into separate and distinct categories that allow medical decisions about treatment and prognosis to be made. As the person skilled in the art will understand, such a diagnosis may not be correct for 100% of the subjects to diagnose, although preferred it is. The term, however, requires that a statistically significant part of the subjects can be identified as suffering from a disease, particularly melanoma in the context of the invention. The skilled in the art may determine whether a party is statistically significant using different statistical evaluation tools well known, for example, by determination of confidence intervals, the p-value determination, Student's-test, the Mann- Whitney, etc. Preferred confidence intervals are at least, 50%, at least 60%, at least 70%, at least 80%, at least 90%) or at least 95%. The p-values are preferably, 0.05, 0.025, 0.001 or lower.

The term *"in vitro",* as used herein, refers to the fact that the method is not carried out on the body of a human or animal subject, but rather on cells or fluids isolated from said subject or in a test tube.

The term "sample" or "biological sample" is intended to refer to biological material isolated from a subject. The biological sample can contain any biological material suitable for detecting the desired biomarker and can comprise cell and/or noncell material of the subject. The sample can be isolated from any suitable tissue or biological fluid such as for example, blood, saliva, plasma, serum, urine, cerebrospinal liquid (CSF), faeces, a buccal or buccal-pharyngeal swab, a surgical specimen, and a specimen obtained from a biopsy.

### Additional aspects of the invention

1. A nanobody directed against the receptor binding domain (RBD) of spike protein from severe acute respiratory syndrome coronavirus 2 (SARS-CoV-2), wherein the nanobody comprises a variable domain comprising a CDR1 comprising the amino acid sequence shown in SEQ ID NO: 7, a CDR2 comprising the amino acid sequence shown in SEQ ID NO: 8 and a CDR3 comprising the amino acid sequence shown in SEQ ID NO: 9, or functionally equivalent variants of said CDRs.
2. The nanobody according to aspect 1, wherein said variable domain comprises an amino acid sequence of SEQ ID NO: 24 or a functionally equivalent variant thereof.
3. The nanobody according to aspect 2, wherein the functionally equivalent variant has at least 80% sequence identity to the amino acid sequence shown in SEQ ID NO: 24.
4. The nanobody according to any one of aspects 1 to 3, wherein the CDR1 comprises the amino acid sequence shown in SEQ ID NO: 7, the CDR 2 comprises the amino acid sequence shown in SEQ ID NO: 8 and the CDR3 comprises the amino acid sequence shown in SEQ ID NO: 9.
5. The nanobody according to any one of aspects 1 to 4, wherein the nanobody is humanized.
6. A fusion protein comprising the nanobody according to any one of aspects 1 to 5 and the Fc region of a heavy chain.
7. The fusion protein according to aspect 6, further comprising a second nanobody directed against the RBD of spike protein from SARS-CoV-2, wherein the second nanobody comprises a variable domain selected from the group consisting of:
   (A) a variable domain comprising a CDR1 comprising the amino acid sequence shown in SEQ ID NO: 1, a CDR2 comprising the amino acid sequence shown in SEQ ID NO: 2 and a CDR3 comprising the amino acid sequence shown in SEQ ID NO: 3, or functionally equivalent variants of said CDRs, or
   (B) a CDR1 comprising the amino acid sequence shown in SEQ ID NO: 4, a CDR2 comprising the amino acid sequence shown in SEQ ID NO: 5 and a CDR3 comprising the amino acid sequence shown in SEQ ID NO: 6, or functionally equivalent variants of said CDRs, or
   (C) a CDR1 comprising the amino acid sequence shown in SEQ ID NO: 10, a CDR2 comprising the amino acid sequence shown in SEQ ID NO: 11 and a CDR3 comprising the amino acid sequence shown in SEQ ID NO: 12, or functionally equivalent variants of said CDRs, or
   (D) a CDR1 comprising the amino acid sequence shown in SEQ ID NO: 13, a CDR2 comprising the amino acid sequence shown in SEQ ID NO: 14 and a CDR3 comprising the amino acid sequence shown in SEQ ID NO: 15, or functionally equivalent variants of said CDRs, or
   (E) a CDR1 comprising the amino acid sequence shown in SEQ ID NO: 16, a CDR2 comprising the amino acid sequence shown in SEQ ID NO: 17 and a CDR3 comprising the amino acid sequence shown in SEQ ID NO: 18, or functionally equivalent variants of said CDRs, or
   (F) a CDR1 comprising the amino acid sequence shown in SEQ ID NO: 19, a CDR2 comprising the amino acid sequence shown in SEQ ID NO: 20 and a CDR3 comprising the amino acid sequence shown in SEQ ID NO: 21, or functionally equivalent variants of said CDRs.
8. The fusion protein according to aspect 7, wherein the variable domain of the second nanobody comprises an amino acid sequence selected from the group consisting of SEQ ID NO: 22-23 and 25-28 or a functionally equivalent variant thereof.
9. The fusion protein according to aspect 8, wherein the functionally equivalent variant of the variable domain of the second nanobody comprises an amino acid sequence selected from the group consisting of SEQ ID NO: 29-32.
10. The fusion protein according to any one of aspects 7 to 9, wherein the first nanobody and the second nanobody are linked by a peptide linker.
11. The fusion protein according to aspect 10, wherein the peptide linker has the amino acid sequence of SEQ ID NO: 33.
12. The fusion protein according to any one of aspects 6 to 11, wherein the first or second nanobody is associated with a detectable marker.
13. The fusion protein according to aspect 12, wherein said detectable marker is biotin.
14. The fusion protein according to aspect 13, wherein the Fc region is the Fc region from human IgG.
15. The fusion protein according to any one of aspects 6 to 14, wherein the fusion protein comprises, from its N-terminus to its C-terminus:
   - the nanobody according to any one of aspects 1 to 5 and
   - the Fc region of a heavy chain,
      or, alternatively,
   - the first nanobody,
   - the second nanobody and
   - the Fc region of a heavy chain,
      or, alternatively:
   - the second nanobody,
   - the first nanobody and
   - the Fc region of a heavy chain.
16. A heavy chain antibody comprising two heavy chains, wherein each heavy chain comprises the fusion protein according to any one of aspects 6 to 15, wherein both heavy chains are identical.
17. A fusion protein comprising two nanobodies, wherein
   (i) the first nanobody is the nanobody according to any one of aspects 1 to 5 and
   (ii) the second nanobody comprises a variable domain selected from the group consisting of:
      (a) a variable domain comprising a CDR1 comprising the amino acid sequence shown in SEQ ID NO: 1, a CDR2 comprising the amino acid sequence shown in SEQ ID NO: 2 and a CDR3 comprising the amino acid sequence shown in SEQ ID NO: 3, or functionally equivalent variants of said CDRs, or
      (b) a CDR1 comprising the amino acid sequence shown in SEQ ID NO: 4, a CDR2 comprising the amino acid sequence shown in SEQ ID NO: 5 and a CDR3 comprising the amino acid sequence shown in SEQ ID NO: 6, or functionally equivalent variants of said CDRs, or
      (c) a CDR1 comprising the amino acid sequence shown in SEQ ID NO: 10, a CDR2 comprising the amino acid sequence shown in SEQ ID NO: 11 and a CDR3 comprising the amino acid sequence shown in SEQ ID NO: 12, or functionally equivalent variants of said CDRs, or
      (d) a CDR1 comprising the amino acid sequence shown in SEQ ID NO: 13, a CDR2 comprising the amino acid sequence shown in SEQ ID NO: 14 and a CDR3 comprising the amino acid sequence shown in SEQ ID NO: 15, or functionally equivalent variants of said CDRs, or
      (e) a CDR1 comprising the amino acid sequence shown in SEQ ID NO: 16, a CDR2 comprising the amino acid sequence shown in SEQ ID NO: 17 and a CDR3 comprising the amino acid sequence shown in SEQ ID NO: 18, or functionally equivalent variants of said CDRs, or
      (f) a CDR1 comprising the amino acid sequence shown in SEQ ID NO: 19, a CDR2 comprising the amino acid sequence shown in SEQ ID NO: 20 and a CDR3 comprising the amino acid sequence shown in SEQ ID NO: 21, or functionally equivalent variants of said CDRs.
18. The fusion protein according to aspect 17, wherein the variable domain of the second nanobody comprises an amino acid sequence selected from the group consisting of SEQ ID NO: 22-23 and 25-28 or a functionally equivalent variant thereof.
19. The fusion protein according to aspect 18, wherein the functionally equivalent variant of the second nanobody comprises an amino acid sequence selected from the group consisting of SEQ ID NO: 29-32.
20. The fusion protein according to any one of aspects 17 to 19, wherein the first nanobody and the second nanobody are linked by a peptide linker.
21. The fusion protein according to aspect 20, wherein the peptide linker has the amino acid sequence of SEQ ID NO: 33.
22. The fusion protein according to any one of aspects 17 to 21, wherein the first or second nanobody is associated with a detectable marker.
23. The fusion protein according to aspect 22, wherein said detectable marker is biotin.
24. A nucleic acid encoding the nanobody according to any one of aspects 1 to 5 or the fusion protein according to any one of aspects 6 to 15 or according to any one of aspects 17 to 23.
25. A vector comprising the nucleic acid according to aspect 24.
26. A host cell comprising the nucleic acid according to aspect 24 or the vector according to aspect 25.
27. A pharmaceutical composition comprising the nanobody according to any one of aspects 1 to 5, or the fusion protein according to any one of aspects 6 to 15, or the heavy chain antibody according to aspect 16, or the fusion protein according to any one of aspects 17 to 23, and a pharmaceutically acceptable excipient or carrier.
28. The nanobody according to any one of aspects 1 to 5, or the fusion protein according to any one of aspects 6 to 15, or the heavy chain antibody according to aspect 16, or the fusion protein according to any one of aspects 17 to 23, for use as a medicament.
29. The nanobody according to any one of aspects 1 to 5, or the fusion protein according to any one of aspects 6 to 15, or the heavy antibody according to aspect 16, or the fusion protein according to any one of aspects 17 to 23, for use in the treatment and/or prevention of an infection caused by severe acute respiratory syndrome coronavirus 2 (SARS-CoV-2).
30. The nanobody, fusion protein or heavy chain antibody for use according to aspect 29, wherein said nanobody, fusion protein or heavy chain antibody is administered in combination with at least an additional nanobody, fusion protein or heavy chain antibody comprising a variable domain selected from the group consisting of:
   (A) a variable domain comprising a CDR1 comprising the amino acid sequence shown in SEQ ID NO: 1, a CDR2 comprising the amino acid sequence shown in SEQ ID NO: 2 and a CDR3 comprising the amino acid sequence shown in SEQ ID NO: 3, or functionally equivalent variants of said CDRs, or
   (B) a CDR1 comprising the amino acid sequence shown in SEQ ID NO: 4, a CDR2 comprising the amino acid sequence shown in SEQ ID NO: 5 and a CDR3 comprising the amino acid sequence shown in SEQ ID NO: 6, or functionally equivalent variants of said CDRs, or
   (C) a CDR1 comprising the amino acid sequence shown in SEQ ID NO: 10, a CDR2 comprising the amino acid sequence shown in SEQ ID NO: 11 and a CDR3 comprising the amino acid sequence shown in SEQ ID NO: 12, or functionally equivalent variants of said CDRs, or
   (D) a CDR1 comprising the amino acid sequence shown in SEQ ID NO: 13, a CDR2 comprising the amino acid sequence shown in SEQ ID NO: 14 and a CDR3 comprising the amino acid sequence shown in SEQ ID NO: 15, or functionally equivalent variants of said CDRs, or
   (E) a CDR1 comprising the amino acid sequence shown in SEQ ID NO: 16, a CDR2 comprising the amino acid sequence shown in SEQ ID NO: 17 and a CDR3 comprising the amino acid sequence shown in SEQ ID NO: 18, or functionally equivalent variants of said CDRs, or
   (F) a CDR1 comprising the amino acid sequence shown in SEQ ID NO: 19, a CDR2 comprising the amino acid sequence shown in SEQ ID NO: 20 and a CDR3 comprising the amino acid sequence shown in SEQ ID NO: 21, or functionally equivalent variants of said CDRs
   wherein the at least one additional nanobody, fusion protein or heavy chain antibody is different from the first nanobody, fusion protein or heavy chain antibody.
31. The nanobody, fusion protein or heavy chain antibody for use according to aspect 30, wherein the additional nanobody, fusion protein or heavy chain antibody comprises a variable domain comprising an amino acid sequence selected from the group consisting of SEQ ID NO: 22-23 and 25-28 or a functionally equivalent variant thereof.
32. The nanobody, fusion protein or heavy chain antibody for use according to aspect 31, wherein the functionally equivalent variant of the variable domain of the additional nanobody, fusion protein or heavy chain antibody comprises an amino acid sequence selected from the group consisting of SEQ ID NO: 29-32.
33. An *in vitro* method for diagnosing an infection by severe acute respiratory syndrome coronavirus 2 (SARS-CoV-2) in a subject comprising contacting the nanobody according to any one of aspects 1 to 5, or the fusion protein according to any one of aspect 6-15, or the heavy chain antibody according to aspect 16, or the fusion protein according to any one of aspects 17 to 23, with a sample from the subject and detecting the specific binding of said nanobody, fusion protein or heavy chain antibody to said sample, wherein if said nanobody, fusion protein or heavy chain antibody specifically bind to said sample, the subject is diagnosed as suffering from an infection by SARS-Cov-2.
34. Use of the nanobody according to any one of aspects 1 to 5, or the fusion protein according to any one of aspects 6-15, or the heavy chain antibody according to aspect 16, or the fusion protein according to any one of aspects 17 to 23 for diagnosing an infection by severe acute respiratory syndrome coronavirus 2 (SARS-CoV-2) in a subject.

### EXAMPLES

### Materials and methods

### Dromedary immunization and VHH library construction.

The animal immunization protocol followed European Union and Spanish guidelines of animal experimentation and was approved by the Ethics Committee for Animal Experimentation from University of *Las Palmas de Gran Canaria* and the *Consejería de Agricultura, Pesca y Aguas* of the Canary Islands Goverment (reference OEBA-ULPGC 01-2020 R2). Two adult and healthy dromedary camels (*Camelus dromedarius*) were immunized by subcutaneous injection of purified RBDmFc protein (described below) once in every week during six weeks. Every injection contained 0.2 mg of RBDmFc in 2 ml sterile saline (0.9% NaCl) and 2 ml of adjuvant (FAMA Adjuvant, GERBU). The total volume (4 ml) was injected in three distinct sites in the camel. Serum samples were collected before the first immunization and four days after the last boost, and they were used to confirm Ab immune response by ELISA. An additional 50 ml of blood was collected from the jugular vein in tubes containing EDTA as anticoagulant (K2EDTA Tubes, Vacutainer^{®}, BD) for lymphocyte isolation. Lymphocyte mRNA extraction, first-strand cDNA synthesis and PCR amplification of VHHs was conducted as previously reported (Salema et al., 2016, J Biotechnol 234, 58-65) with oligonucleotide primers VHHSfi2 (5'-GTC CTC GCA ACT GCG GCC CAG CCG GCC ATG GCT CAG GTG CAG CTG GTG GA) (SEQ ID NO: 35) and VHHNot2 (5'-GGA CTA GTG CGG CCG CTG AGG AGA CGG TGA CCT GGGT) (SEQ ID NO: 36). The amplified VHH fragments from dromedary 1 (D1) and dromedary 2 (D2) were digested with *Sfi*I and *Not*I restriction enzymes, ligated into the same sites of pNeae2 vector and electroporated separately into *E. coli* DH10B-T1R cells for surface display of the Nbs as fusions with the intimin anchoring domain (Salema V and Fernández L (2017) Microb Biotechnol 10: 1468-1484; Salema V et al., (2013) PLoS ONE 8: e75126).

### Nanobody surface display in bacteria.

The bacteria that displayed the D1 and D2 Nb libraries were grown at 30 °C in Lysogeny broth (LB) liquid medium, or on LB-agar 1.5% (w/v) plates, both with chloramphenicol (Cm, 30 µg/ml) and 2% (w/v) glucose (Glu) for repression of the *lac* promoter. For induction of Nb fusions, bacteria were grown on liquid LB-Cm (without Glu) containing 0.05 mM isopropylthio-β-D-galactoside (IPTG).

### Selection of RBD-binding Nbs from immune libraries displayed on E. coli.

Duplicated bacterial samples representing each library (D1 and D2) with ~5×10⁸ CFU in 100 µl of PBS-BSA (phosphate buffered saline with 0.5% w/v of bovine serum albumin) were mixed in a total volume of 200 µl with biotinylated RBD in the same buffer at 100 nM or 50 nM for the first and second magnetic cell sorting (MACS) steps, respectively. After 1 h incubation at RT, bacteria were washed, incubated with antibiotin paramagnetic beads (Miltenyi Biotec), loaded onto a MACS MS column (Miltenyi Biotec), and bound bacteria recovered. After a second MACS cycle the bound bacteria were selected by fluorescence activated cell sorting (FACS). The induced bacteria (~5×10⁸ CFU) were mixed in a total volume of 200 µl of PBS-BSA with biotinylated RBD (50 nM final concentration) and mouse anti-c-myc monoclonal Ab (1:500, 9B11 clone, Cell Signaling). After 1 h incubation at RT, bacteria were washed and stained with goat anti-mouse IgG-Alexa Fluor 488 conjugated polyclonal Ab (1:500; ThermoFisher Scientific) and Streptavidin-Allophycocyanin (APC) (1:100; Southern Biotech). After 1 h incubation at 4 °C (in the dark), bacteria were washed and resuspended PBS for sorting in a FACS vintage SE cytometer (Becton Dickinson). At least ~1×10⁶ bacterial events were processed per sample. Double-stained bacterial population with high fluorescence intensity signals was collected (at least ~5×10³ events) in LB-Cm-Glu medium and plated.

### Flow cytometry analysis of antigen binding to E. coli bacteria displaying Nbs.

Binding of biotin-labeled antigens to Nbs displayed on bacteria from libraries (enriched pools or individual clones) were analyzed by flow cytometry. To this end, induced bacteria cultures were washed and incubated with the indicated biotin-labeled antigen (i.e., 5 nM RBD, 5 nM S, or 100 nM human fibrinogen) and anti-c-myc monoclonal Ab, as described above for FACS selection. Bacteria were stained with secondary fluorophore reagents as for FACS selection and analyzed in a Gallios cytometer (Beckman Coulter).

### Expression and purification of hcAbs and Nbs.

The DNA segments coding for the selected Nbs were PCR amplified from bacterial pNeae2-VHH plasmids with oligonucleotide primers Nb1-N2-plg-Agel (5'-ACT GCA ACC GGT GTA CAT TCT CAG GTG CAG CTG GTG GAA) (SEQ ID NO: 37) and Nb1-C5-plg-BamHI (5'-ACT GGA TCC AGA ACC ACT TGC CGC TGA GGA GAC GGT GAC CTG) (SEQ ID NO: 38) and cloned in *Age*I-BamHI sites in frame with a IgH signal peptide and the human IgG1 hinge and Fc portion (Fc) of mammalian expression vector pIgΔCH1, derived from pIgΔ1HC (kindly provided by Prof. M. Nussenzweig, Rockefeller University) (Tiller T et al. (2008). J Immunol Methods 329: 112-124). The hcAbs were routinely produced in mammalian cells with these recombinant plasmids. For Nb preparation, constructs with a thrombin recognition site (LVPRGS) (SEQ ID NO: 34) between the Nb domain and the Fc using oligonucleotide primers Nb1-N2-plg-Agel (see above) and Nb1-C2-pIg-BamHI (5'-TCA CTC GAG GCG GAT CCA CGC GGA ACC AGC GCT GAG ACG GTG AC) (SEQ ID NO: 39) were generated and cloned between *Age*I-*Ba*mHI sites of pIgΔCH1. In the bispecific hcAbs, a GS-linker encoding (GGGGS)x3 (SEQ ID NO: 33) sequence was cloned between the C-terminus of the first VHH domain and N-terminus of the second VHH domain. To this end, the second VHH domain was amplified with the GS-linker using oligonucleotide primers BamHI-Nb-N-linker (5'-ATG GGA TCC GGA GGT GGC GGG TCC GGA GGT GGC GGG TCC GGA GGT GGC GGG TCC CAG GTG CAG CTG GTG GAG TCT) (SEQ ID NO: 40) and Nb1-C5-pIg-BamHI (see above) and cloned in the BamHI site of the pIgΔCH1 vector carrying VHH1.

In all cases, suspension human embryonic kidney (HEK-293F) mammalian cells were transfected with purified expression vector DNAs using polyethylenimine (PEI) and following standard procedures. Cultures with the proteins in supernatants were collected 5 days post-transfection. Cells were then centrifuged, and proteins purified from the cell supernatant with an Ig Select or protein A affinity columns (Cytiva), as indicated by the manufacturer. The Ab samples were further purified by size exclusion chromatography (SEC) with a Superdex75 (10/300) column (Cytiva) in HBS (20 mM HEPES, 150 mM NaCl, pH 7.4). Single Nbs were generated by thrombin digestion of hcAb proteins with a thrombin site after the VHH domains (see above). The Fc and Nb portions were separated by SEC with a Superdex75 (16/600) column (Cytiva) in HBS.

### Production of the S and RBD proteins.

A recombinant DNA fragment coding for the soluble S (residues 1 to 1208) protein was obtained by gene synthesis (GeneArt, ThermoFisherScientific) and cloned betweem *Xho*I*-Not*I sites of pcDNA3.1 vector for expression in HEK-293F cells (García-Arriaza et al., 2021, J Virol 95: e02260-20). The recombinant DNA construct contained the S signal sequence at the N-terminus, and a T4 fibritin trimerization sequence, a Flag peptide and a 6x or 8xHis-tag at the C-terminus. In the S protein, the furinrecognition motif (RRAR) (SEQ ID NO: 41) was replaced by the GSAS sequence (SEQ ID NO: 42), and it contained the A942P, K986P and V987P substitutions in the S2 portion (Hsieh et al., 2020. Science 369: 1501-1505). The soluble S was purified by Ni-NTA affinity chromatography from transfected suspension HEK293F cell supernatants, and it was transferred to HBS pH 7.5, during concentration or by SEC in a Superose 6 (16/60) column.

Several RBD constructs have been used in this study. Recombinant DNA fragments encoding the RBD and thrombin site were obtained by gene synthesis (GeneArt, ThermoFisherScientific). In some constructs, the RBD fragments were cloned between *Age*I and BamHI sites of pIgΔCH1 (see above). In other constructs, the DNA coding for the RBD portion was cloned in frame with the IgK leader sequence and an HA-tag (YPYDVPDYA) (SEQ ID NO: 43 and contained thrombin recognition sites (LVPRGS) (SEQ ID NO: 34) at the 5' and 3' RBD ends. Initially, a recombinant RBD (residues 334-528 of the S) C-terminal fused to the TIM-1 mucin domain and the human IgG1 Fc region (RBDmFc) was also prepared in HEK293F cells, which was used for camel immunization. Subsequently, RBD-Fc fusion proteins without the mucin domain and with S residues 332 to 534 were produced, as well as a monomeric RBD (332-534) with a FLAG peptide and an 8xHis-tag at the C-terminus (RBD-FH). The recombinant RBD constructs in mammalian expression vectors were used for protein production by transient transfection of HEK293F cells. The RBD-Fc proteins were purified as described for hcAbs, whereas the RBD-FH was prepared by Ni-NTA affinity chromatography. An isolated RBD without tags was generated from the fusion protein as described in detail elsewhere (Martinez-Fleta et al., 2020, The Journal of Immunology 205: 3130).

### Enzyme-linked immunosorbent assays (ELISA) to evaluate hcAb binding to S and RBD proteins.

Different assays were conducted to test binding of camel sera or purified hcAbs to RBD and S proteins. 96-well ELISA plates (Maxisorp, Nunc) were coated o/n at 4 °C with 50 µl/well of the indicated protein antigen (RBD, RBD-FH, RBD-Fc or S), or BSA as negative control, at ~2 µg/ml in PBS, or 10 mM Tris-HCl 50 mM NaCl, pH 7.4. Specific conditions of blocking, incubation with primary (camel sera or hcAbs) and secondary reagents conjugated to horseradish peroxidase (HRP), and development are described in the Supplementary Materials and methods. Optical Density at 490 nm (OD₄₉₀) values were corrected with the background OD₄₉₀ of wells without antigen or with BSA.

### hcAb competition of RBD-hACE2 interaction.

ELISA plates (Maxisorp, Nunc) were coated o/n at 4 °C with 5 µg/ml of the soluble extracellular domain of hACE2 (residues 19-615) in PBS (50 µl/well). Soluble hACE2 with a C-terminal his-tag was purified from insect cells (a kind gift of Dr. Jose F. Rodriguez, CNB-CSIC). After coating, plates were washed with PBS and blocked at RT for 2 h with 200 µl/well of 3% (w/v) skimmed milk and 1% (w/v) BSA in PBS. A dilution of the biotin-labeled RBD-Fc at 20 nM was prepared in 1% (w/v) skimmed milk-PBS containing the indicated concentrations of unlabeled hcAb competitor (i.e. 0, 50, 100, or 200 nM), and the mixture was added to hACE2-coated immunoplates for 90 min at RT. After incubation, the wells were washed five times with PBS and the binding of biotinylated RBD-Fc detected with secondary streptavidin-HRP (1:1000; Merck-Sigma) in 1% (w/v) skimmed milk-PBS. After 45 min incubation at RT, plates were washed, developed, and OD₄₉₀ determined as above.

### Binding kinetics and affinity determination by Surface Plasmon Resonance (SPR).

SPR was applied to monitor hcAb/Nb binding to S/RBD proteins in a BIAcore 3000 instrument (GE Healthcare) with CM5 chips in HBS buffer. An anti-Flag antibody (M2, Merck-Sigma) was covalently immobilized into the dextran surface of the chip (-9000 RU) with the "amine coupling kit" (BIAcore) to capture S or RBD proteins.

In each cycle, 15 µl (20-30 µg/ml) of S or RBD-FH were injected at 5 µl/min, followed by the injection of 40 µl of hcAb or Nb, at 10 µl/min in HBS. Chip surfaces were regenerated with a 5 µl pulse of 50 mM phosphoric acid at 100 µl/min. Two binding surfaces (Fc2 and Fc3) with immobilized Ab were monitored in each experiment. Binding kinetics were determined by the analysis of the sensorgrams with the BIAEvaluation 3.0 software after the subtraction of the signal obtained from the control flow cell surface with no anti-Flag (Fc1); baseline drifting due to ligand dissociation from the Flag Ab was corrected with a sensorgram recorded with HBS injection or with the BIAevaluation program during kinetic determination. The sensorgrams were adjusted to a Langmuir (1:1) model for binding kinetics estimation, which were determined following separate and simultaneous fitting procedures in the BIAevaluation program, and then, averaged.

### hcAb neutralization of pseudotyped retroviral particles in vitro.

The neutralization capacity of hcAbs was first determined using luciferasebased reporter retroviral particles pseudotyped with the S protein of SARS-CoV-2 (Spp). Pseudotyped viruses were produced by transfection of the S protein expressing plasmid together with packaging plasmids in HEK-293T cells as previously described (García-Arriaza et al., 2021 J Virol 95: e02260-20). Retroviral particles pseudotyped with G glycoprotein (VSVGpp) from the vesicular stomatitis virus (VSV) were produced and used in parallel as control. Luciferase reporter activity was used to titrate the pseudotyped viruses in Vero-E6 cells and with complete DMEM + 2% Fetal Bovine Serum (FBS).

The hcAbs were diluted in complete DMEM + 2% FBS at 4, 0.4, and 0.04 µg/ml, and 50 µl of the hcAb dilutions were mixed with 50 µl of pseudotyped virus and kept for 1 h at 37°C. The mixtures were added to wells of flat-bottom 96-well plates with the Vero-E6 (1×10⁴ cell/well), and incubated -20 h at 37°C, after which the medium was replaced by 100 µl/well of complete DMEM + 2% FBS. Plates were further incubated for additional 24 h at 37°C. The next day (48 h.p.i.) medium was discarded and cell lysates were prepared in passive lysis buffer (Promega). Luciferase activity assay was performed as indicated by the manufacturer (Promega). Luciferase activity levels in wells without hcAb were used as reference and set to 100%.

### hcAb neutralization of SARS-CoV-2 in vitro

Neutralization of SARS-CoV-2 virus (strain NL/2020) was carried out in Vero-E6 cells at the CNB-CSIC BSL3 facility. This viral strain was isolated from an infected patient in the Netherlands in February 2020 and was obtained through the EVAg repository from Dr. Molenkamp (Erasmus University-Rotterdam, NL). As described for pseudovirus neutralization experiments, hcAb-virus mixtures (100 µl/sample) with 140 FFU (MOI of 0.007) of SARS-CoV-2 were added to wells of flat-bottom 96-well plates with Vero-E6 (2×10⁴ cell/well), and incubated for 1 h at 37°C. After the incubation, the infective mixtures were removed, the cells were washed once with complete PBS and 100 µl/well of complete DMEM+2% FBS added. Plates were incubated for 24 h at 37°C, before medium was discarded and cells fixed with a 4% formaldehyde-PBS solution for 20 min at RT, after which fixative was discarded and cells were extensively washed with PBS. Cells were then incubated in binding buffer (3% BSA; 0.3% Triton X100 in PBS) for 1h at RT, and immunofluorescence performed using a primary rabbit monoclonal antibody against N protein (Genetex) diluted 1:2000 in binding buffer. After 1h incubation at RT, cells were washed with PBS and subsequently incubated with a 1:500 dilution of a secondary goat anti-rabbit conjugated to Alexa 488 (Invitrogen) antibody. Cells were counterstained with Dapi (Life Technologies) during the secondary antibody incubation time. Automated fluorescence microscopy imaging was performed in a SparkCyto (Tecan) multimode plate reader. Immunofluorescence signal in wells without hcAb were used as reference and set to 100%.

### hcAb inhibition of COVID-19 progression in mice

Animal experimentation was conducted in the biosafety level 3 facilities at Centro de Investigacion en Sanidad Animal at Instituto Nacional de Investigación y Tecnología Agraria y Alimentaria (CISA-INIA, CSIC). Experiments were approved by the Ethical Committee of Animal Experimentation of INIA and by the Division of Animal Protection of the Comunidad de Madrid (PROEX 115.5-21). Animals were handled in strict accordance with the guidelines of the European Community 86/609/CEE.

A total of 36 six-week-old hemizygous K18-hACE2 transgenic (B6.Cg-Tg(K18-ACE2)2Prlmn/J) female mice were used (The Jackson Laboratory, ME, USA). Animals were anesthetized under isoflurane and inoculated intranasally (i.n.) with 50 µl of DMEM containing 5×10⁴ PFU of SARS-CoV-2 isolate hCoV-19/Spain/SP-VHIR.02, D614G(S) from lineage B.1.610, kindly provided by Dr. Miguel Chillón (Universitat Pompeu i Fabra, Barcelona, Spain). Uninfected control mice were inoculated in parallel with DMEM. Six mice per group were inoculated intraperitoneally (i.p.) with 150 µg of each hcAb (equivalent to ~8 mg/kg for a mouse of -18 g) in 150 µl of HBS at 24 h.p.i. Uninfected control mice were inoculated at the same time only with buffer. Animals received water and food *ad libitum* and were monitored daily for clinical signs and body weight. Euthanasia was applied when the animals exhibited irreversible disease signs. All surviving mice were anesthetized and euthanized at the end of the experiment.

### Cryo-EM of the S-Nb complexes.

Ni-NTA affinity purified S with 6xHis-tag was mixed with the Nb (1:1.5 molar ratio of monomer:Nb) during 30 min at 10°C, and the S-Nb complex purified by size exclusion chromatography in a Superose 6 (16/60) column with 20 mM Tris-buffer and 200 mM NaCl, pH 7.7. The sample was concentrated to 1mg/ml and applied to glowdischarged holey carbon grids (Quantifoil, Au 300 mesh, R 0.6/1). The grids were blotted and then plunged into liquid ethane using an FEI Vitrobot Mark IV at 4°C. Data were collected at FEI Talos Arctica electron microscope operated at 200 kV and equipped with a Falcon III electron detector, a dataset was collected in a Titan Krios at the ESRF CM01 line. At the Talos Arctica, the images were recorded at a defocus range of -1 µm to -2.5 µm with a pixel size of 0.855 Å; exposure time was 40 s, with a total exposure dose of 32 e/Å² over 60 frames. At the Titan Krios, a super resolution acquisition mode was used with a 105k magnification and a pixel size of 0.84 Å, with a defocus range of -1.0 to -2.4 um in 0.2 um step; the exposure time was of 1.8 s, with a total exposure dose of 39.0 e/Å² (15.3 e/pixel/s) over 40 frames.

Particle reconstructions were carried out throughout an INSTRUCT project in the Electron Microscopy Image Processing, I2PC, Madrid, which resulted in EM maps of the trimeric S with the different bound Nbs. Scipion 2.0 (de la Rosa-Trevín et al., 2016, J Struct Biol 195: 93-99) was used in order to easily combine different software suites in the analysis workflows of CryoEM data: Movie frames were aligned using MotionCor2; the contrast transfer function (CTF) of the micrographs was estimated using CTFFIND4; particles were automatically selected from the micrographs using autopicking from Gautomatch. Evaluation of the quality of particles and selection after 2D classifications, the initial volume for 3D image processing, the 3D-classification and the final refinement were calculated using cryoSparc, whereas the sharpening was estimated by DeepEMhancer.

Model building for the S-Nb complexes was performed with a S protein structure (PDB 6ZXN) and Nb models, which were prepared with the program Modeller based on the 1ZV5 (1.10), 3TPK (1.29) and 6DBA (2.15) structures. The S structure and the Nb models were fitted into the EM map with the chimera and coot programs. Subsequently, the structures were subjected to real-space refinement in PHENIX (Liebschner et al., 2019, Acta Crystallogr D Struct Biol 75: 861-877), which included cycles of rigid body, global and adp minimization. NCS was applied among the S domains, but excluding the RBDs. Nb-RBD binding interfaces were determined with the PISA server, and main figures of the structures were prepared with pymol (pymol.org). The refined structures are being deposited in the PDB.

### Quantification and statistical analysis.

Means and standard errors (SEM), or standard deviations (SD), of experimental values from independent experiments (n, as indicated in Figure Legends) were calculated using Prism 7.0 and 8.0 (GraphPad software Inc). Binding curves represented using GraphPad Prism performing a non-linear regression (curve fit). Differences in binding were evaluated using two-tailed paired t-test. Log-rank (Mantel-Cox) test was used for survival analysis of mice using Prism 7.0 (GraphPad software Inc).

### Results

### Selection of a panel of Nbs binding to the RBD of SARS-CoV-2.

Two healthy dromedary camels were immunized using the RBD of the SARS-CoV-2 from the WA1 strain fused to a mucin domain and the Fc portion of human IgG1 (RBDmFc in Fig. 7A). ELISA of sera from both immunized animals showed specific Ab response against the RBD alone (Fig. 7B). A blood sample of each immunized dromedary was used for RT-PCR amplification of the VHHs from lymphocyte mRNA samples. Each VHH pool was cloned into the pNeae2 vector for Nb display on the E. *coli* bacterial surface (Salema & Fernández, 2017, supra; Salema *et al.,* 2013, supra), generating two immune libraries of VHHs (D1 and D2) with ~6.2×10⁷ and ~3.5×10⁷ independent clones, respectively. Bacterial clones binding to monomeric RBD (Fig. 7A) were enriched from each library by two cycles of magnetic cell sorting (MACS), followed by one cycle of fluorescence-activated cell sorting (FACS) (Fig. 7C). Individual colonies selected by FACS (30 from each library) were randomly picked, grown and analyzed by flow cytometry for binding to monomeric RBD and trimeric S proteins. Clones showing strong binding signals to low concentrations (5 nM) of RBD and S were picked for DNA sequencing of the VHHs. Five distinct Nb sequences from the D1-pool (1.10, 1.16, 1.26, 1.28, 1.29) and 4 Nb sequences from the D2-pool (2.1, 2.11, 2.15 and 2.20) were identified. Flow cytometry demonstrated specific binding of the RBD and S proteins to bacteria displaying these Nbs (Fig. 7D). Alignment of the amino acid sequence of the selected Nb clones binding the RBD of SARS-CoV-2 is shown in Figure 1A, indicating their unique CDRs.

### Production of Nbs and hcAbs, ligand binding and cross-competition.

hcAbs were constructed by the fusion of the selected VHH domains to the Fc region of human IgG1 (Fc) in a mammalian expression vector (Fig. 8A). Nine hcAbs were generated from the corresponding VHH clones and purified from culture supernatants of transfected 293F cells (Fig. 8B top). Monomeric Nbs were prepared after thrombin digestion of hcAbs with an engineered thrombin cleavage site between the Nb and Fc moieties (Fig. 8B, bottom).

Initially, binding of hcAbs to the S and RBD ligands was analyzed by ELISA, using serial hcAb dilutions and immunoplates coated with the SARS-CoV-2 antigens. The nine hcAbs showed similar binding curves to the trimeric S protein (Fig. 1B, left), while their binding activities to monomeric RBD were more variable (Fig. 1B, right). The half-maximal effective concentration (EC₅₀) for hcAb binding to the S ranged from 0.3 nM (1.26) to 1.8 nM (1.29), and from 1.0 nM (1.26) to 35 nM (1.29) for binding to RBD. These data confirmed the S and RBD binding specificity of the Nbs selected by bacterial display.

To gain insights on the hcAb epitopes in the SARS-CoV-2 RBD, cross-binding competition assays were carried out. Binding of a biotin-labeled hcAb (0.1 µg/ml, 2.5 nM) to plastic-bound S was determined by ELISA without or with 200-fold molar excess of unlabeled hcAbs (Fig. 9), and it is summarized as a heat map in Figure 1C. These experiments identified two distinct binding epitopes or Nb classes in the panel. Most hcAbs (1.10, 1.16, 1.26, 1.28, 2.1, 2.11, 2.15) cross-competed for S binding and they were clustered in the same A group, whereas binding of the 1.29 and 2.20 hcAbs to S, which cross-competed, was not inhibited by the others, and thus formed a distinct B group. Competition data also suggested certain epitope variability among the A group Nbs. For instance, hcAbs 1.10 or 2.1 did not blocked completely 1.26 or 2.15 binding to S (Fig. 1C and Fig. 9). Together, these data suggested that the Nbs preferentially targeted two distinct non-overlapping RBD regions, whereas group A Nbs had overlapping but still specific epitopes, determined by their unique CDRs.

### Affinity and kinetic constants for Nb and hcAb binding to S and RBD ligands.

Surface plasmon resonance (SPR) was used to establish the kinetic constants for hcAb and single domain Nb binding to the RBD or S ligands. To achieve homogenous surfaces and more accessible epitopes, protein ligands were produced with a C-terminal Flag-tag and captured with anti-Flag monoclonal Ab (mAb) covalently bound to a dextran sensor chip (CM5, BIAcore). After its capture, Nbs or hcAbs were injected through surfaces with and without ligand (RBD or S), which allowed the determination of specific binding (data not shown). As expected for their higher mass and dimeric nature, resonance units (RUs) were 3-4 times higher with the hcAbs compared to the Nbs (data not shown). The monomeric Nbs showed consistent low and sub-nanomolar affinities in binding to RBD and S, with the K_{D} ranging from 13 nM for 1.29 Nb binding to RBD to ~0.3 nM for 1.26 or 2.15 binding to either RBD or S. Nb dimerization in the hcAbs reduced at least 10-fold their k_{dis} from the ligand (Table 2), whereas the *k*ₐₛₛ did not changed for RBD binding or reduced slightly (~2-fold) for S binding. Indeed, some Nbs bound faster to the S than their corresponding hcAbs, likely due to its smaller size. Also, the similar Nb association rates to RBD alone and to the S indicated a good accessibility of their binding sites in the S trimer. Notably, the 1.26 and 2.15 hcAbs bound to S and RBD with affinity values in the picomolar (pM) range and all the hcAbs showed low *k_{dis}* (~10⁻⁴ s⁻¹; Table 2).

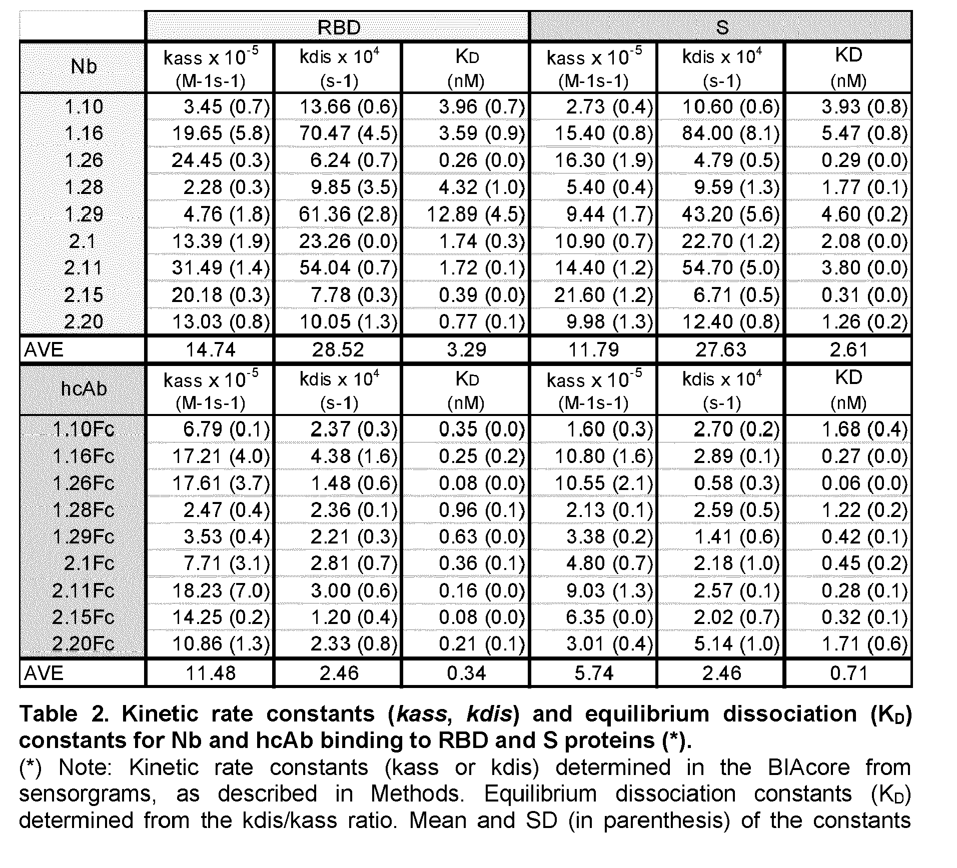

### hcAb inhibition of SARS-CoV-2 RBD binding to its cell entry receptor.

SARS-CoV-2 attaches to its cell surface ACE2 receptor through the RBD, which initiates the virus cell entry process. To further characterize the RBD-specific Nbs, their ability to compete RBD binding to human ACE2 (hACE2) was evaluated. To this end, the binding of biotin-labeled RBD-Fc (20 nM) to hACE2-coated immunoplates without or with an excess of each hcAb at increasing concentrations (from 50 to 200 nM) was compared (Fig. 2A). Except for 1.16, 2.11 and 2.20, the selected hcAbs fully inhibited (≥95%) RBD binding to hACE2 at 10-fold molar excess (200 nM). At this concentration, hcAbs 1.16 , 2.11 and 2.20 only reduced RBD binding to 20-40% of the maximal signal, whereas a control (C) hcAb had no significant effect (Fig. 2A). Notably, 1.10 and 1.26 fully inhibited RBD-hACE2 interaction at 5-fold molar excess (100 nM), showing that they were the most potent inhibitors of the SARS-CoV-2 RBD-receptor interaction. Other group A (2.1 and 2.15) and one group B (1.29) were able to inhibit RBD binding over 50% at 100 nM, whereas the remaining hcAbs showed poorer inhibition activity at this concentration.

### hcAb neutralization of S-pseudotyped and SARS-CoV-2 viruses in vitro.

The ability of the hcAbs to inhibit RBD binding to hACE2 should contribute to SARS-CoV-2 neutralization, as it would prevent virus cell entry. The hcAb neutralization of luciferase-encoding retroviruses pseudotyped with the SARS-CoV-2 S (namely, Spp), or with the G glycoprotein of Vesicular Stomatitis virus (VSVGpp) as a negative control was then tested. Vero-E6 cells were cultured with Spp or VSVGpp preincubated without and with the hcAbs at increasing concentrations (0.5, 5, and 50 nM), and infectivity measured as cell associated luciferase at 48 hours post infection (h.p.i.). All but the 1.16 and the 2.11 hcAbs inhibited Spp cell entry with diverse efficacy (Fig. 2B). The 1.26 hcAb, which bound to S with pM affinity (Table 1), was the most effective and prevented at least 90% of the cell infection at any of the tested concentrations. Similarly, other group A hcAbs such as 1.10, 1.28, 2.1 and 2.15, which blocked RBD binding to hACE2, inhibited more than 90% the infection at 5 and 50 nM (Fig. 2B. The 1.29 and the 2.20 group B hcAbs showed at least 90% neutralization activity at 50 nM (Fig. 2B). No inhibition of Spp cell entry was observed with a specificity control (C) hcAb at any of the concentrations tested (Fig. 2B). Also, no inhibition of VSVGpp cell entry was detected with any of the hcAbs tested (Fig. 10).

Next, *in vitro* neutralization assays with the SARS-CoV-2 virus was performed, and the same hcAb concentrations used above (0.5, 5, and 50 nM). In this assay, viruses were preincubated with the hcAbs and added to Vero-E6 cells for 1 h before they were removed by washing, and cell infection was quantified by immunofluorescence microscopy at 24 h.p.i. These experiments (Fig. 2C) showed complete inhibition (>90%) with the two highest concentrations tested of the group A hcAb that fully inhibited RBD binding to hACE2 (1.10, 1.26, 1.28, 2.1, and 2.15). Notably, 1.26 and 2.15 showed complete SARS-CoV-2 neutralization even at 0.5 nM, indicating a potent neutralization activity. On the contrary, the group A hcAb clones that showed weak inhibition of RBD-ACE2 interaction (1.16 and 2.11) had a deficient virus neutralization (Fig. 2C). Lastly, the group B 1.29 and 2.20 hcAbs inhibited >90% SARS-CoV-2 infection only at 50 nM (Fig. 2C), as seen with the Spp pseudovirus. The hcAb 1.29 showed slightly better neutralization of SARS-CoV-2 than 2.20, which was consistent with its greater inhibition of RBD-hACE2 interaction (Fig. 2A). As for the Spp assay, no inhibition of SARS-CoV-2 infection was observed with a control (C) hcAb (Fig. 2C).

### hcAb protection of hACE2-transgenic mice infected with a lethal dose of SARS-CoV-2

Based on hcAb inhibition of RBD-hACE2 interaction and neutralization of SARS-CoV-2 *in vitro* (Fig. 2), three group A hcAbs (1.10, 1.26 and 2.15) and the hcAb 1.29 from group B were selected to determine their therapeutic efficacy in an animal model of SARS-CoV-2 infection. A post-exposure protection assay in K18-hACE2 transgenic mice that reproduce major clinical symptoms of COVID-19 in humans (Winkler et al., 2020, Nature Immunology 21: 1327-1335) was performed. Groups of K18-hACE2 mice (n=6) were infected intranasally (i.n.) with ~5×10⁴ PFU of SARS-CoV-2, or mock infected with sterile HBS. One day post-infection (d.p.i.) each experimental group of mice received the 1.10, 1.26, 2.15, 1.29, or a control hcAb, intraperitoneally (i.p.) in a single dose of 150 µg/mouse (Fig. 3A). This dose is equivalent to ~8 mg/kg (for a mouse of 18 g), which is in the dose range of most therapeutic Abs administered systemically in humans (from 3 to 20 mg/kg) (Ovacik & Lin, 2018, Clin Transl Sci 11: 540-552). As control, the uninfected group was also mock-treated i.p. with sterile HBS.

The hcAb did not prevent weight loss of the infected animals in this stringent challenge model, when compared to uninfected controls (Fig. 3B). All infected groups showed a clear decline in body weight until 5 or 6 d.p.i., with 1.10-treated mice having the less severe reduction in weight compared with uninfected controls. Nonetheless, the four anti-RBD hcAbs significantly reduced lethality (Fig. 3C), in contrast to control hcAb-treated animals (Mantel-Cox log-rank test for survival P<0.0001 between control and 1.10, 1.26, 1.29 and 2.15 hcAbs). All infected mice treated with the control hcAb died between 5 and 7 d.p.i., whereas none died in the groups treated with 1.29 and 2.15, and only one animal out of 6 deceased (~16.6 %) in the groups treated with 1.10 or 1.26 at 7 d.p.i. (Fig. 3C). Notably, all the mice that survived the infection reached the body weight of the uninfected controls 15 d.p.i. (Fig. 3B), so that they recovered from the disease. Thus, the four RBD-binding hcAbs prevented the lethality of a SARS-CoV-2 infection in K18-hACE2 mice, suggesting their therapeutic potential to reduce the risk of death by COVID-19.

### Structure of the trimeric S protein in complex with RBD-specific Nbs.

Next, cryo-electron microscopy (cryo-EM) was used to determine the Nb binding mode to the RBD in a trimeric soluble S protein without the furin cleavage site and stabilized with a T4 phage trimerization domain, as well as with three proline residues in the S2 portion that enhanced protein expression and maintained its prefusion conformation (Hsieh et al., 2020, Science 369: 1501-1505) (Fig. 4A). S-Nb complexes with the 1.10, 1.26, 1.29 or 2.15 Nbs that showed *in vivo* efficacy against SARS-CoV-2 were prepared, which were purified for EM data collection. Subsequently, particle classification and S-Nb complex reconstruction was carried out. The open RBDs exhibited high flexibility and certain loops of the NTD and RBD were not properly defined in the structures.

In the trimeric complex structures, the S in the prefusion state had two monomers with the RBD in the open conformation and the third monomer with the RBD closed (Fig. 4B), a form that cannot engage the hACE2 receptor. S-Nb structures showed density corresponding to Nbs attached to the RBDs in the two conformations. The S-1.29 and S-2.15 reconstructions showed good densities for Nbs bound to the closed RBD and to one open RBD, but poor density to the second open RBD, so that, only two 1.29 and 2.15 Nbs bound to the S were modeled (Fig. 4B). In the S-1.10 complex structure, the Nbs were poorly defined in the map because of the relatively low number of particles used in the reconstruction, likely due to S disruption upon Nb binding; nonetheless, the Nb bound to one open RBD was modelled and refined. The S-1.26 complex reconstruction also showed weak density corresponding to the bound Nb at the top of the RBM; thus, 1.26 Nb fitting was avoided because of its binding mode looked like similar to 2.15.

### Modes of Nb binding to the SARS-CoV-2 RBD that mediated virus neutralization.

Modelling of the 1.10, 1.29 and 2.15 Nbs bound to the SARS-CoV-2 S showed three distinct ways of targeting the RBD that prevented virus infection (Fig. 4C). Neutralizing 1.10 and 2.15 Nbs from group A recognized the RBM, similar to class 2 human Abs (Fig. 4C), which bind the RBM in the RBD open and closed conformations. Nonetheless, whereas the 2.15 accessed the RBD top, the 1.10 Nb approached the RBM from the RBD outer face (Fig. 4C), and laid over the NTD. In the 2.15 Nb, the immunoglobulin (Ig) domain tip with the CDRs contacted preferentially the RBM valley and ridge, but the 1.10 bound to the valley and the RBM mesa at the terminal side of the receptor-binding subdomain (Fig. 4C). The group B 1.29 Nb bound to the edge of the RBD core and to its inner face, in a region that overlaps with that described for class 4 human Abs (Fig. 4C). Nonetheless, differing from human Abs, the 1.29 Nb interacted through one side of its Ig domain (see below), which run perpendicular to the RBD edge, this binding mode and the small Nb size, allows the single Ig domain to accommodate between closed and open RBDs in the S.

Model fitting into the cryo-EM maps allowed determination of the Nb binding epitopes in the RBD by analysis of their binding interfaces, as well as its correlation with the hACE2 receptor binding surface in the SARS-CoV-2 RBD (Fig. 5). The RBD engages hACE2 with the RBM top (Fig. 5A); the valley cradles the hACE2 α1 helix, its N-terminus and α2 contact the RBM ridge, whereas the RBM mesa region interacts with other hACE2 regions (β4- β5 turn and α10). The RBD region that connects with its receptor largely overlapped the group A 1.10 and 2.15 Nb binding epitopes. As hACE2, the 2.15 Nb Ig domain held onto the RBM top, and its CDRs made an extensive interaction with the RBM (Fig. 5B). In the modelled Nb-RBD complex, the basic residues in CDR2 of Nb 2.15 (Fig. 1A) penetrated into the RBM valley and the Nb Arg32 formed a salt bridge with the RBD E484, whereas the CDR3 bound to the ridge and other Ig domain loops contact the RBM mesa. Conversely, the 1.10 Nb approached the RBD from its outer face, and its CDR3 laid onto the RBM valley, whereas the other CDRs connected to the mesa (Fig. 5B).

According to cross-competition experiments (Fig. 1C), the group B 1.29 Nb had a non-overlapping epitope with the group A Nbs, which was confirmed by the cryo-EM structures (Fig. 5B and 5C). This Nb engaged the RBD core, and its epitope was mostly outside the hACE2 biding region. Nonetheless, the bound Nb contacted a few residues at the terminal RBM region, and its binding configuration likely interfered with RBD docking into hACE2. In contrast to the other Nbs, the 1.29 CDR3 (FG-loop) and the GFC β-sheet of its Ig domain made an extended interaction with the edge of the RBD core and its inner face (Fig. 5C). The CDR1 and CDR2 of 1.29 were not at the RBD-Nb binding interface.

### hcAb binding to prevailing RBD variants

Circulating SARS-CoV-2 variants of concern contain RBD residue substitutions that increase its hACE2 receptor-binding affinity and enhance virus transmission. These substitutions are exclusively at the RBM for the alpha, zeta, kappa and delta variants (e.g, N501Y, E484K/Q, T478K, L452R), whereas beta and gamma contains an additional K417N/T mutation in the RBD core that is close to the RBM (Fig. 5D). In contrast, the omicron variant contains multiple mutations located both at the RBM as well as the RBD core (Fig. 5E), which overlapped with the epitopes determined here for the neutralizing Nbs (Fig. 5B, 5C).

Group A (1.10, 1.26, 2.15) and group B (1.29) Nbs were evaluated for binding to the RBDs from variants of concern (alpha, beta, gamma, delta and omicron), as well as the related kappa and zeta variants. To facilitate hcAb binding to plastic-immobilized ligand RBD-Fc fusion proteins rather than monomeric RBDs were used. The RBD-Fc proteins from Wuhan strain (WA1) and from the different virus variants were used to determine binding activity of biotin-labeled hcAbs (Fig. 6A). The four hcAbs bound similarly to the alpha and WA1 RBDs, indicating that the RBD N501Y substitution did not affect binding of the hcAbs tested. In contrast, the single RBD E484K substitution in the zeta variant severely impaired 1.26 binding and completely abolished 2.15 recognition (Fig. 6A); this residue is within the epitopes recognized by these Nbs (Fig. 5), and residue E484 formed a salt bridge with Arg32 at the 2.15 CDR2 in the modeled interaction. Likely, this is a key interaction for the 2.15 Nb recognition of the RBM, as this Nb did not bind to RBD variants with the E484K (beta, gamma or zeta) or poorly to kappa with E484Q (Fig. 6A). The 1.26 Nb recognized weakly the RBDs with K484, but it bound the kappa variant that bears Q484 (Fig. 6A), which indicated that the RBD E484 is not essential for 1.26 binding. In contrast to the 1.26 and 2.15 hcAbs, 1.10 bound to the variants with the E484K substitution (beta, gamma or zeta) (Fig. 6A), but failed to bind to delta or kappa variants that shared the L452R mutation at the Nb epitope according to structural data (Fig. 5B and 5D). Importantly, the RBD mutations L452R and T478K found in the prevalent delta variant did not affect 1.26 or 2.15 hcAb binding to the RBD (Fig. 6A). However, none of the group A hcAbs bound to omicron RBD (Fig. 6A). The group B hcAb 1.29, which bound outside the RBM (Fig. 5C), was able to recognize all RBD variants except omicron (Fig. 6A). Notably, the binding activity of 1.29 to alpha, zeta, beta, gamma, delta and kappa variants was similar to WA1 RBD or only moderately reduced (ca. 2 to 5-fold) according to EC₅₀ values (Fig. 6A). In contrast, the 1.29 hcAb binding activity to omicron was weak, decreasing about 100-fold with respect to the WA1 RBD (Fig. 6A). Contrary to other variants, omicron RBD core mutations S371L-S373P-S375F overlapped with the 1.29 epitope (Fig. 5D _{6D}). Analysis of omicron recognition by the other hcAbs of the panel (Fig. 1) showed absence of binding by all group A hcAbs, and a weak RBD recognition by the other group B 2.20 hcAb (data not shown), similar to 1.29 hcAb.

### Bispecific hcAbs with increased affinity for RBD variants.

To increase the binding activity of hcAbs to RBD variants, bispecific hcAbs combining group B (1.29) and group A (1.10 or 1.26) Nbs in tandem fused to the Fc region were evaluated (Fig. 8C and 8D). Biotin-labeled bispecific hcAbs 1.29-1.10 and 1.29-1.26 were tested for binding to RBD-Fc molecules of WA1, beta, gamma, delta, kappa and omicron variants (Fig. 6B). The 1.29-1.10 hcAb showed greater binding to the WA1, beta and gamma RBDs than the monospecific 1.10 and 1.29 hcAbs, and similar EC₅₀ to kappa and delta variants as the 1.29 hcAb (Fig. 6B left panel). The bispecific 1.29-1.10 hcAb binding to omicron RBD was slightly enhanced, but still showed weak signals at high concentrations (Fig. 6B left panel). Similarly, the 1.29-1.26 hcAb improved RBD variant recognition with respect to the monospecific hcAbs (Fig. 6B right panel). Importantly, except for omicron, this bispecific hcAb EC₅₀ for the other variants were lower than the 1.29 EC₅₀ with the WA1 RBD. Since the 1.29 hcAb molecule was sufficient to provide full protection against lethal SARS-CoV-2 WA1 infection *in vivo,* these bispecific hcAbs, and particularly 1.29-1.26, are promising molecules for therapies against all current SARS-CoV-2 variants except omicron, which escapes from this panel of neutralizing hcAbs.

### Humanization of nanobodies

The sequence of camelid VHH domains were humanized by replacing specific residues in their framework regions by the most common amino acid found in the corresponding positions of human VH3 sequences (Vincke et al., 2009, J Biol Chem 284: 3273-3284; Soler, et al., 2021, Biomolecules 11). These modifications did not alter the antigen-binding activity of the original VHH. Individual VHH genes and gene libraries of VHHs have been humanized in this way (Moutel, et al., 2016, eLife 5: e16228; Nie et al., 2021, Biomedicine and Pharmacotherapy 137; 111328).

A similar strategy was followed to humanize the sequences of the VHHs encoding 1.26 and 1.29 nanobodies. Mutations in the framework regions of these nanobodies were rationally designed by replacing specific residues of the camelid VHH by the amino acid found in a consensus sequence of human VH3. The amino acid sequences of 1.26 and 1.29 were compared with the consensus sequence of human VH3 using Clustal Omega (Sievers and Higgings, 2014, Methods Mol Biol 1079: 105-116) and the residues expected to be neutral for nanobody binding and stability were chosen for replacement by the corresponding residue found in the human VH consensus (Figures 11A and 12A). The humanized VHH sequences of 1.26H1 and 1.29H1 were gene synthesized, cloned in the mammalian expression vector pIgΔCH1 fused to the Fc of human IgG1, and transfected to 293F cells for secretion as hcAb fusions. The parental 1.26 and 1.29 hcAbs were also expressed in parallel cell cultures. The binding activity of cell culture supernantants containing parental and humanized hcAbs were compared in ELISA plates coated with the S protein of SARS-CoV-2. Binding signals of serial dilutions confirmed similar binding activity for 1.26 and 1.26H1 hcAbs (Figure 11B), and for 1.29 and 1.29H1 (Figure 12B).

The amino acid sequence of 1.26H1 and 1.29H1 are SEQ ID NO: 30 and SEQ ID NO: 31 respectively.

### 2.20 hcAb protection of hACE2-transgenic mice infected with a lethal dose of SARS-CoV-2.

Based on hcAb inhibition of RBD-hACE2 interaction and neutralization of SARS-CoV-2 *in vitro* (Fig. 2), the hcAb 2.20 from group B was selected to determine their therapeutic efficacy in an animal model of SARS-CoV-2 infection. A post-exposure protection assay in K18-hACE2 transgenic mice that reproduce major clinical symptoms of COVID-19 in humans {Winkler, 2020, supra} was performed. Groups of K18-hACE2 mice (n=6) were infected intranasally (i.n.) with ~5×10⁴ PFU of SARS-CoV-2, or mock infected with sterile HBS. One day post-infection (d.p.i.) each experimental group of mice received the 2.20 or a control hcAb, intraperitoneally (i.p.) in a single dose of 150 µg/mouse (Fig. 13A). This dose is equivalent to ~8 mg/kg (for a mouse of 18 g), which is in the dose range of most therapeutic Abs administered systemically in humans (from 3 to 20 mg/kg) (Ovacik, 2018, supra). As control, the uninfected group was also mock-treated i.p. with sterile HBS.

The hcAb did not prevent weight loss of the infected animals in this stringent challenge model, when compared to uninfected controls (Fig. 13B). The infected groups showed a clear decline in body weight until 5 or 6 d.p.i., with 2.20-treated mice having less severe reduction in weight compared with the control group. The 2.20 hcAb significantly reduced lethality (Fig. 13C), in contrast to control hcAb-treated animals (Mantel-Cox log-rank test for survival *P*=0.0009 between control and 2.20 hcAb). All infected mice treated with the control hcAb died between 6 d.p.i., whereas only two animals out of 6 (~33.3 %) deceased in the group treated with 2.20 (Fig. 13C). Notably, all the mice that survived the infection reached the body weight of the uninfected controls 15 d.p.i. (Fig. 13B), so that they recovered from the disease. Thus, the 2.20 hcAb diminished the lethality of a SARS-CoV-2 infection in K18-hACE2 mice, suggesting their therapeutic potential to reduce the risk of death by COVID-19.

## Claims

1. A nanobody directed against the receptor binding domain (RBD) of spike protein from severe acute respiratory syndrome coronavirus 2 (SARS-CoV-2), wherein the nanobody comprises a variable domain comprising a CDR1 comprising the amino acid sequence shown in SEQ ID NO: 7, a CDR2 comprising the amino acid sequence shown in SEQ ID NO: 8 and a CDR3 comprising the amino acid sequence shown in SEQ ID NO: 9, or functionally equivalent variants of said CDRs.

2. The nanobody according to claim 1, wherein said variable domain comprises an amino acid sequence of SEQ ID NO: 24 or a functionally equivalent variant thereof, preferably wherein said functionally equivalent variant has at least 80% sequence identity to the amino acid sequence shown in SEQ ID NO: 24.

3. The nanobody according to any one of claims 1 or 2, wherein the CDR1 comprises the amino acid sequence shown in SEQ ID NO: 7, the CDR 2 comprises the amino acid sequence shown in SEQ ID NO: 8 and the CDR3 comprises the amino acid sequence shown in SEQ ID NO: 9.

4. The nanobody according to any one of claims 1 to 3, wherein the nanobody is humanized.

5. A fusion protein comprising the nanobody according to any one of claims 1 to 4 and the Fc region of a heavy chain.

6. The fusion protein according to claim 5, further comprising a second nanobody directed against the RBD of spike protein from SARS-CoV-2, wherein the first nanobody and the second nanobody are optionally linked by a peptide linker, and wherein the second nanobody comprises a variable domain selected from the group consisting of:
(A) a variable domain comprising a CDR1 comprising the amino acid sequence shown in SEQ ID NO: 1, a CDR2 comprising the amino acid sequence shown in SEQ ID NO: 2 and a CDR3 comprising the amino acid sequence shown in SEQ ID NO: 3, or functionally equivalent variants of said CDRs, or
(B) a CDR1 comprising the amino acid sequence shown in SEQ ID NO: 4, a CDR2 comprising the amino acid sequence shown in SEQ ID NO: 5 and a CDR3 comprising the amino acid sequence shown in SEQ ID NO: 6, or functionally equivalent variants of said CDRs, or
(C) a CDR1 comprising the amino acid sequence shown in SEQ ID NO: 10, a CDR2 comprising the amino acid sequence shown in SEQ ID NO: 11 and a CDR3 comprising the amino acid sequence shown in SEQ ID NO: 12, or functionally equivalent variants of said CDRs, or
(D) a CDR1 comprising the amino acid sequence shown in SEQ ID NO: 13, a CDR2 comprising the amino acid sequence shown in SEQ ID NO: 14 and a CDR3 comprising the amino acid sequence shown in SEQ ID NO: 15, or functionally equivalent variants of said CDRs, or
(E) a CDR1 comprising the amino acid sequence shown in SEQ ID NO: 16, a CDR2 comprising the amino acid sequence shown in SEQ ID NO: 17 and a CDR3 comprising the amino acid sequence shown in SEQ ID NO: 18, or functionally equivalent variants of said CDRs, or
(F) a CDR1 comprising the amino acid sequence shown in SEQ ID NO: 19, a CDR2 comprising the amino acid sequence shown in SEQ ID NO: 20 and a CDR3 comprising the amino acid sequence shown in SEQ ID NO: 21, or functionally equivalent variants of said CDRs.

7. A heavy chain antibody comprising two heavy chains, wherein each heavy chain comprises the fusion protein according to any one of claims 5 or 6, wherein both heavy chains are identical.

8. A fusion protein comprising two nanobodies, wherein
(i) the first nanobody is the nanobody according to any one of claims 1 to 4 and
(ii) the second nanobody comprises a variable domain selected from the group consisting of:
(a) a variable domain comprising a CDR1 comprising the amino acid sequence shown in SEQ ID NO: 1, a CDR2 comprising the amino acid sequence shown in SEQ ID NO: 2 and a CDR3 comprising the amino acid sequence shown in SEQ ID NO: 3, or functionally equivalent variants of said CDRs, or
(b) a CDR1 comprising the amino acid sequence shown in SEQ ID NO: 4, a CDR2 comprising the amino acid sequence shown in SEQ ID NO: 5 and a CDR3 comprising the amino acid sequence shown in SEQ ID NO: 6, or functionally equivalent variants of said CDRs, or
(c) a CDR1 comprising the amino acid sequence shown in SEQ ID NO: 10, a CDR2 comprising the amino acid sequence shown in SEQ ID NO: 11 and a CDR3 comprising the amino acid sequence shown in SEQ ID NO: 12, or functionally equivalent variants of said CDRs, or
(d) a CDR1 comprising the amino acid sequence shown in SEQ ID NO: 13, a CDR2 comprising the amino acid sequence shown in SEQ ID NO: 14 and a CDR3 comprising the amino acid sequence shown in SEQ ID NO: 15, or functionally equivalent variants of said CDRs, or
(e) a CDR1 comprising the amino acid sequence shown in SEQ ID NO: 16, a CDR2 comprising the amino acid sequence shown in SEQ ID NO: 17 and a CDR3 comprising the amino acid sequence shown in SEQ ID NO: 18, or functionally equivalent variants of said CDRs, or
(f) a CDR1 comprising the amino acid sequence shown in SEQ ID NO: 19, a CDR2 comprising the amino acid sequence shown in SEQ ID NO: 20 and a CDR3 comprising the amino acid sequence shown in SEQ ID NO: 21, or functionally equivalent variants of said CDRs.

9. A nucleic acid encoding the nanobody according to any one of claims 1 to 4 or the fusion protein according to any one of claims 5 or 6 or according to claim 8, or a vector comprising said nucleic acid, or a host cell comprising said nucleic acid or said vector.

10. A pharmaceutical composition comprising the nanobody according to any one of claims 1 to 4, or the fusion protein according to any one of claims 5 or 6, or the heavy chain antibody according to claim 7, or the fusion protein according to claim 8, and a pharmaceutically acceptable excipient or carrier.

11. The nanobody according to any one of claims 1 to 4, or the fusion protein according to any one of claims 5 or 6, or the heavy chain antibody according to claim 7, or the fusion protein according to claim 8, for use as a medicament.

12. The nanobody according to any one of claims 1 to 4, or the fusion protein according to any one of claims 5 or 6, or the heavy antibody according to claim 7, or the fusion protein according to claim 8, for use in the treatment and/or prevention of an infection caused by severe acute respiratory syndrome coronavirus 2 (SARS-CoV-2).

13. An *in vitro* method for diagnosing an infection by severe acute respiratory syndrome coronavirus 2 (SARS-CoV-2) in a subject comprising contacting the nanobody according to any one of claims 1 to 4, or the fusion protein according to any one of claims 5 or 6, or the heavy chain antibody according to claim 7, or the fusion protein according to claim 8, with a sample from the subject and detecting the specific binding of said nanobody, fusion protein or heavy chain antibody to said sample, wherein if said nanobody, fusion protein or heavy chain antibody specifically bind to said sample, the subject is diagnosed as suffering from an infection by SARS-Cov-2.

14. Use of the nanobody according to any one of claims 1 to 4, or the fusion protein according to any one of claims 5 or 6, or the heavy chain antibody according to claim 7, or the fusion protein according to claim 8 for diagnosing an infection by severe acute respiratory syndrome coronavirus 2 (SARS-CoV-2) in a subject.
